(19) Europäisches Patentamt
European Patent Office
Office européen des brevets

(11) **EP 4 209 821 B1**

(12) **EUROPEAN PATENT SPECIFICATION**

(45) Date of publication and mention
of the grant of the patent:
**10.12.2025 Bulletin 2025/50**

(21) Application number: **21863868.2**

(22) Date of filing: **08.03.2021**

(51) International Patent Classification (IPC):
*G02B 23/24* (2006.01)    *G02B 23/26* (2006.01)
*A61B 1/00* (2006.01)    *A61B 1/045* (2006.01)
*A61B 5/107* (2006.01)    *A61B 1/06* (2006.01)

(52) Cooperative Patent Classification (CPC):
**A61B 5/1076; A61B 1/00006; A61B 1/00009;**
**A61B 1/0005; A61B 1/00059; A61B 1/0605;**
**A61B 1/0655; A61B 5/1079; G02B 23/2423;**
A61B 1/00194; A61B 1/0676

(86) International application number:
**PCT/JP2021/008993**

(87) International publication number:
**WO 2022/049807 (10.03.2022 Gazette 2022/10)**

(54) **ENDOSCOPE SYSTEM AND METHOD FOR OPERATING SAME**

ENDOSKOPSYSTEM UND VERFAHREN ZUM BETRIEB DAVON

SYSTÈME D'ENDOSCOPE ET SON PROCÉDÉ DE FONCTIONNEMENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **02.09.2020 JP 2020147691**

(43) Date of publication of application:
**12.07.2023 Bulletin 2023/28**

(73) Proprietor: **FUJIFILM Corporation**
**Tokyo 106-8620 (JP)**

(72) Inventors:
• **YOSHIOKA, Masato**
**Ashigarakami-gun, Kanagawa 258-8538 (JP)**
• **SAKAGUCHI, Yuichi**
**Ashigarakami-gun, Kanagawa 258-8538 (JP)**
• **SUGIZAKI, Makoto**
**Ashigarakami-gun, Kanagawa 258-8538 (JP)**
• **INOMATA, Kazuaki**
**Ashigarakami-gun, Kanagawa 258-8538 (JP)**

(74) Representative: **Dehns Germany Partnerschaft**
**mbB**
**Theresienstraße 6-8**
**80333 München (DE)**

(56) References cited:
WO-A1-2017/047321    WO-A1-2018/051680
JP-A- 2006 187 427    JP-A- 2011 031 026
JP-A- 2016 054 842    JP-A- 2020 014 807
US-A1- 2018 008 124    US-A1- 2019 204 068
US-A1- 2019 320 886    US-A1- 2020 107 698

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**BACKGROUND OF THE INVENTION**

1. Field of the Invention

The present invention relates to an endoscope system that displays a virtual scale to be used to measure the size of a subject.

2. Description of the Related Art

A distance to a subject, the size of a subject, or the like is acquired in an endoscope system that includes a light source device, an endoscope, and a processor device. For example, in WO2018/051680A, a subject is irradiated with illumination light and measurement light and a measurement light-irradiation region, such as spotlight, appears on the subject due to irradiation with measurement light. Then, a virtual scale used to measure the size of the subject is displayed in an image to correspond to the position of the spotlight.

**SUMMARY OF THE INVENTION**

As in WO2018/051680A, a length measurement-compatible endoscope, which can emit measurement light, is necessary to display a virtual scale using measurement light. In order to execute a length measurement mode in which a virtual scale is displayed in an endoscope system, it is necessary to determine whether or not a length measurement-compatible endoscope is connected. US 2019/204068 A1 discloses a measurement support device, an endoscope system, a processor for endoscope system and a measurement support method. US 2019/320886 A1 discloses an endoscope apparatus. US 2018/008124 A1 discloses a power supply system.

An object of the present invention is to provide an endoscope system that can determine whether or not a length measurement mode can be executed depending on connection of an endoscope.

The invention is defined by the features of independent claim 1. Preferred embodiments are defined in the dependent claims 2-9.

According to the present invention, it is possible to determine whether or not a length measurement mode can be executed depending on the connection of an endoscope.

**BRIEF DESCRIPTION OF THE DRAWINGS**

Fig. 1 is a schematic diagram of an endoscope system.
Fig. 2 is a perspective view of a balloon.
Fig. 3 is a front view of the balloon.
Fig. 4 is a diagram showing the balloon that is deflated in an intestinal canal.
Fig. 5 is a diagram showing the balloon that is inflated in the intestinal canal.
Fig. 6 is a front view of a distal end part of an endoscope.
Fig. 7 is a perspective view of the distal end part of the endoscope.
Fig. 8 is a block diagram showing the functions of the endoscope system.
(A) of Fig. 9 is an image diagram showing a state where a digital zoom function is turned off and (B) of Fig. 9 is an image diagram showing a state where a digital zoom function is turned on.
Fig. 10 is a schematic diagram showing a measurement light-emitting unit.
Fig. 11 is a cross-sectional view of a distal end part of an endoscope including the measurement light-emitting unit.
Fig. 12 is a plan view of a transparent lid.
Fig. 13 is a schematic diagram showing a travel direction of measurement light.
Fig. 14 is a block diagram showing the functions of a system controller.
Fig. 15 is an image diagram in which a scale display icon, a scale non-display icon, or the like is displayed.
Fig. 16 is a block diagram showing the functions of the system controller.
Fig. 17 is a diagram showing a first control.
Fig. 18 is an image diagram showing a message that is displayed in a case where the first control is performed.
Fig. 19 is a diagram showing that a special observation mode is canceled and switched to a length measurement mode.
Fig. 20 is a diagram showing a second control.

Fig. 21 is an image diagram showing a message that is displayed in a case where the second control is performed.

Fig. 22 is a diagram showing a third control.

Fig. 23 is an image diagram showing a message that is displayed in a case where the third control is performed.

Fig. 24 is a block diagram showing the functions of the system controller.

Fig. 25 is a diagram showing a first light emission control table.

Fig. 26 is a diagram showing Coordinate areas 1 to 5.

Fig. 27 is a diagram showing a second light emission control table.

Fig. 28 is a diagram showing a light emission control in a length measurement mode.

Fig. 29 is a diagram showing light emission and an image pickup control of a first pattern of the length measurement mode.

Fig. 30 is a diagram showing light emission and an image pickup control of a second pattern of the length measurement mode.

Fig. 31 is a block diagram of the functions of a signal processing unit.

Fig. 32 is an image diagram showing a virtual scale that is displayed in the case of a near end Px.

Fig. 33 is an image diagram showing a virtual scale that is displayed in the case of an intermediate vicinity Py.

Fig. 34 is an image diagram showing a virtual scale that is displayed in the case of a far end Pz.

Fig. 35 is a diagram showing virtual scales having various shapes.

Fig. 36 is a diagram showing virtual scales having different sizes.

Fig. 37 is a diagram showing virtual scales having different colors.

Fig. 38 is a diagram showing a distorted concentric circular virtual scale.

Fig. 39 is a diagram showing representative point data.

Fig. 40 is a diagram showing the processing of a table updating unit.

Fig. 41 is an image diagram showing a virtual scale that is displayed in a case where planar measurement light is emitted.

Fig. 42 is a diagram showing planar light that includes two first feature lines.

Fig. 43 is a diagram showing the functions of the signal processing unit.

Fig. 44 is an image diagram showing an image that is displayed in a case where planar light including two first feature lines is emitted.

Fig. 45 is a diagram showing the travel direction of planar light in a case where the planar light including two first feature lines is emitted.

Fig. 46 is a diagram showing diffraction spots.

Fig. 47 is a diagram regarding a method of calculation two-dimensional information and three-dimensional information of a subject in a case where the diffraction spots are used.

Fig. 48 is a block diagram showing the functions of the signal processing unit.

Fig. 49 is a diagram showing processing for obtaining a first captured image from which noise has been removed.

Fig. 50 is a diagram showing a binarized first color information image.

Fig. 51 is a diagram showing a binarized second color information image.

Fig. 52 is a diagram showing the binarized second color information image.

Fig. 53 is a diagram showing the binarized first color information image.

Fig. 54 is a block diagram showing the functions of the signal processing unit.

Fig. 55 is a diagram showing processing for obtaining a first difference image or a second difference image.

Fig. 56 is a diagram showing first difference processing.

Fig. 57 is a diagram showing second difference processing.

Fig. 58 is a block diagram showing the functions of the signal processing unit.

Fig. 59 is a schematic diagram showing a spot that includes a white central region and a peripheral region.

Fig. 60 is a graph showing the distribution of the pixel values of various images of a captured image.

Fig. 61 is a graph showing a relationship between the transmission distribution of color filters having the respective colors and the wavelength range of measurement light.

Fig. 62 is a block diagram showing the functions of an irradiation region recognition unit.

Fig. 63 is diagram showing examples of a pattern of a spot that is deformed from a circular shape.

Fig. 64 is a block diagram showing the functions of the signal processing unit.

Fig. 65 is a schematic diagram showing a convex polyp.

Fig. 66 is a diagram showing the heights of spots.

Fig. 67 is a diagram regarding the calculation of an offset distance D6.

Figs. 68A to 68E are schematic diagrams showing virtual scales having different widths of lines.

Fig. 69 is a schematic diagram showing a concentric circular virtual scale.

Fig. 70 is a schematic diagram showing a virtual scale in which gradation is added to a line.

Figs. 71A to 71C are schematic diagrams showing virtual scales having different gaps of broken lines.

Figs. 72A to 72C are schematic diagrams showing virtual scales having different numbers of lines.

Fig. 73 is a block diagram showing the functions of the signal processing unit.

Fig. 74 is an image diagram showing a spot that is emitted to a peripheral edge of a polyp.

Fig. 75 is a diagram showing a virtual scale of which a proximal end is aligned with the position of the spot.

Fig. 76 is a diagram showing two virtual scales of which proximal ends are aligned with the position of the spot.

Fig. 77 is a diagram showing a virtual scale of which a proximal end is aligned with the position of the spot and which is formed of a circle and a line segment.

Figs. 78A to 78C are diagrams showing virtual scales of which proximal ends are aligned with the position of the spot.

Fig. 79 is a block diagram showing the functions of the signal processing unit.

Fig. 80 is a block diagram showing the functions of a reference scale setting unit.

Fig. 81 is an image diagram showing a virtual scale that is superimposed and displayed on a polyp.

Fig. 82 is a block diagram showing the functions of a measured value scale generation unit.

Fig. 83 is an image diagram showing a region of interest.

Fig. 84 is a diagram showing a measurement portion.

Fig. 85 is an image diagram regarding a measured value scale.

Fig. 86 is an image diagram showing a measured value scale that is superimposed and displayed on a polyp.

Fig. 87 is a diagram showing a distorted grid region.

Fig. 88 is a diagram showing a square grid region.

Figs. 89A to 89C are diagrams showing examples in which virtual scales having different shapes are displayed inside and outside an effective measurement region.

Figs. 90A to 90C are diagrams showing examples in which virtual scales formed of different types of lines are displayed inside and outside an effective measurement region.

Fig. 91 is a diagram regarding the acquisition of a static image in the length measurement mode.

Fig. 92 is a diagram showing first to third captured images.

Fig. 93 is an image diagram showing the second and third captured images displayed in a case where a static image is acquired.

Fig. 94 is a diagram regarding the acquisition of a static image in the length measurement mode.

Fig. 95 is a diagram regarding the acquisition of a static image in the length measurement mode.

Fig. 96 is a block diagram showing the functions of the signal processing unit.

Fig. 97 is a block diagram showing the functions of a calibration apparatus.

Fig. 98 is a schematic diagram of an inspection system.

Fig. 99 is a plan view showing a test chart.

Fig. 100 is an image diagram showing an inspection reference position, a spot, and a virtual scale that are displayed in an inspection image.

Fig. 101 is an image diagram showing the inspection reference position, the spot, and the virtual scale that are displayed in the inspection image.

Fig. 102 is an image of a chart with 5 mm ruled lines.

Fig. 103 is an image of a chart with 5 mm ruled lines (captured on a side close to a far end as compared to Fig. 102).

Fig. 104 is a diagram showing pixel positions of spots in an X direction.

Fig. 105 is a diagram showing pixel positions of spots in a Y direction.

Fig. 106 is a diagram showing pixel positions of spots in the X direction.

Fig. 107 is a diagram showing pixel positions of spots in the Y direction.

Fig. 108 is a diagram showing stripe-pattern light ZPL.

Fig. 109 is a diagram showing the light emission patterns of stripe-pattern light ZPL having a phase X, stripe-pattern light ZPL having a phase Y, and stripe-pattern light ZPL having a phase Z.

Fig. 110 is a diagram showing grid-pattern measurement light LPL.

Fig. 111 is a diagram showing a light emission pattern in which grid-pattern measurement light is intermittently emitted.

Fig. 112 is a diagram showing three-dimensional planar light TPL.

Fig. 113 is a diagram showing a light emission pattern in which three-dimensional planar light TPL is intermittently emitted.

## DESCRIPTION OF THE PREFERRED EMBODIMENTS

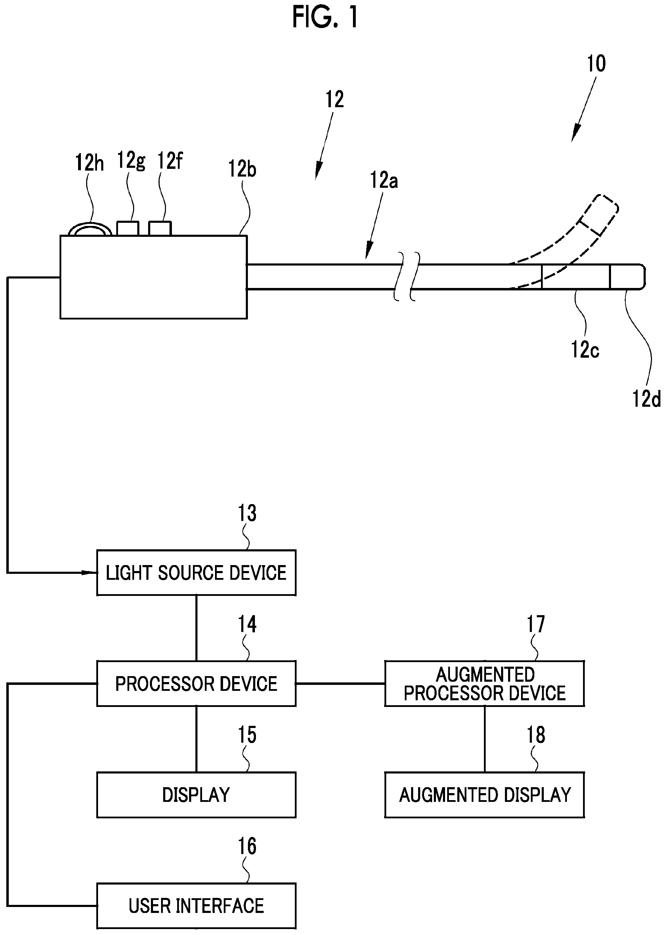

As shown in Fig. 1, an endoscope system 10 includes an endoscope 12, a light source device 13, a processor device 14, a display 15, a user interface 16, an augmented processor device 17, and an augmented display 18. The endoscope 12 is optically connected to the light source device 13, and is electrically connected to the processor device 14. The endoscope 12 includes an insertion part 12a that is to be inserted into a body of an object to be observed, an operation part 12b that is provided at a proximal end portion of the insertion part 12a, and a bendable part 12c and a distal end part 12d that are provided on a distal end side of the insertion part 12a. In a case where the operation part 12b is operated, the bendable part 12c is operated to be bent. As the bendable part 12c is operated to be bent, the distal end part 12d is made to face in a desired direction.

Further, the operation part 12b is provided with an observation mode selector switch 12f that is used for an operation for switching an observation mode, a static image-acquisition instruction switch 12g that is used to give an instruction to acquire a static image of the object to be observed, and a zoom operation part 12h that is used for an operation of a zoom lens 21b.

The processor device 14 is electrically connected to the display 15 and the user interface 16. The display 15 outputs and displays an image, information, or the like of the object to be observed that is processed by the processor device 14. The user interface 16 includes a keyboard, a mouse, a touch pad, a microphone, and the like and has a function to receive an input operation, such as function settings. The augmented processor device 17 is electrically connected to the processor device 14. The augmented display 18 outputs and displays an image, information, or the like that is processed by the augmented processor device 17.

The endoscope 12 has a normal observation mode, a special observation mode, and a length measurement mode, and these modes are switched by the observation mode selector switch 12f. The normal observation mode is a mode in which an object to be observed is illuminated with illumination light. The special observation mode is a mode in which an object to be observed is illuminated with special light different from the illumination light. In the length measurement mode, an object to be observed is illuminated with the illumination light or measurement light and a virtual scale to be used for the measurement of the size and the like of an object to be observed is displayed in a subject image obtained from the image pickup of the object to be observed. A subject image on which the virtual scale is not superimposed and displayed is displayed on the display 15, but a subject image on which the virtual scale is superimposed and displayed is displayed on the augmented display 18.

The illumination light is light that is used to apply brightness to the entire object to be observed to observe the entire object to be observed. The special light is light that is used to enhance a specific region of the object to be observed. The measurement light is light that is used for the display of the virtual scale. Further, a virtual scale to be displayed in an image will be described in the present embodiment, but a real scale may be provided in a real lumen so that the real scale can be checked using an image. In this case, it is conceivable that the real scale is inserted through a forceps channel of the endoscope 12 and is made to protrude from the distal end part 12d.

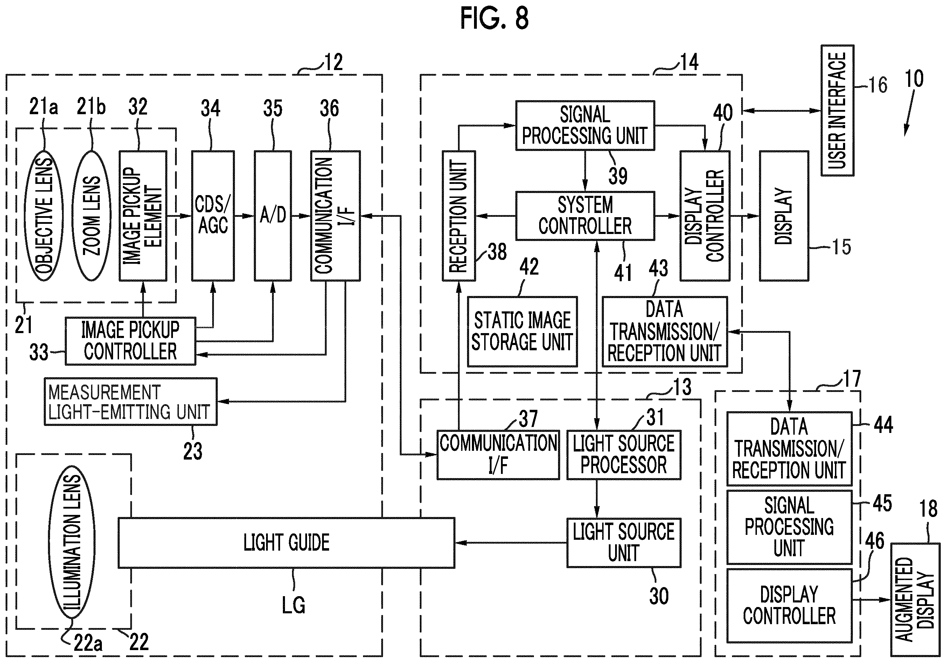

In a case where a user operates the static image-acquisition instruction switch 12g, the screen of the display 15 is frozen and displayed and an alert sound (for example, "beep") informing the acquisition of a static image is generated together. Then, the static images of the subject image, which are obtained before and after the operation timing of the static image-acquisition instruction switch 12g, are stored in a static image storage unit 42 (see Fig. 8) provided in the processor device 14. The static image storage unit 42 is a storage unit, such as a hard disk or a universal serial bus (USB) memory. In a case where the processor device 14 can be connected to a network, the static images of the subject image may be stored in a static image storage server (not shown), which is connected to the network, instead of or in addition to the static image storage unit 42.

A static image-acquisition instruction may be given using an operation device other than the static image-acquisition instruction switch 12g. For example, a foot pedal may be connected to the processor device 14, and may be adapted to give a static image-acquisition instruction in a case where a user operates the foot pedal (not shown) with a foot. A static image-acquisition instruction may also be given by a foot pedal that is used to switch a mode. Further, a gesture recognition unit (not shown), which recognizes the gestures of a user, may be connected to the processor device 14, and may be adapted to give a static image-acquisition instruction in a case where the gesture recognition unit recognizes a specific gesture of a user. The gesture recognition unit may also be used to switch a mode.

Further, a sight line input unit (not shown), which is provided close to the display 15, may be connected to the processor device 14, and may be adapted to give a static image-acquisition instruction in a case where the sight line input unit recognizes that a user's sight line is in a predetermined region of the display 15 for a predetermined time or longer. Furthermore, a voice recognition unit (not shown) may be connected to the processor device 14, and may be adapted to give a static image-acquisition instruction in a case where the voice recognition unit recognizes a specific voice generated by a user. The voice recognition unit may also be used to switch a mode. Moreover, an operation panel (not shown), such as a touch panel, may be connected to the processor device 14, and may be adapted to give a static image-acquisition instruction in a case where a user performs a specific operation on the operation panel. The operation panel may also be used to switch a mode.

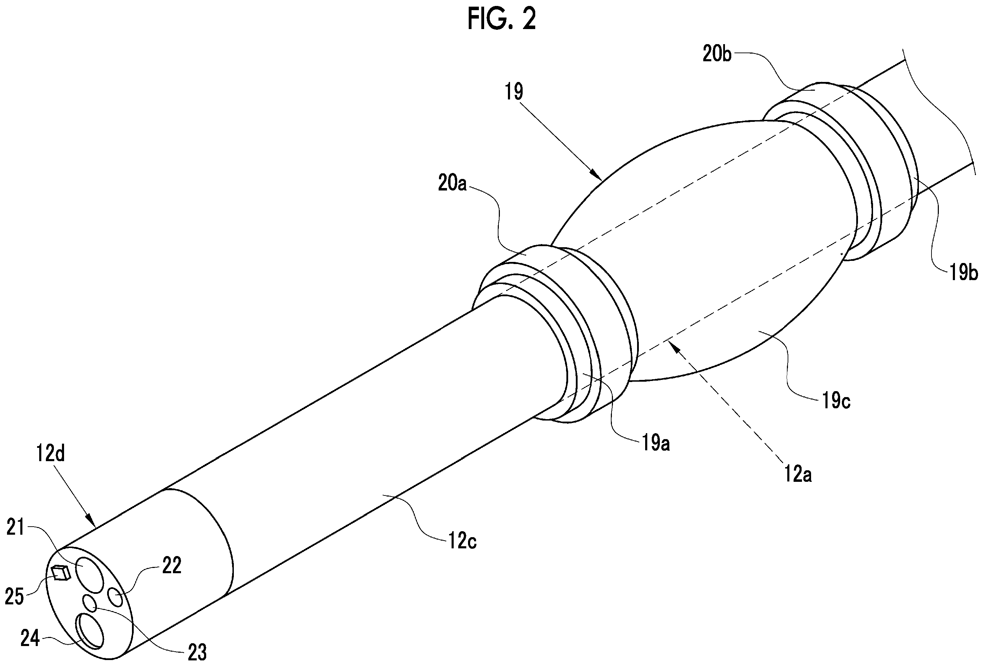

As shown in Fig. 2, the distal end part 12d is provided with an image pickup optical system 21 that receives light from a subject, illumination optical systems 22 that are used to irradiate the subject with the illumination light, a measurement light-emitting unit 23 that radiates the measurement light used in the length measurement mode to the subject, an opening 24 that allows a treatment tool to protrude toward the subject, and an air/water supply nozzle 25 that is used to supply air and water.

A balloon 19 as a fixing member is attachably and detachably mounted on the insertion part 12a. The balloon 19 is a disposable type balloon, is discarded after being used one time or a small number of times, and is replaced with a new one. The number of times mentioned here means the number of cases, and a small number of times refer to 10 times or less.

The balloon 19 is formed in a substantially tubular shape, of which end portions are narrowed, using an elastic material, such as rubber. The balloon 19 includes a distal end portion 19a and a proximal end portion 19b having a small diameter and an intermediate bulging portion 19c. The insertion part 12a is inserted into the balloon 19, the balloon 19 is disposed at a predetermined position, and rings 20a and 20b made of rubber are then fitted to the distal end portion 19a and the proximal end portion 19b, so that the balloon 19 is fixed to the insertion part 12a.

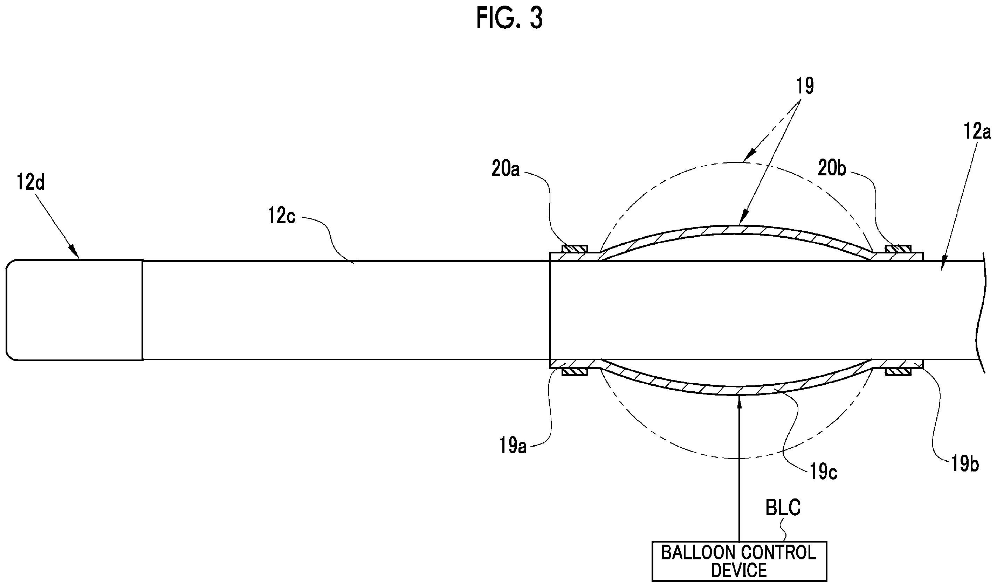

It is preferable that, as shown in Fig. 3, the predetermined position at which the balloon 19 is fixed to the insertion part 12a is closer to the proximal end of the insertion part 12a than the bendable part 12c and is a position where the distal end of the distal end portion 19a of the balloon 19 and the proximal end of the bendable part 12c coincide with each other. Accordingly, the balloon 19 does not hinder the bending operation of the bendable part 12c and the bendable part 12c also does not hinder the inflation or deflation of the balloon 19. As described later, the inflation and deflation of the balloon 19 are controlled by a balloon control device BLC. It is preferable that the balloon control device BLC is operated by the user interface 16.

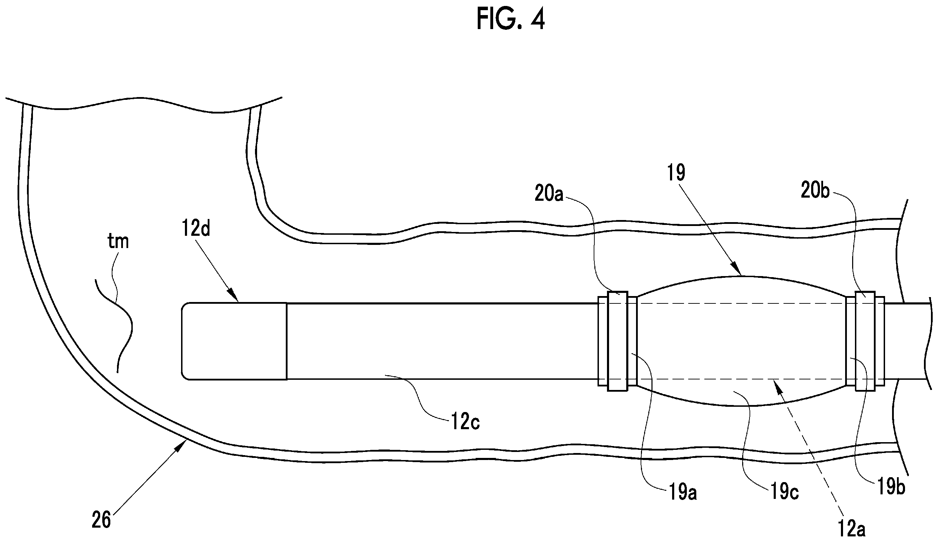

In a state where the balloon 19 is deflated as shown in Fig. 4, the insertion part 12a is not fixed to an intestinal canal 26. Accordingly, in a case where measurement light is emitted and an image is picked up in a state where the balloon is deflated, the position of the distal end part 12d may be moved in a vertical direction and a horizontal direction. For this reason, there is a case where an object to be observed that a user wants to measure cannot be accurately irradiated with the measurement light.

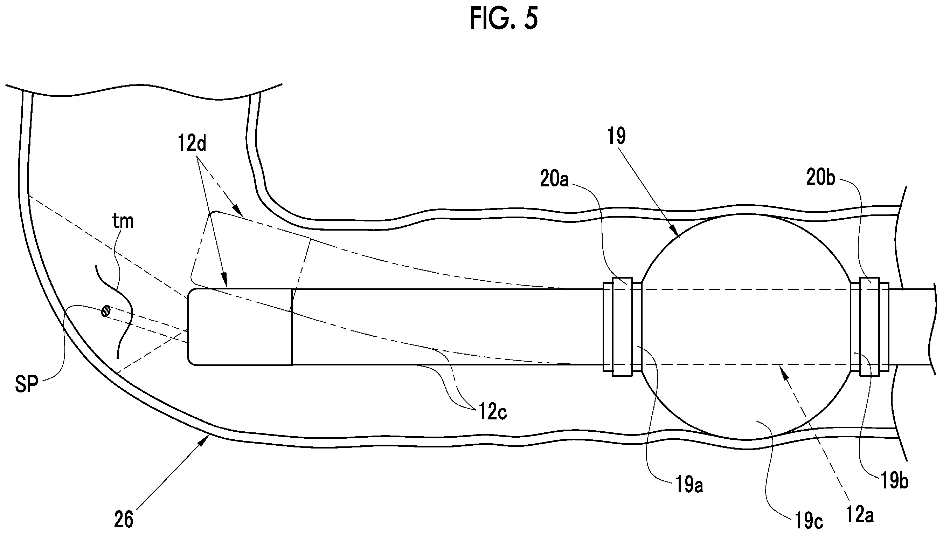

Accordingly, a user sets the balloon 19 to an inflated state as shown in Fig. 5 by the control of the balloon control device BLC. Since the outer diameter of the balloon 19 in the inflated state is formed depending on the inner diameter of the intestinal canal 26, the insertion part 12a is in a state where the position of the insertion part 12a is fixed in the intestinal canal 26. Accordingly, since the insertion part 12a is fixed to the intestinal canal 26, the object to be observed measured by the user can be accurately irradiated with the measurement light. "A state where ~ is fixed" mentioned here includes a state where the position of the insertion part 12a in an insertion direction is fixed but the orientation of the distal end part 12d can be finely adjusted.

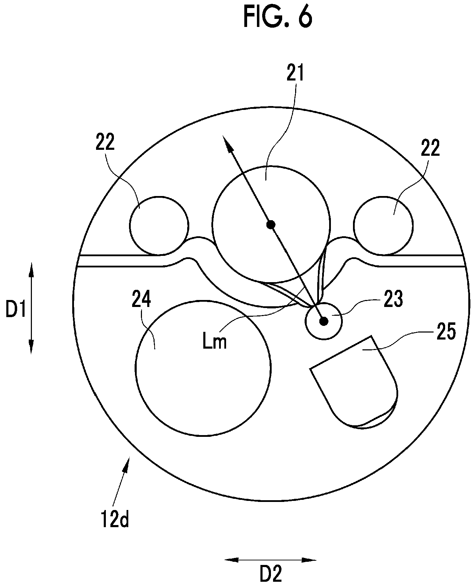

As shown in Fig. 6, the distal end part 12d of the endoscope has a substantially circular shape, and is provided with the image pickup optical system 21 and the illumination optical systems 22 and the opening 24 and the air/water supply nozzle 25 in a first direction D1. Two illumination optical systems 22 are provided on both sides of the image pickup optical system 21 in a second direction orthogonal to the first direction. The measurement light-emitting unit 23 is provided between the image pickup optical system 21 and the air/water supply nozzle 25 in the first direction. Accordingly, since an air/water supply port of the air/water supply nozzle 25 faces the image pickup optical system 21 and the measurement light-emitting unit 23, both the image pickup optical system 21 and the measurement light-emitting unit 23 can be washed using the supply of air or the supply of water.

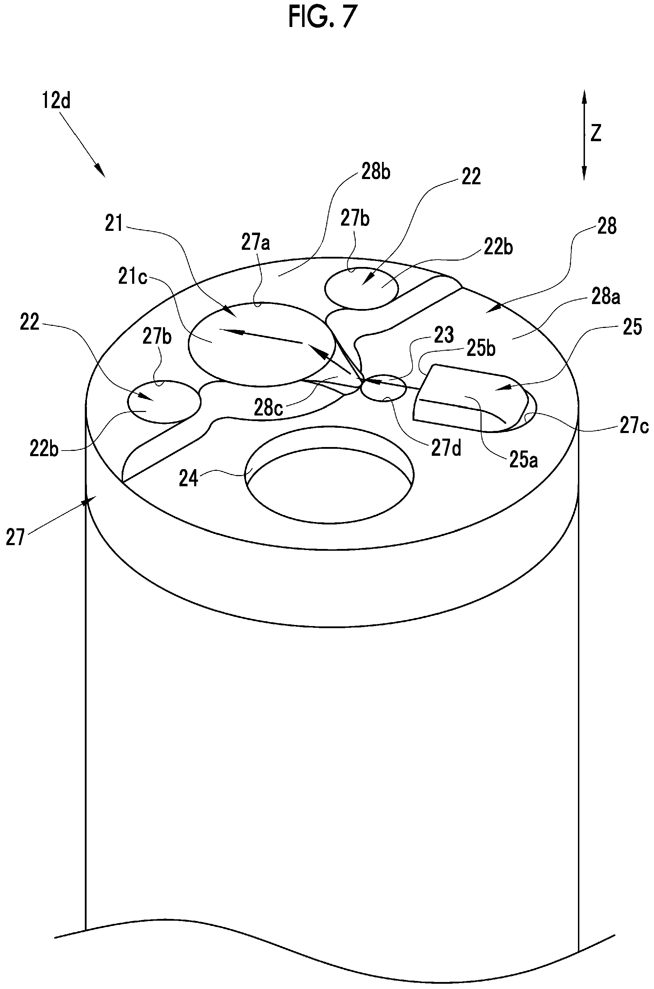

As shown in Fig. 7, a distal end cap 27 is mounted on the distal end part 12d. The distal end cap 27 is provided with a distal end surface 28. The distal end surface 28 includes a flat surface 28a, a flat surface 28b, and a guide surface 28c. The flat surface 28a is a flat surface orthogonal to an axial direction Z. The flat surface 28b is parallel to the flat surface 28a, and is positioned closer to the distal end side than the flat surface 28a in the axial direction Z. The guide surface 28c is disposed between the flat surface 28a and the flat surface 28b.

The flat surface 28b is provided with a through-hole 27a through which a distal end surface 21c of the image pickup optical system 21 is exposed to the outside and through-holes 27b through which distal end surfaces 22b of the pair of illumination optical systems 22 are exposed to the outside. The distal end surface 21c, the distal end surfaces 22b, and the flat surface 28b are disposed on the same plane.

Through-holes 27c and 27d are disposed on the flat surface 28a. The air/water supply nozzle 25 is exposed from the through-hole 27c. That is, the flat surface 28a is a mounting position for the air/water supply nozzle 25 in the axial direction Z. A jetting tube portion 25a is formed on the distal end side of the air/water supply nozzle 25. The jetting tube portion 25a is formed in the shape of a tube that protrudes in a direction bent by, for example, 90° from the proximal end portion of the air/water supply nozzle 25, and includes a jetting port 25b at the distal end thereof. The jetting tube portion 25a is disposed to protrude from the through-hole 27c to the distal end side in the axial direction Z.

The jetting port 25b is disposed toward the image pickup optical system 21. Accordingly, the air/water supply nozzle 25 jets a washing solution or gas, which is fluid, to the distal end surface 21c of the image pickup optical system 21 and the peripheral portion of the distal end surface 21c.

In a case where washing water or gas is jetted from the air/water supply nozzle 25 to the image pickup optical system 21, it is preferable that a flow speed F1 of the washing water at a position where the washing water reaches the image pickup optical system 21, that is, at an outer peripheral edge of the image pickup optical system 21 is 2 m/s or more and a flow speed F2 of the gas at the outer peripheral edge of the image pickup optical system 21 is 40 m/s or more. It is preferable that the flow speeds F1 and F2 satisfy the above-mentioned values regardless of the orientation of the distal end part 12d. For example, in a case where the air/water supply nozzle 25 is positioned immediately below the image pickup optical system 21 in a vertical direction, the flow speed of washing water or gas is reduced due to an influence of gravity but it is preferable that the flow speeds F1 and F2 satisfy the above-mentioned values even in this case.

The distal end surface of the measurement light-emitting unit 23, which is exposed from the through-hole 27d, is disposed on the flat surface 28a. That is, the mounting position for the air/water supply nozzle 25 and the distal end surface of the measurement light-emitting unit 23 are disposed at the same position in the axial direction Z. The measurement light-emitting unit 23 is disposed between the image pickup optical system 21 and the air/water supply nozzle 25 in a range where fluid is jetted from the air/water supply nozzle 25. In the present embodiment, the measurement light-emitting unit 23 is disposed in a region that connects the jetting port 25b of the air/water supply nozzle 25 to the outer peripheral edge of the image pickup optical system 21 in a case where the distal end surface 28 is viewed in the axial direction Z. Accordingly, in a case where fluid is jetted from the air/water supply nozzle 25 to the image pickup optical system 21, fluid can also be jetted to the measurement light-emitting unit 23 at the same time.

The guide surface 28c is formed of a continuous surface that connects the flat surface 28a to the flat surface 28b. The guide surface 28c is an inclined surface that is formed in a flat shape from a position where the guide surface 28c is in contact with the outer peripheral edge of the measurement light-emitting unit 23 to a position where the guide surface 28c is in contact with the outer peripheral edge of the image pickup optical system 21. Since the guide surface 28c is disposed in the range where fluid is jetted from the air/water supply nozzle, fluid is jetted to even the guide surface 28c in a case where fluid is jetted from the air/water supply nozzle 25. The fluid jetted to the guide surface 28c is diffused and blown to the image pickup optical system 21. In this case, the entire guide surface 28c may be included in the range where fluid is jetted from the air/water supply nozzle or only a part of the guide surface 28c may be included in the range where fluid is jetted from the air/water supply nozzle. In the present embodiment, the entire guide surface 28c is included in the region that connects the jetting port 25b of the air/water supply nozzle 25 to the outer peripheral edge of the image pickup optical system 21.

As shown in Fig. 8, the light source device 13 comprises a light source unit 30 and a light source processor 31. The light source unit 30 generates the illumination light or the special light that is used to illuminate the subject. The illumination light or the special light, which is emitted from the light source unit 30, is incident on a light guide LG, and the subject is irradiated with the illumination light or the special light through an illumination lens 22a. A white light source emitting white light, a plurality of light sources, which include a white light source and a light source emitting another color light (for example, a blue light source emitting blue light), or the like is used as a light source of the illumination light in the light source unit 30. Further, a light source, which emits broadband light including blue narrow-band light used to enhance superficial information about superficial blood vessels and the like, is used as a light source of the special light in the light source unit 30. Mixed white light in which at least one of violet light, blue light, green light, or red light is combined may be used as the illumination light. In this case, it is preferable that the illumination optical system 22 is optically designed to allow the irradiation range of green light to be wider than the irradiation range of red light.

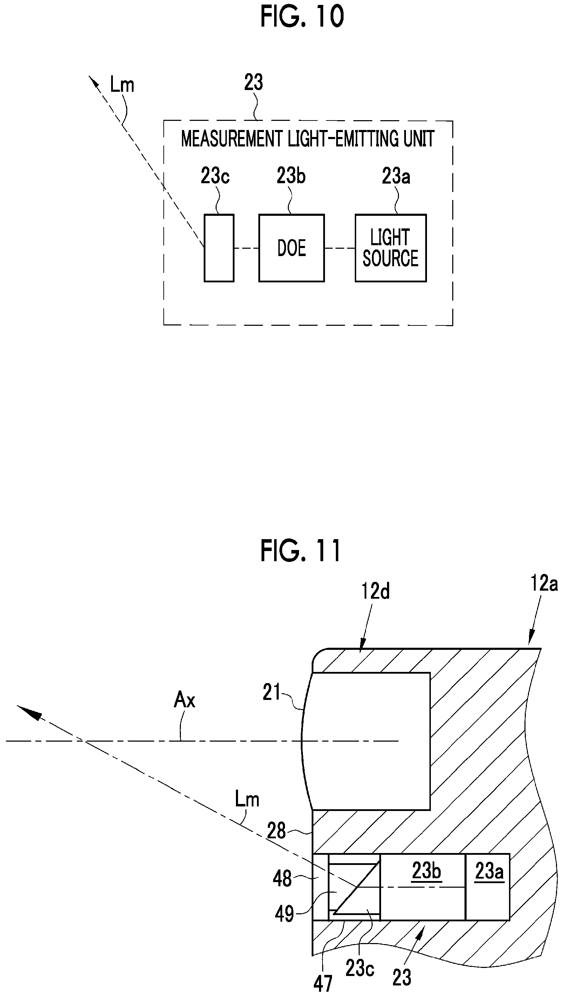

The light source processor 31 controls the light source unit 30 on the basis of an instruction given from a system controller 41. The system controller 41 not only gives an instruction related to light source control to the light source processor 31 but also controls a light source 23a (see Fig.10) of the measurement light-emitting unit 23. In the case of the normal observation mode, the system controller 41 performs a control to turn on the illumination light and to turn off the measurement light. In the case of the special observation mode, the system controller 41 performs a control to turn on the special light and to turn off the measurement light. In the case of the length measurement mode, the system controller 41 performs a control to turn on or off the illumination light or the measurement light.

The illumination optical system 22 includes the illumination lens 22a, and the object to be observed is irradiated with light, which is emitted from the light guide LG, through the illumination lens 22a. The image pickup optical system 21 includes an objective lens 21a, a zoom lens 21b, and an image pickup element 32. Light reflected from the object to be observed is incident on the image pickup element 32 through the objective lens 21a and the zoom lens 21b. Accordingly, the reflected image of the object to be observed is formed on the image pickup element 32.

The zoom lens 21b has an optical zoom function to enlarge or reduce the subject as a zoom function by moving between a telephoto end and a wide end. ON/OFF of the optical zoom function can be switched by the zoom operation part 12h (see Fig. 1) provided on the operation part 12b of the endoscope, and the subject can be enlarged or reduced at a specific magnification ratio in a case where the zoom operation part 12h is further operated in a state where the optical zoom function is turned on.

The image pickup element 32 is a color image pickup sensor, and picks up the reflected image of an object to be examined and outputs image signals. It is preferable that the image pickup element 32 is a charge coupled device (CCD) image pickup sensor, a complementary metal-oxide semiconductor (CMOS) image pickup sensor, or the like. The image pickup element 32 used in the present invention is a color image pickup sensor that is used to obtain red images, green images, and blue images corresponding to three colors of R (red), G (green), and B (blue). The red image is an image that is output from red pixels provided with red color filters in the image pickup element 32. The green image is an image that is output from green pixels provided with green color filters in the image pickup element 32. The blue image is an image that is output from blue pixels provided with blue color filters in the image pickup element 32. The image pickup element 32 is controlled by an image pickup controller 33.

Image signals output from the image pickup element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to a communication interface (I/F) 37 of the light source device 13 through a communication interface (I/F) 36.

In the processor device 14, programs related to various types of processing, control, or the like are incorporated into a program storage memory (not shown). The system controller 41 formed of an image control processor operates the programs incorporated into the program storage memory, so that the functions of a reception unit 38 connected to the communication interface (I/F) 37 of the light source device 13, a signal processing unit 39, and a display controller 40 are realized.

The reception unit 38 receives the image signals, which are transmitted from the communication I/F 37, and transmits the image signals to the signal processing unit 39. A memory, which temporarily stores the image signals received from the reception unit 38, is built in the signal processing unit 39, and the signal processing unit 39 processes an image signal group, which is a set of the image signals stored in the memory, to generate the subject image. The reception unit 38 may directly transmit control signals, which are related to the light source processor 31, to the system controller 41.

In a case where the endoscope 12 is set to the normal observation mode, signal assignment processing for assigning the blue image of the subject image to B channels of the display 15, assigning the green image of the subject image to G channels of the display 15, and assigning the red image of the subject image to R channels of the display 15 is performed in the signal processing unit 39. As a result, a color subject image is displayed on the display 15. The same signal assignment processing as that in the normal observation mode is performed even in the length measurement mode.

On the other hand, in a case where the endoscope 12 is set to the special observation mode, the red image of the subject image is not used for the display of the display 15, the blue image of the subject image is assigned to the B channels and the G channels of the display 15, and the green image of the subject image is assigned to the R channels of the display 15 in the signal processing unit 39. As a result, a pseudo-color subject image is displayed on the display 15. Further, in a case where the endoscope 12 is set to the length measurement mode, the signal processing unit 39 transmits a subject image, which includes the irradiation position of the measurement light, to a data transmission/reception unit 43. The data transmission/reception unit 43 transmits data, which are related to the subject image, to the augmented processor device 17. The data transmission/reception unit 43 can receive data and the like from the augmented processor device 17. The received data can be processed by the signal processing unit 39 or the system controller 41.

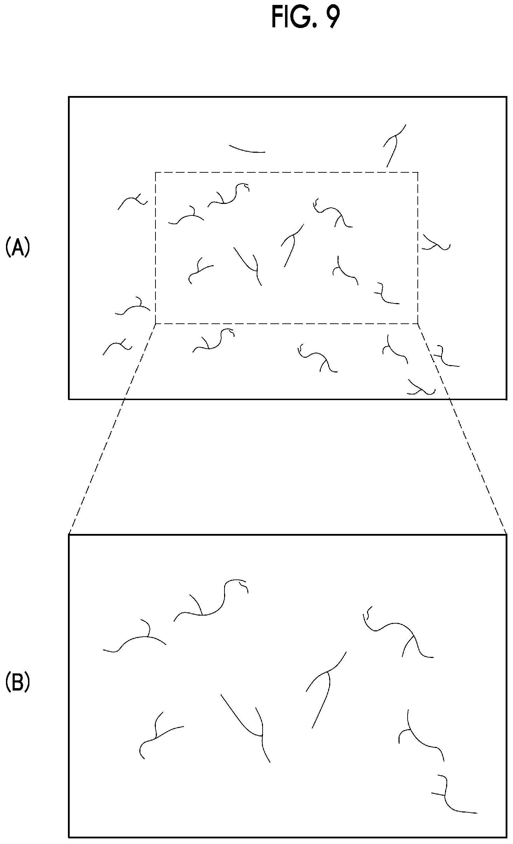

In a case where a digital zoom function is set to ON as a zoom function by the user interface 16, the signal processing unit 39 cuts out a portion of the subject image and enlarges or reduces the cut portion. As a result, the subject is enlarged or reduced at a specific magnification. (A) of Fig. 9 shows a subject image obtained in a state where the digital zoom function is turned off and (B) of Fig. 9 shows a subject image obtained in a state where the digital zoom function is turned on so that a central portion of the subject image shown in (A) of Fig. 9 is cut out and enlarged. In a case where the digital zoom function is turned off, the enlargement or reduction of the subject using the cutout of the subject image is not performed.

The display controller 40 causes the display 15 to display the subject image that is generated by the signal processing unit 39. The system controller 41 performs various controls on the endoscope 12, the light source device 13, the processor device 14, and the augmented processor device 17. The system controller 41 performs the control of the image pickup element 32 via the image pickup controller 33 provided in the endoscope 12. The image pickup controller 33 also performs the control of the CDS/AGC circuit 34 and the A/D converter 35 in accordance with the control of the image pickup element 32.

The augmented processor device 17 receives data, which are transmitted from the processor device 14, by a data transmission/reception unit 44. A signal processing unit 45 performs processing related to the length measurement mode on the basis of the data that are received by the data transmission/reception unit 44. Specifically, the signal processing unit 45 performs processing for determining the size of a virtual scale from the subject image including the irradiation position of the measurement light and superimposing and displaying the determined virtual scale on the subject image. A display controller 46 causes the augmented display 18 to display the subject image on which the virtual scale is superimposed and displayed. The data transmission/reception unit 44 can transmit data and the like to the processor device 14.

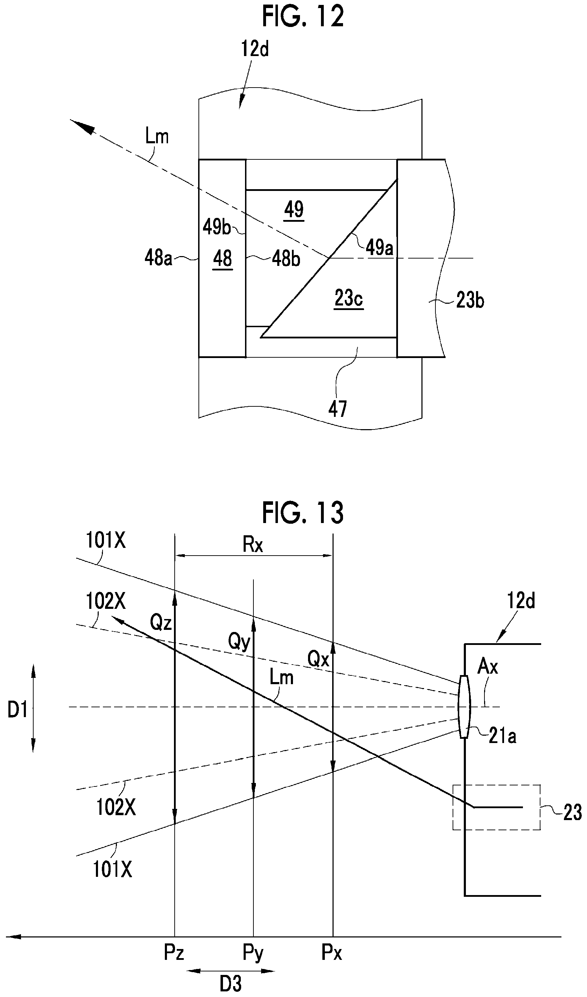

As shown in Fig. 10, the measurement light-emitting unit 23 emits measurement light obliquely with respect to an optical axis Ax (see Fig. 13) of the image pickup optical system 21. The measurement light-emitting unit 23 comprises a light source 23a, a diffractive optical element (DOE) 23b, a prism 23c, and an emission part 23d. The light source 23a is to emit light having a color that can be detected by pixels of the image pickup element 32 (specifically, visible light), and includes a light emitting element, such as a laser light source (laser diode (LD)) or a light emitting diode (LED), and a condenser lens that condenses light emitted from the light emitting element. The light source 23a is provided on a scope electric board (not shown). The scope electric board is provided in the distal end part 12d of the endoscope, and receives power supplied from the light source device 13 or the processor device 14 and supplies power to the light source 23a. The light source 23a is provided in the distal end part 12d of the endoscope, but may be provided in a connector that is used to connect the endoscope 12 to the processor device 14. Even in this case, members (the diffractive optical element DOE 23b, the prism 23c, and the emission part 23d) of the measurement light-emitting unit 23 except for the light source 23a are provided in the distal end part 12d of the endoscope.

For example, red (the color of beam light) laser light having a wavelength of 600 nm or more and 650 nm or less is used as the light emitted from the light source 23a in the present embodiment, but light having other wavelength ranges, for example, green light having a wavelength of 495 nm or more and 570 nm or less may be used. The light source 23a is controlled by the system controller 41, and emits light on the basis of an instruction given from the system controller 41. The DOE 23b converts the light, which is emitted from the light source, into the measurement light that is used to obtain measurement information. It is preferable that the amount of measurement light is adjusted from a standpoint of protecting a human body, eyes, and internal organs and is adjusted to the amount of light enough to cause halation (pixel saturation) in the observation range of the endoscope 12.

The prism 23c is an optical member that is used to change the travel direction of the measurement light converted by the DOE 23b. The prism 23c changes the travel direction of the measurement light such that the measurement light intersects with the visual field of the image pickup optical system 21 including the objective lens 21a. The details of the travel direction of the measurement light will also be described later. The subject is irradiated with measurement light emitted from the prism 23c.

As shown in Fig. 11, the measurement light-emitting unit 23 is housed in a housing portion 47 for a measurement light-emitting unit that is provided in the distal end part 12d of the endoscope. The housing portion 47 for a measurement light-emitting unit includes a hole portion corresponding to the size of the measurement light-emitting unit 23. The housing portion 47 for a measurement light-emitting unit is closed by a transparent lid 48. The transparent lid 48 has the shape of a transparent plate, and one end surface of the transparent lid 48 is formed of a flat portion 48a. The transparent lid 48 is disposed such that the flat portion 48a is flush with the distal end surface 28 of the distal end part 12d. Since the transparent lid 48 flush with the distal end surface 28 is provided, foreign substances or the like that may block the measurement light to be emitted are not caught.

As shown in Fig. 12, a prism 49 is disposed between the transparent lid 48 and the prism 23c. The prism 49 includes a first close contact surface 49a and a second close contact surface 49b, the first close contact surface 49a is in close contact with the prism 23c, and the second close contact surface 49b is in close contact with the transparent lid 48. Gas is eliminated from a space between the transparent lid 48 and the prism 23c by the prism 49, so that an airtight state is made. Since the airtight state is made in this way, condensation can be prevented. That is, it is possible to prevent problems, such as the attenuation, diffusion, convergence, and refraction of measurement light caused by condensation, from occurring.

An example in which "n1<n2" is satisfied and a light-emitting surface of the prism 23c is inclined toward the optical axis Ax in a case where a refractive index of the prism 23c is denoted by "n1" and a refractive index of the prism 49 is denoted by "n2" has been described, but configuration contrary to this example may be provided. "n1>n2" may be satisfied and the light-emitting surface of the prism 23c may be provided on a side opposite to the optical axis Ax. However, since there is a possibility that light is totally reflected by the light-emitting surface of the prism 23c in this case, it is necessary to impose a limitation on the light-emitting surface of the prism 23c.

The prism 23c may be formed of an auxiliary measurement slit, which is formed at the distal end part 12d of the endoscope, instead of being formed of an optical member. Further, in a case where the prism 23c is formed of an optical member, it is preferable that an anti-reflection coating (AR coating) (anti-reflection portion) is provided on an emission surface of the prism 23c. The reason why the anti-reflection coating is provided as described above is that it is difficult for an irradiation position detector 61 to be described later to recognize the position of a spot SP formed on the subject by the measurement light in a case where the measurement light is reflected without being transmitted through the emission surface of the prism 23c and a ratio of the measurement light with which a subject is irradiated is reduced.

The measurement light-emitting unit 23 has only to be capable of emitting the measurement light to the visual field of the image pickup optical system 21. For example, the light source 23a may be provided in the light source device and light emitted from the light source 23a may be guided to the DOE 23b by optical fibers. Further, the prism 23c may not be used and the orientations of the light source 23a and the DOE 23b may be inclined with respect to the optical axis Ax of the image pickup optical system 21 so that the measurement light is emitted in a direction crossing the visual field of the image pickup optical system 21.

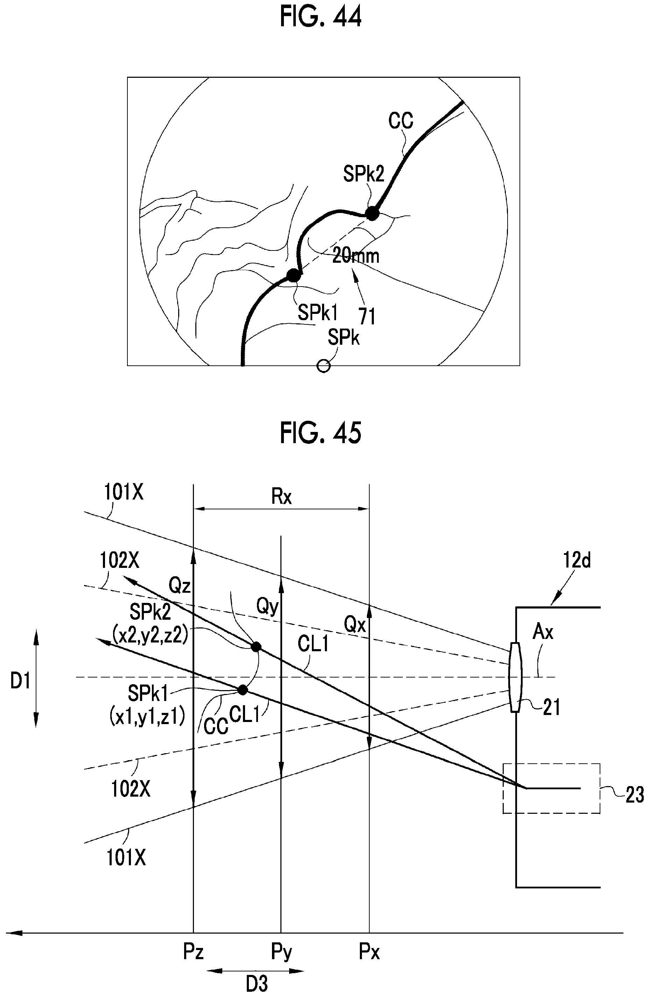

With regard to the travel direction of the measurement light, the measurement light is emitted in a state where an optical axis Lm of the measurement light intersects with the optical axis Ax of the image pickup optical system 21 as shown in Fig. 13. In a case where the subject can be observed in a range Rx of an observation distance, it is understood that the positions (points where the respective arrows Qx, Qy, and Qz intersect with the optical axis Ax) of the spot SP, which is formed on the subject by the measurement light, in image pickup ranges (shown by arrows Qx, Qy, and Qz) at a near end Px, an intermediate vicinity Py, and a far end Pz of the range Rx are different from each other. The image pickup angle of view of the image pickup optical system 21 is represented by a region between two solid lines 101X, and measurement is performed in a central region (a region between two dotted lines 102X), in which an aberration is small, of this image pickup angle of view. Further, a third direction D3 is a direction that is orthogonal to the first direction D1 and the second direction D2 (the same applies to Fig. 45).

Since the measurement light is emitted in a state where the optical axis Lm of the measurement light intersects with the optical axis Ax as described above, the size of the subject can be measured from the movement of the position of the spot with respect to a change in observation distance. Then, the image of the subject illuminated with the measurement light is picked up by the image pickup element 32, so that a subject image including the spot SP is obtained. In the subject image, the position of the spot SP varies depending on a relationship between the optical axis Ax of the image pickup optical system 21 and the optical axis Lm of the measurement light and an observation distance. The number of pixels showing the same actual size (for example, 5 mm) is increased in the case of a short observation distance, and the number of pixels showing the same actual size is reduced in the case of a long observation distance.

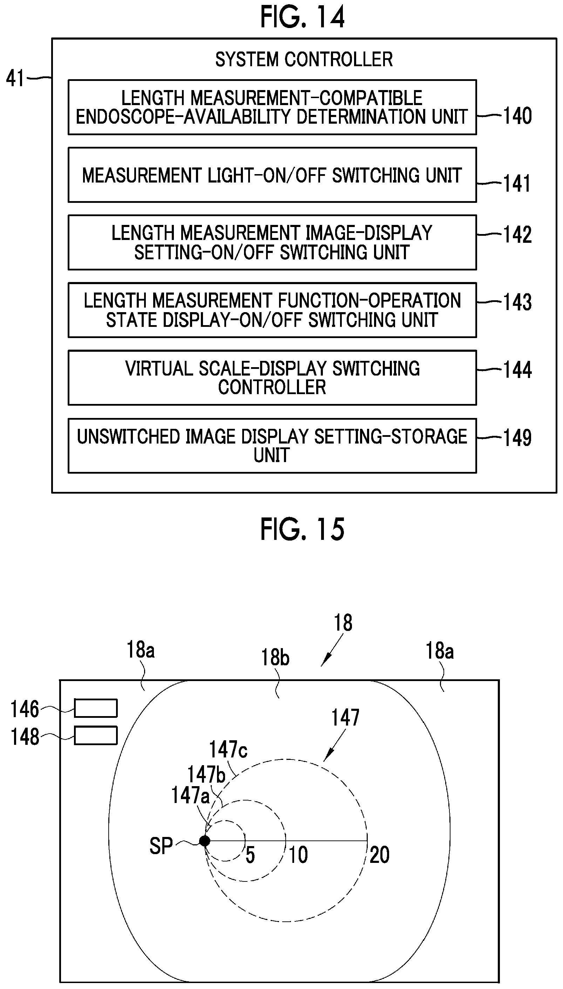

As shown in Fig. 14, the system controller 41 comprises a length measurement-compatible endoscope-availability determination unit 140, a measurement light-ON/OFF switching unit 141, a length measurement image-display setting-ON/OFF switching unit 142, a length measurement function-operation state display-ON/OFF switching unit 143, a virtual scale-display switching controller 144, and an unswitched image display setting-storage unit 149.

The length measurement-compatible endoscope-availability determination unit 140 determines whether or not the endoscope 12 is a length measurement-compatible endoscope in a case where the endoscope 12 is connected to the processor device 14. In a case where the endoscope 12 is a length measurement-compatible endoscope, the switching of a mode to the length measurement mode is enabled. A length measurement-compatible endoscope refers to an endoscope that can emit and receive measurement light and causes the augmented display 18 (or the display 15) to display a length measurement image displaying a virtual scale based on the measurement light. The length measurement-compatible endoscope-availability determination unit 140 comprises a scope ID table (not shown) in which a scope ID given to the endoscope 12 and a flag for the presence or absence of a length measurement-compatible endoscope (for example, the flag is set to "1" in the case of a length measurement-compatible endoscope and is set to "0" in the cases of other endoscopes) are associated with each other. Further, in a case where the endoscope 12 is connected, the length measurement-compatible endoscope-availability determination unit 140 reads out the scope ID of the endoscope. The length measurement-compatible endoscope-availability determination unit 140 determines whether or not the scope ID read out is a scope ID of a length measurement-compatible endoscope with reference to the flag of the scope ID table.

The measurement light-ON/OFF switching unit 141 controls the light source 23a to switch the turning-on (ON) or turning-off (OFF) the measurement light. The length measurement image-display setting-ON/OFF switching unit 142 allows the various image display settings in the length measurement mode, such as the display settings (a color tone and the like) of a length measurement image, to be available (ON) or unavailable (OFF) via the user interface 16 or the like. The virtual scale-display switching controller 144 switches the virtual scale to any one of display (ON), non-display (OFF), or the change of a display aspect on the augmented display 18.

In a state where the switching of a mode to the length measurement mode is enabled, the system controller 41 performs at least one of the switching of ON or OFF of measurement light, the switching of ON or OFF of length measurement image-display settings, the switching of ON or OFF of length measurement function-operation state display, and the switching of ON or OFF of the display of a virtual scale or display aspect change of the virtual scale by an operation for switching a mode to the length measurement mode using the observation mode selector switch 12f.

For example, it is preferable that the system controller 41 switches the measurement light to ON, switches the length measurement image-display settings to ON, switches the length measurement function-operation state display to ON, and switches the display of a virtual scale to ON by an operation for switching a mode to the length measurement mode. On the other hand, it is preferable that the system controller 41 switches the measurement light to OFF, switches the length measurement image-display settings to OFF, switches the length measurement function-operation state display to OFF, and switches the display of a virtual scale to OFF by an operation for switching the length measurement mode to the other mode (the normal observation mode or the special observation mode).

It is preferable that the length measurement function-operation state display is displayed in an accessory information display region 18a of the augmented display 18 by a scale display icon 146 as shown in Fig. 15. The scale display icon 146 is displayed according to an operation for switching a mode to the length measurement mode, and the scale display icon 146 is hidden according to an operation for switching the length measurement mode to the other mode. Further, it is preferable that a virtual scale 147 is displayed in an observation image display region 18b of the augmented display 18. The display aspect of the virtual scale 147 is changed by the virtual scale-display switching controller 144.

The virtual scale 147 comprises a virtual scale 147a of 5 mm, a virtual scale 147b of 10 mm, and a virtual scale 147c of 20 mm. Each of the virtual scales 147a, 147b, and 147c includes a circular scale (displayed with a dotted line) and a line segment scale (displayed with a solid line). "5" of the virtual scale 147a indicates a scale of 5 mm, "10" of the virtual scale 147b indicates a scale of 10 mm, and "20" of the virtual scale 147c indicates a scale of 20 mm.

The display aspect of a virtual scale is changed according to a selection from a plurality of predetermined scale patterns. Examples of a plurality of scale patterns include a scale pattern that is formed of a combination of two virtual scales 147b and 147c each of which includes a circular scale and a line segment scale, a scale pattern that is formed of a combination of three virtual scales each of which includes only a line segment among the virtual scales 147a, 147b, and 147c, and the like in addition to a scale pattern that is formed of a combination of the three virtual scales 147a, 147b, and 147c each of which includes a circular scale and a line segment scale as shown in Fig. 15. The scale pattern is represented by one selected from a plurality of scale sizes and a plurality of scale shapes, such as a circular scale and a line segment scale, or a combination of a plurality of scale sizes and scale shapes selected from them.

Further, in a case where the length measurement image-display settings are switched to ON, it is preferable that image display settings before the switching of a mode to the length measurement mode are stored in the unswitched image display setting-storage unit 149. For example, in a case where an observation mode before the switching of a mode to the length measurement mode is the normal observation mode, it is preferable that image display settings in the normal observation mode set in the signal processing unit 39 are stored in the unswitched image display setting-storage unit 149. Further, in a case where the length measurement image-display settings are switched to OFF, it is preferable that image display settings are switched to the image display settings stored in the unswitched image display setting-storage unit 149. For example, in a case where image display settings in the normal observation mode are stored in the unswitched image display setting-storage unit 149, the signal processing unit 39 sets image display settings to the image display settings in the normal observation mode, which are stored in the unswitched image display setting-storage unit 149, according to the switching of a mode to the normal observation mode.

On the other hand, in a case where conditions in switching a mode are not satisfied in an operation for switching a mode to the length measurement mode, the system controller 41 prohibits the switching of the measurement light to ON, prohibits the switching of the length measurement image-display settings to ON, prohibits the switching of the length measurement function-operation state display to ON, and prohibits the switching of the display of a virtual scale to ON. The conditions in switching a mode are conditions that are suitable for the execution of the length measurement mode under setting conditions related to the endoscope 12, the light source device 13, the processor device 14, and the augmented processor device 17. It is preferable that the conditions in switching a mode are conditions not corresponding to the following prohibition setting conditions. In a case where the conditions in switching a mode are not satisfied, it is preferable that length measurement function-operation state-unavailability display showing that the virtual scale 147 is not being displayed on the augmented display 18 is displayed (ON) instead of prohibiting the displaying of the scale display icon 146. It is preferable that the length measurement function-operation state-unavailability display is displayed in the accessory information display region 18a by a scale non-display icon 148.

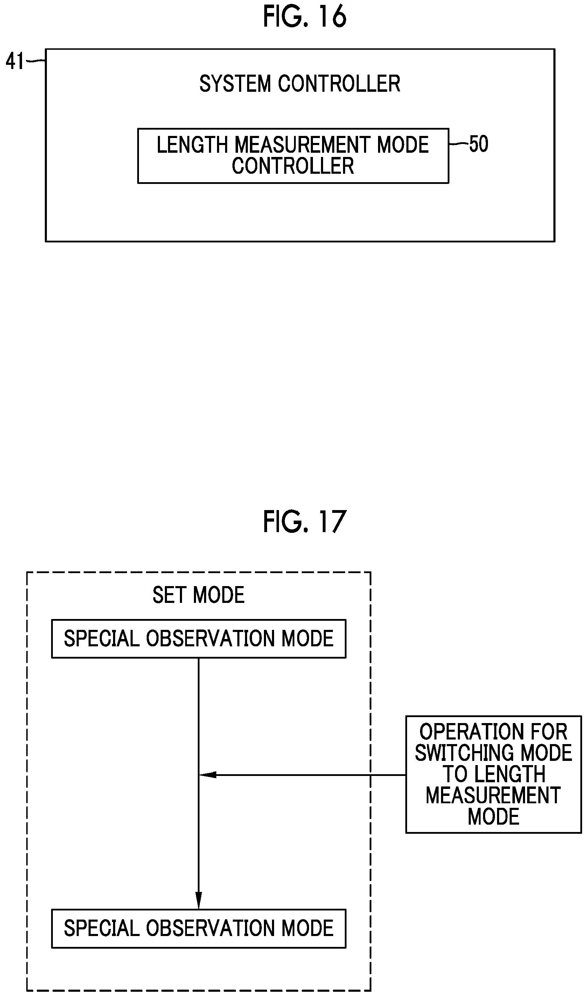

As shown in Fig. 16, the system controller 41 is provided with a length measurement mode controller 50 that performs a control related to whether or not the length measurement mode can be executed and the like. The length measurement mode controller 50 performs: at least one of a first control that prohibits the switching of a mode to the length measurement mode in a case where an operation for switching a mode to the length measurement mode is performed by the observation mode selector switch 12f and currently set setting conditions related to the endoscope 12, the light source device 13, and the processor device 14 correspond to predetermined prohibition setting conditions; a second control that disables a setting change operation in a case where the setting change operation for the setting conditions is performed in the length measurement mode by the user interface 16 and the setting conditions to be changed by the setting change operation correspond to the prohibition setting conditions; or a third control that automatically switches a mode to the other mode from the length measurement mode in a case where the setting change operation for the setting conditions is performed in the length measurement mode by the user interface 16 and the setting conditions to be changed by the setting change operation correspond to the prohibition setting conditions.

The setting condition related to the light source device 13 includes an illumination condition for the illumination light that is used in the normal observation mode or the length measurement mode, an illumination condition for the special light that is used in the special observation mode, or an illumination condition for the measurement light that is used in the length measurement mode. The illumination condition includes, for example, the amount of illumination light and the like. The setting condition related to the endoscope 12 includes an image pickup condition related to the image pickup of the subject. The image pickup condition includes, for example, a shutter speed and the like. The setting condition related to the processor device 14 includes a processing condition, such as image processing related to the subject image. The processing condition includes, for example, color balance, brightness correction, various types of enhancement processing, and the like. In the length measurement mode, it is preferable that the detection of the position of the spot SP is optimized and the setting conditions are set to setting conditions (the amount of illumination light, a shutter speed, color balance, brightness correction, and various types of enhancement processing) satisfying visibility in the user's dimensional measurement.

The prohibition setting conditions include a first prohibition setting condition that causes the detection of the irradiation position of the measurement light from the subject image in the length measurement mode to be hindered, and a second prohibition setting condition that causes the accurate display of a virtual scale corresponding to an observation distance in a length measurement image to be hindered. The first prohibition setting condition includes, for example, the special observation mode, brightness enhancement or red emphasis in the subject image, and the like. Since a red image used to detect the spot SP or the like in the length measurement mode is not used for the display of an image in the special observation mode, it is difficult to detect the irradiation position of the measurement light. It is preferable that the brightness of the subject image is set to be low and redness is suppressed in the length measurement mode as compared to the normal observation mode or the special observation mode.

Further, for example, the use (ON) of a zoom function, such as the optical zoom function or the digital zoom function, is included as the second prohibition setting condition. The reason for this is that it is difficult for the virtual scale to be displayed to correspond to an observation distance in a case where the zoom function is turned on since a virtual scale displayed in a measurement image is determined according to the position of the spot SP and is not determined according to the magnification of the zoom function.

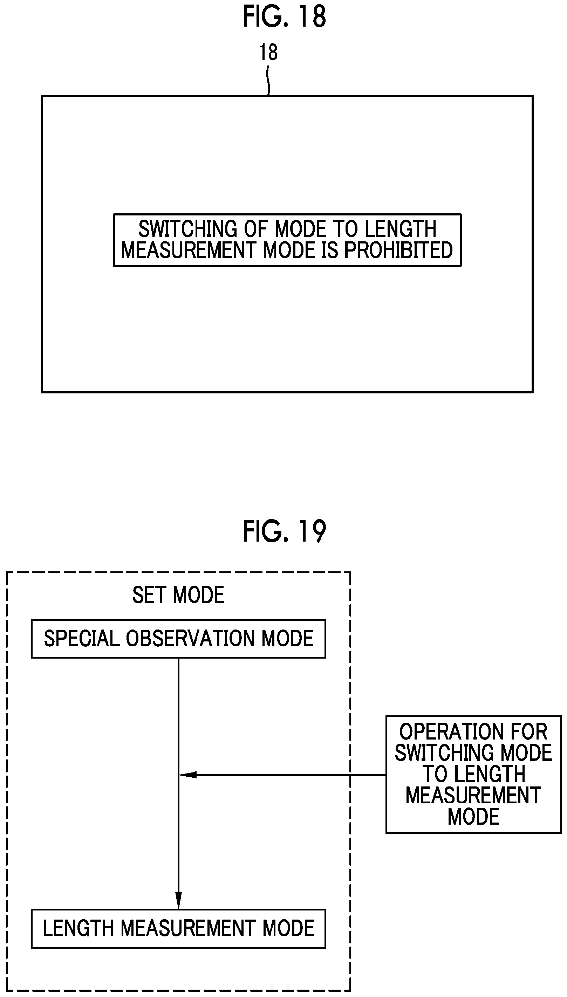

For example, in a case where an operation for switching a mode to the length measurement mode is performed by the observation mode selector switch 12f in a state where the endoscope 12 is set to the special observation mode, the length measurement mode controller 50 performs the first control that prohibits the switching of a mode to the length measurement mode and maintains the state of the special observation mode as shown in Fig. 17. In a case where the first control is performed, the length measurement mode controller 50 causes the augmented display 18 to display a message that notifies that the switching of a mode to the length measurement mode is prohibited as shown in Fig. 18 (warning sound may be made). The length measurement mode controller 50 may perform a control to cancel the special observation mode and to switch a mode to the length measurement mode as shown in Fig. 19 instead of prohibiting the switching of a mode to the length measurement mode.

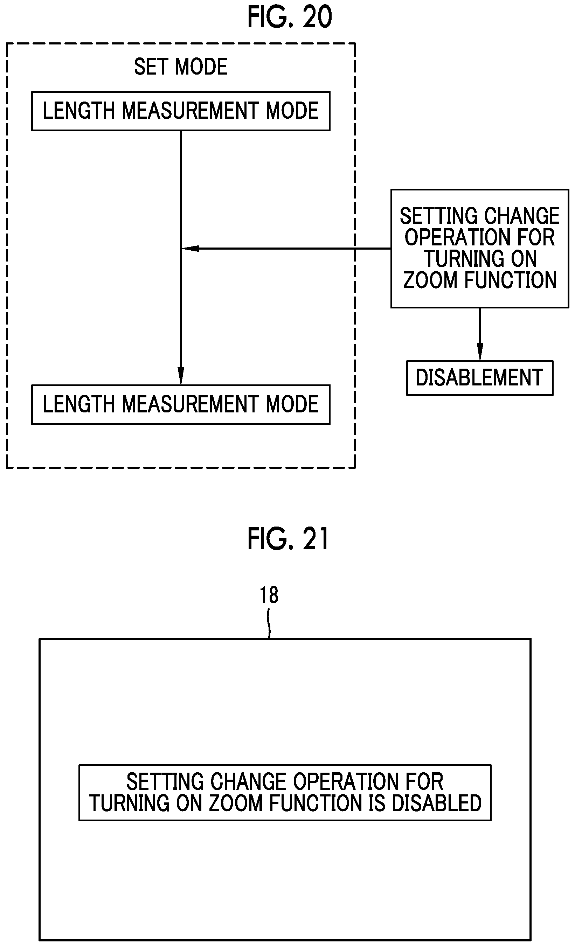

Further, in a case where a setting change operation for turning on the zoom function by the operation of the zoom operation part 12h is performed in the length measurement mode, the length measurement mode controller 50 performs the second control that disables the setting change operation for turning on the zoom function as shown in Fig. 20. In a case where the second control is performed, the length measurement mode controller 50 causes the augmented display 18 to display a message that notifies that the setting change operation for turning on the zoom function is disabled as shown in Fig. 21 (warning sound may be made).

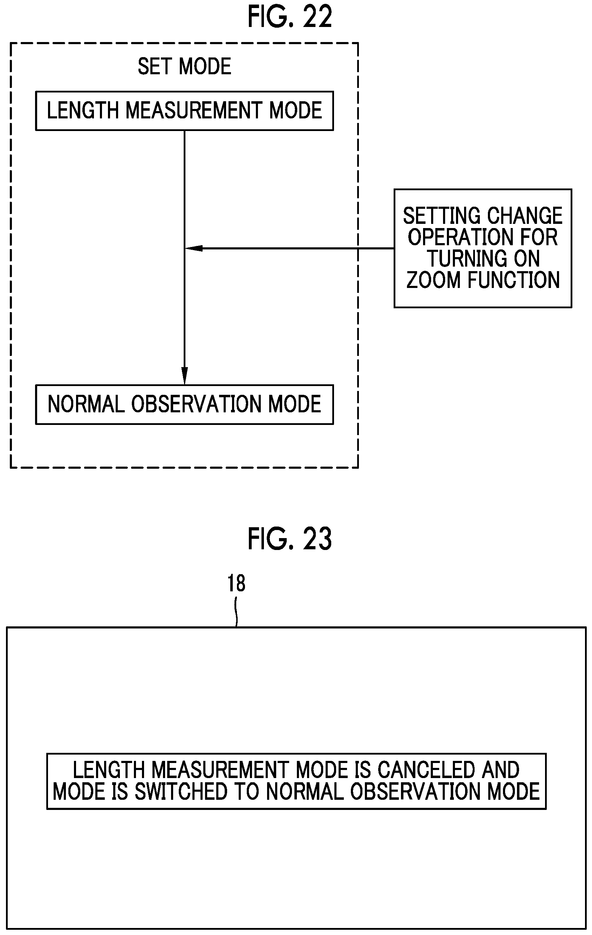

Furthermore, in a case where a setting change operation for turning on the zoom function by the operation of the zoom operation part 12h is performed in the length measurement mode, the length measurement mode controller 50 performs the third control that cancels the length measurement mode and switches a mode to the normal observation mode as the other mode as shown in Fig. 22. In a case where the third control is performed, the length measurement mode controller 50 causes the augmented display 18 to display a message that notifies that the length measurement mode is canceled (the virtual scale is not displayed) and is switched to the normal observation mode as shown in Fig. 23 (warning sound may be made). In a case where the third control is performed, the setting change operation for turning on the zoom function is enabled and the subject in the subject image is enlarged or reduced by the zoom function.

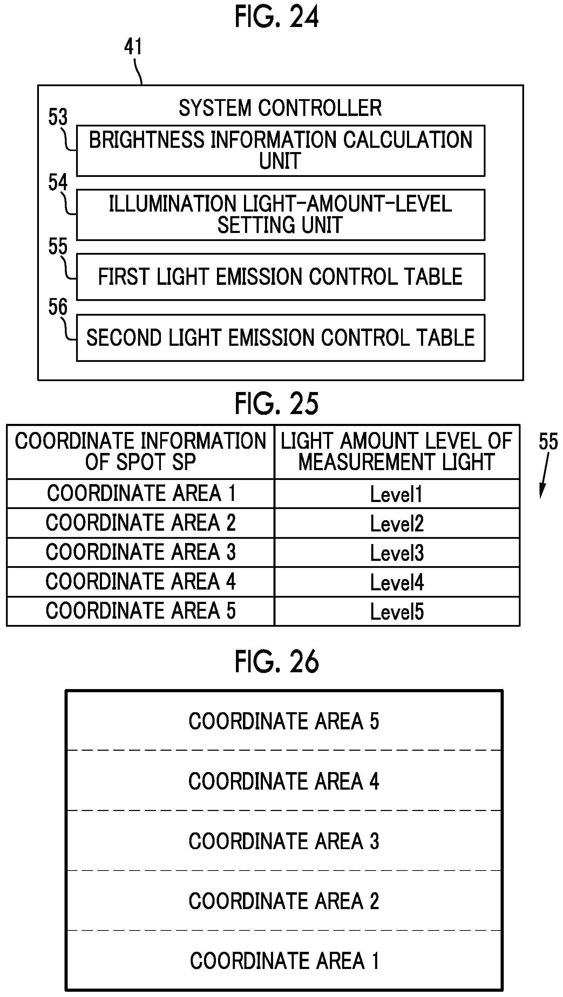

As shown in Fig. 24, the system controller 41 may be provided with a brightness information calculation unit 53, an illumination light-amount-level setting unit 54, a first light emission control table 55, and a second light emission control table 56. The brightness information calculation unit 53 calculates brightness information about the brightness of the subject on the basis of an image obtained in the normal observation mode or a first captured image obtained in the length measurement mode (an image based on illumination light and measurement light). The illumination light-amount-level setting unit 54 sets a light amount level of illumination light on the basis of the brightness information. Five levels of Level 1, Level 2, Level 3, Level 4, and Level 5 are set as the light amount level of illumination light. Information about the light amount level of illumination light is sent to the light source processor 31. The light source processor 31 controls the light source unit 30 so that the amount of illumination light is set to the light amount level of illumination light.

The first light emission control table 55 is used for the control of the amount of measurement light, and stores a first relationship between the coordinate information of the spot SP and the light amount level of measurement light. Specifically, as shown in Fig. 25, the light amount levels of measurement light, that is, Level 1, Level 2, Level 3, Level 4, and Level 5 are determined for five coordinate areas to which the coordinate information of the spot SP belongs, respectively. The system controller 41 specifies a light amount level, which corresponds to a coordinate area to which the position of the spot SP belongs, with reference to the first light emission control table 55. The system controller 41 controls the light source 23a to control the amount of measurement light so that the amount of measurement light is set to the specified light amount level. Which one of the first light emission control table 55 and the second light emission control table 56 is to be used for the control of the amount of measurement light is properly set via the operation of the user interface 16.

As shown in Fig. 26, Coordinate area 1 is an area that is set on the lowest side in the first captured image, and the spot SP is present at a position corresponding to the shortest observation distance in a case where the spot SP belongs to Coordinate area 1. Accordingly, the lowest Level 1 is assigned to Coordinate area 1 as the light amount level of measurement light. Further, Coordinate area 2 is an area that is set above Coordinate area 1, and the spot SP is present at a position corresponding to an observation distance long as compared to the case of Coordinate area 1 in a case where the spot SP belongs to Coordinate area 2. Accordingly, Level 2 higher than Level 1 is assigned as the light amount level of measurement light. The moving direction of the spot SP is changed depending on a direction in which the optical axis Ax of the image pickup optical system 21 and the optical axis Lm of the measurement light intersect with each other.

Likewise, Coordinate area 3 is provided above Coordinate area 2. In a case where the spot SP belongs to Coordinate area 3, the spot SP is present at a position corresponding to an observation distance long as compared to the case of Coordinate area 2. Accordingly, Level 3 higher than Level 2 is assigned as the light amount level of measurement light. Further, Coordinate area 4 is provided above Coordinate area 3. In a case where the spot SP belongs to Coordinate area 4, the spot SP is present at a position corresponding to an observation distance long as compared to the case of Coordinate area 3. Accordingly, Level 4 higher than Level 3 is assigned as the light amount level of measurement light. Furthermore, Coordinate area 5 is an area that is set on the highest side. In a case where the spot SP belongs to Coordinate area 5, the spot SP is present at a position corresponding to an observation distance longest as compared to the cases of the other coordinate areas 1 to 4. Accordingly, the highest Level 5 is assigned as the light amount level of measurement light.

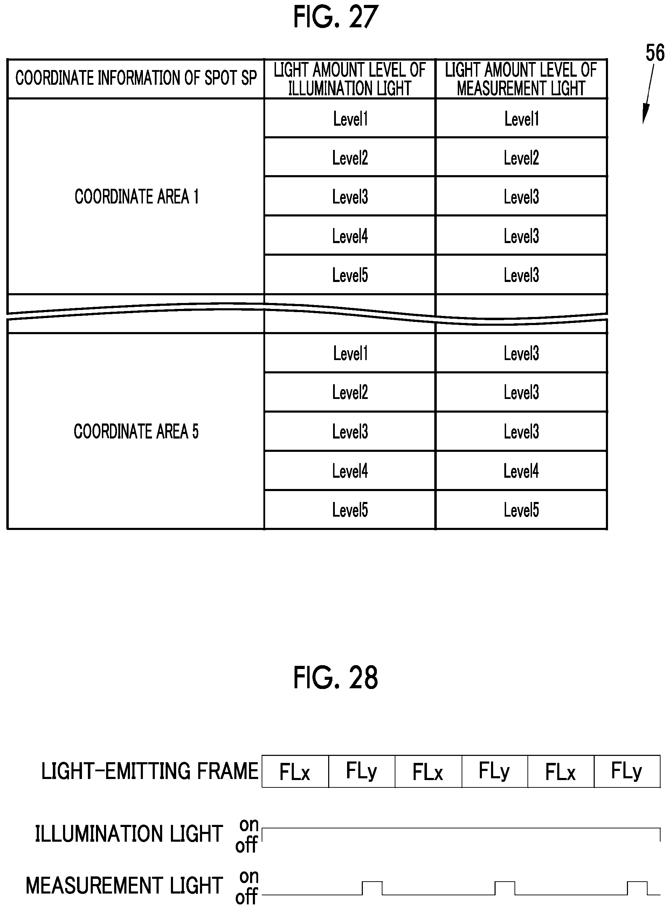

The second light emission control table 56 is used for the control of the amount of measurement light, and stores a second relationship between the coordinate information of the spot SP and the light amount level of illumination light and the light amount level of measurement light. Specifically, as shown in Fig. 27, the light amount levels of measurement light are determined for five coordinate areas to which the coordinate information of the spot SP belongs and the light amount levels of illumination light, that is, Level 1, Level 2, Level 3, Level 4, and Level 5, respectively. For example, in a case where the spot SP belongs to Coordinate area 1 and the light amount level of illumination light is Level 3, Level 3 is assigned as the light amount level of measurement light.

The system controller 41 specifies the light amount level of measurement light from Coordinate area to which the position of the spot SP belongs and the light amount level of illumination light with reference to the second light emission control table 56. The system controller 41 controls the light source 23a to control the amount of measurement light so that the amount of measurement light is set to the specified light amount level.

In the second light emission control table 56, the light amount level of illumination light and the light amount level of measurement light are set to ratios that are required to specify the position of the spot SP. The reason for this is that it is difficult to specify the position of the spot SP since the contrast of the spot SP is lowered in a case where a ratio of the amount of illumination light to the amount of measurement light is not proper.

In the length measurement mode, the light source processor 31 continuously emits illumination light, which is used to illuminate the entire object to be observed, but emits the measurement light in the form of a pulse. Accordingly, as shown in Fig. 28, exclusive illumination light-emitting frames FLx in which measurement light is not emitted and illumination light is emitted alone and measurement light-emitting frames FLy in which illumination light and measurement light are emitted are included as frames in which light is emitted in the length measurement mode. Further, in the length measurement mode, the position of the spot SP is detected from a first captured image obtained in the measurement light-emitting frame FLy and a virtual scale is displayed in a second captured image obtained in the exclusive illumination light-emitting frame FLx. A solid line shown in a portion of Fig. 28 corresponding to illumination light or measurement light shows a state where light is emitted in a certain frame. A period in which a solid line is positioned at a portion corresponding to "on" is a period in which illumination light or measurement light is emitted, and a period in which a solid line is positioned at a portion corresponding to "off" is a period in which illumination light or measurement light is not emitted.

The patterns of light emission and image pickup in the length measurement mode are as follows. A first pattern is a case where a global shutter type image pickup element (CCD), which performs exposure and reads out electric charges in the respective pixels in the same timing to output image signals, is used as the image pickup element 32. Further, in the first pattern, measurement light is emitted at intervals of two frames as a specific frame interval.

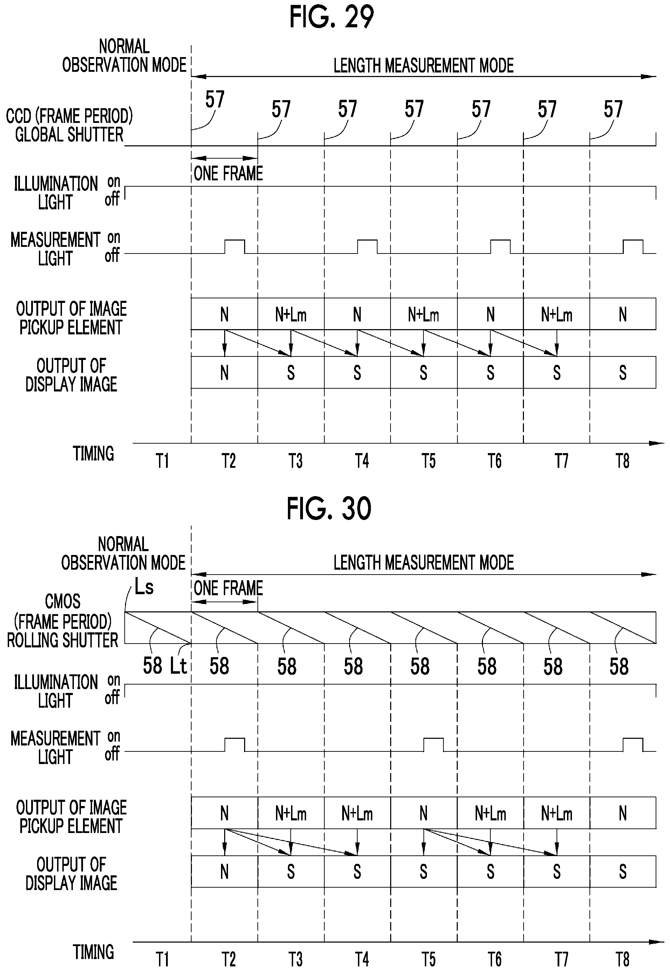

In the first pattern, as shown in Fig. 29, electric charges are simultaneously read out (global shutter) in switching between the normal observation mode and the length measurement mode (in switching from a timing T1 to a timing T2) on the basis of exposure to illumination light in the timing T1 in the normal observation mode, so that a second captured image N including only components of illumination light is obtained. The second captured image N is displayed on the augmented display 18 in the timing T2. With regard to "CCD (frame period) global shutter" shown in Fig. 29, a rising line 57 rising in a vertical direction in switching from the timing T1 to the timing T2 indicates that a global shutter is performed. The same applies to the other rising lines 57.

Further, illumination light and measurement light are emitted in the timing T2. Electric charges are simultaneously read out in switching from the timing T2 to a timing T3 on the basis of exposure to illumination light and measurement light in this timing T2, so that a first captured image N+Lm including components of illumination light and measurement light is obtained. The position of the spot SP is detected on the basis of this first captured image N+Lm. A virtual scale corresponding to the position of the spot SP is displayed in the second captured image N displayed in the timing T2. Accordingly, a length measurement image S in which a virtual scale is displayed in the second captured image N displayed in the timing T2 is displayed in the timing T3.

The second captured image N obtained in the timing T2 (first timing) is displayed on the augmented display 18 not only in the timing T2 but also in the timing T3. That is, the second captured image obtained in the timing T2 is continuously displayed for two frames until a timing T4 (second timing) in which the next second captured image is obtained (the same subject image is displayed in the timings T2 and T3). The first captured image N+Lm is not displayed on the augmented display 18 in the timing T3. Here, the second captured image N is displayed while being changed every frame in the normal observation mode, but the same second captured image N is continuously displayed for two frames as described above in the first pattern of the length measurement mode. Accordingly, a frame rate of the first pattern of the length measurement mode is substantially 1/2 of that of the normal observation mode.

The same applies to the timing T4 or later. That is, a second captured image obtained in the timing T4 is continuously displayed in the length measurement image S in the timings T4 and T5, and a second captured image N obtained in a timing T6 is continuously displayed in the length measurement image S in the timings T6 and T7. On the other hand, the first captured image N+Lm is not displayed on the augmented display 18 in the timings T4, T5, T6, and T7. Since the second captured image N not including components of measurement light is displayed in the display of the length measurement image S as described above, a frame rate is slightly lowered but hindrance to the visibility of an object to be observed, which is likely to occur due to the emission of measurement light, does not occur.

A second pattern is a case where a rolling shutter type image pickup element (CMOS), which includes a plurality of lines for picking up an image of an object to be observed illuminated with illumination light or measurement light, performs exposure in an exposure timing different for each line, and reads out electric charges in a readout timing different for each line to output image signals, is used as the image pickup element 32. Further, in the second pattern, measurement light is emitted at intervals of three frames as a specific frame interval.

In the second pattern, as shown in Fig. 30, exposure to illumination light and the readout of electric charges are performed for each line in the timing T1 and the readout of electric charges is completed (rolling shutter) in switching from the normal observation mode to the length measurement mode (in switching from the timing T1 to the timing T2), so that the second captured image N including only components of illumination light is obtained. The second captured image N is displayed on the augmented display 18 in the timing T2. With regard to "CMOS (frame period) rolling shutter" shown in Fig. 30, a diagonal line 58 indicates a timing at which exposure to light and the readout of electric charges are performed, a line Ls indicates that exposure and the readout of electric charges are started, and a line Lt indicates that exposure and the readout of electric charges are completed.

Further, illumination light and measurement light are emitted in the timing T2. A rolling shutter is performed on the basis of illumination with illumination light from the timing T1 to the timing T2 and illumination with measurement light in the timing T2, so that a first captured image N+Lm including components of illumination light and measurement light is obtained in switching from the timing T2 to the timing T3. Furthermore, a first captured image N+Lm including components of illumination light and measurement light is obtained even in switching from the timing T3 to the timing T4. The position of the spot SP is detected on the basis of the first captured images N+Lm described above. In addition, measurement light is not emitted in the timings T3 and T4.

A virtual scale corresponding to the position of the spot SP is displayed in the second captured image N displayed in the timing T2. Accordingly, a length measurement image S in which a virtual scale is displayed in the second captured image N displayed in the timing T2 is displayed in the timings T3 and T4. The second captured image N obtained in the timing T2 (first timing) is displayed on the augmented display 18 not only in the timing T2 but also in the timings T3 and T4. That is, the second captured image obtained in the timing T2 is continuously displayed for three frames until the timing T5 (second timing) at which the next second captured image is obtained (the same subject image is displayed in the timings T2, T3, and T4). On the other hand, the first captured image N+Lm is not displayed on the augmented display 18 in the timings T3 and T4. Since the same second captured image N is continuously displayed for three frames in the second pattern of the length measurement mode, a frame rate of the second pattern of the length measurement mode is substantially 1/3 of that of the normal observation mode.

The same applies to the timing T5 or later. That is, a second captured image obtained in the timing T5 is displayed in the length measurement image S in the timings T5, T6, and T7. On the other hand, the first captured image N+Lm is not displayed on the augmented display 18 in the timings T5, T6, and T7. Since the second captured image not including components of measurement light is displayed in the display of the length measurement image S as described above, a frame rate is lowered but hindrance to the visibility of an object to be observed, which is likely to occur due to the emission of planar measurement light, does not occur.

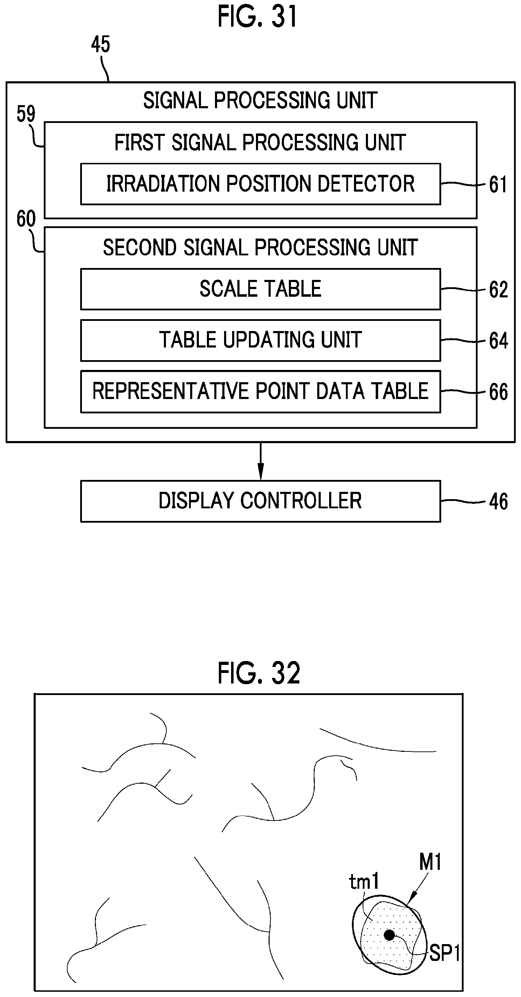

As shown in Fig. 31, the signal processing unit 45 of the augmented processor device 17 comprises a first signal processing unit 59 and a second signal processing unit 60 to recognize the position of a spot SP and to set a virtual scale. The first signal processing unit 59 detects the position of the spot SP in a captured image, and the second signal processing unit 60 sets a virtual scale according to the position of the spot SP. The captured image includes the first captured image that is obtained in turning on both illumination light and measurement light in a case where illumination light is constantly turned on but measurement light is turned on or turned off, in addition to a captured image that is obtained in a case where illumination light and measurement light are constantly turned on.

The first signal processing unit 59 comprises an irradiation position detector 61 that detects the irradiation position of the spot SP from the captured image. It is preferable that the coordinates of the position of the centroid of the spot SP are acquired in the irradiation position detector 61 as the irradiation position of the spot SP.

The second signal processing unit 60 sets a first virtual scale as a virtual scale, which is used to measure the size of a subject, on the basis of the irradiation position of the spot SP, and sets a scale display position of the first virtual scale. The second signal processing unit 60 sets a virtual scale corresponding to the irradiation position of the spot SP with reference to a scale table 62 in which a virtual scale image of which the display aspect varies depending on the irradiation position of the spot SP and the scale display position is stored in association with the irradiation position of the spot. For example, the size or shape of the virtual scale varies depending on the irradiation position of the spot SP and the scale display position. The display of the virtual scale image will be described later. Further, the contents stored in the scale table 62 are maintained even in a case where the power of the augmented processor device 17 is turned off. The virtual scale image and the irradiation position are stored in the scale table 62 in association with each other, but a distance to the subject (a distance between the distal end part 12d of the endoscope 12 and the subject) corresponding to the irradiation position and the virtual scale image may be stored in the scale table 62 in association with each other.

Since a virtual scale image is required for each irradiation position, the amount of data is increased. For this reason, considering the standpoint of the storage capacity of a memory, startup, a processing time, and the like in the endoscope 12, it is preferable that the virtual scale images are stored in the augmented processor device 17 (or the processor device 14) rather than in a memory (not shown) in the endoscope 12. Further, virtual scale images are created from representative points of a virtual scale image obtained from calibration as described later, but a loss time occurs and the real-time property of processing is impaired in a case where virtual scale images are created from representative points in the length measurement mode. For this reason, after the endoscope 12 is connected to an endoscope connection portion and virtual scale images are created from representative points once to update the scale table 62, virtual scale images are not created from representative points and virtual scale images are displayed using the updated scale table 62. Further, in the second signal processing unit 60, in the case of an emergency where it is difficult to superimpose and display images, a reference scale, which is used to determine the size of a scale, is displayed in a length measurement image from a relationship between the irradiation position of a spot SP and the number of pixels corresponding to the actual size of a subject, instead of a virtual scale image that is to be superimposed and displayed on the length measurement image.

Furthermore, the second signal processing unit 60 comprises a table updating unit 64 that updates the scale table 62 in a case where the endoscope 12 is connected to the endoscope connection portion. The reason why the scale table 62 is adapted to be capable of being updated is that a positional relationship between the optical axis Lm of measurement light and the image pickup optical system 21 varies depending on the model and the serial number of the endoscope 12 and the display aspect of a virtual scale image is also changed according to the positional relationship. A representative point data table 66 in which representative point data related to representative points extracted from a virtual scale image are stored in association with irradiation positions is used in the table updating unit 64. Details of the table updating unit 64 and the representative point data table 66 will be described later. In the representative point data table 66, a distance to a subject corresponding to an irradiation position (a distance between the distal end part 12d of the endoscope 12 and the subject) and representative point data may be stored in association with each other.

In a case where a length measurement image in which a virtual scale is superimposed on a captured image is displayed on the augmented display 18, the display controller 46 performs a control where the display aspect of the virtual scale varies depending on the irradiation position of the spot SP and a scale display position. Specifically, the display controller 46 causes the augmented display 18 to display a length measurement image in which the first virtual scale is superimposed to be centered on the spot SP. For example, a circular measurement marker is used as the first virtual scale. In this case, as shown in Fig. 32, a virtual scale M1, which shows an actual size of 5 mm (a horizontal direction and a vertical direction of the captured image), is displayed at the center of a spot SP1 formed on a tumor tm1 of a subject in a case where an observation distance is close to the near end Px (see Fig. 13).

Since the scale display position of the virtual scale M1 is positioned at the peripheral portion of the captured image that is affected by distortion caused by the image pickup optical system 21, the virtual scale M1 has an elliptical shape due to an influence of the distortion or the like. Since the above-mentioned virtual scale M1 substantially coincides with the range of the tumor tm1, the size of the tumor tm1 can be measured as about 5 mm. In the captured image, the spot may not be displayed and only the first virtual scale may be displayed.

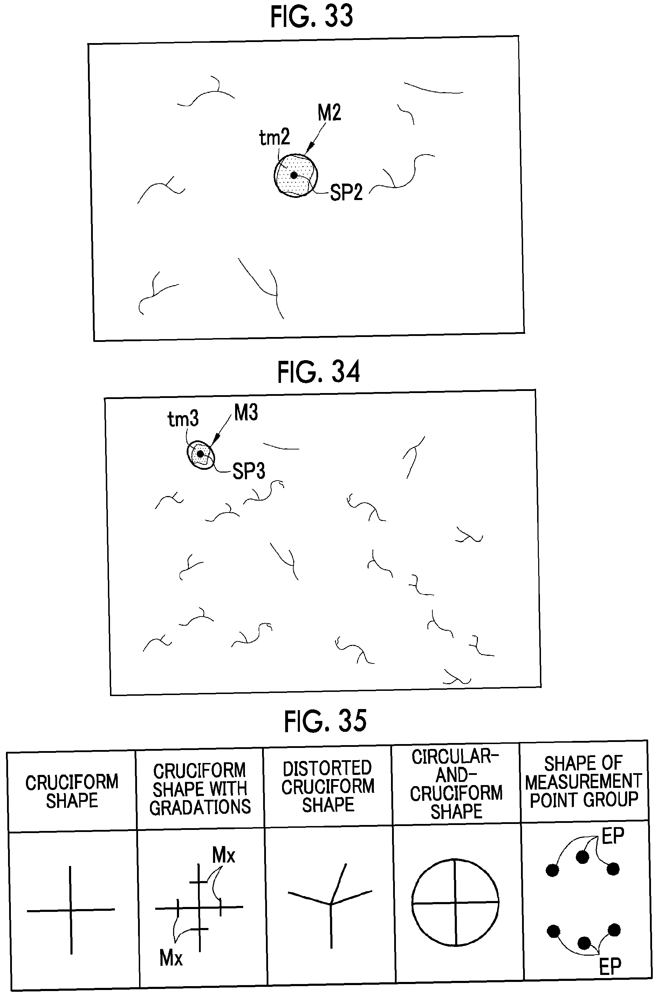

Further, as shown in Fig. 33, a virtual scale M2, which shows an actual size of 5 mm (the horizontal direction and the vertical direction of the captured image), is displayed at the center of a spot SP2 formed on a tumor tm2 of a subject in a case where an observation distance is close to the intermediate vicinity Py. Since the scale display position of the virtual scale M2 is positioned at the central portion of the captured image that is less likely to be affected by distortion caused by the image pickup optical system 21, the virtual scale M2 has a circular shape without being affected by the distortion or the like.

Furthermore, as shown in Fig. 34, a virtual scale M3, which shows an actual size of 5 mm (the horizontal direction and the vertical direction of the captured image), is displayed at the center of a spot SP3 formed on a tumor tm3 of a subject. Since the scale display position of the virtual scale M3 is positioned at the peripheral portion of the captured image that is affected by distortion caused by the image pickup optical system 21, the virtual scale M3 has an elliptical shape due to an influence of the distortion or the like. As shown in Figs. 32 to 34 having been described above, the size of the first virtual scale corresponding to the same actual size of 5 mm is reduced with an increase in an observation distance. Moreover, the shape of the first virtual scale also varies depending on the scale display position due to an influence of distortion caused by the image pickup optical system 21.

In Figs. 32 to 34, the center of the spot SP and the center of the marker are shown to coincide with each other. However, the first virtual scale may be displayed at a position away from the spot SP in a case where there is no problem in measurement accuracy. Even in this case, it is preferable that the first virtual scale is displayed near the spot. Further, the deformed first virtual scale is not displayed, and the distortion of the captured image may be corrected so that an undeformed first virtual scale is displayed in a corrected captured image.

The first virtual scale corresponding to the actual size of the subject, which is 5 mm, is displayed in Figs. 32 to 34, but the actual size of the subject may be set to any value (for example, 2 mm, 3 mm, 10 mm, or the like) according to an object to be observed or the purpose of observation. Further, the first virtual scale has a substantially circular shape in Figs. 32 to 34, but may have a cruciform shape where a vertical line and a horizontal line intersect with each other as shown in Fig. 35. Furthermore, the first virtual scale may have a cruciform shape with gradations where gradations Mx are given to at least one of a vertical line or a horizontal line of a cruciform shape. Further, the first virtual scale may have a distorted cruciform shape of which at least one of a vertical line or a horizontal line is inclined. Furthermore, the first virtual scale may have a circular-and-cruciform shape where a cruciform shape and a circle are combined with each other. In addition, the first virtual scale may have the shape of a measurement point group where a plurality of measurement points EP corresponding to an actual size from a spot are combined with each other. Further, one first virtual scale may be displayed or a plurality of first virtual scales may be displayed, and the color of the first virtual scale may be changed according to an actual size.

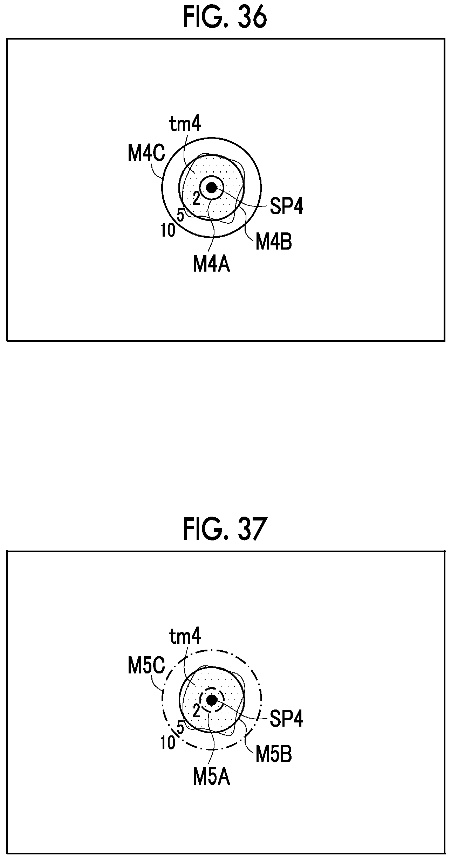

As shown in Fig. 36, three concentric circular virtual scales M4A, M4B, and M4C having different sizes (diameters as the sizes are 2 mm, 5 mm, and 10 mm, respectively) may be displayed in the captured image as the first virtual scale to be centered on a spot SP4 formed on a tumor tm4. Since the three concentric circular virtual scales are displayed as a plurality of virtual scales, time and effort required to switch a virtual scale can be saved and measurement can be performed even in a case where a subject has a non-linear shape. In a case where a plurality of concentric circular virtual scales are to be displayed to be centered on a spot, a size and a color are not designated for each virtual scale and combinations of a plurality of conditions may be prepared in advance and one can be selected from these combinations.

In Fig. 36, all the three concentric circular virtual scales are displayed with the same color (black). However, in a case where a plurality of concentric circular markers are to be displayed, a plurality of concentric circular color markers of which colors are different from each other may be used. As shown in Fig. 37, a virtual scale M5A is displayed with a dotted line representing red, a virtual scale M5B is displayed with a solid line representing blue, and a virtual scale M5C is displayed with a one-dot chain line representing white. Since identifiability can be improved in a case where the colors of the virtual scales are different from each other in this way, measurement can be easily performed.

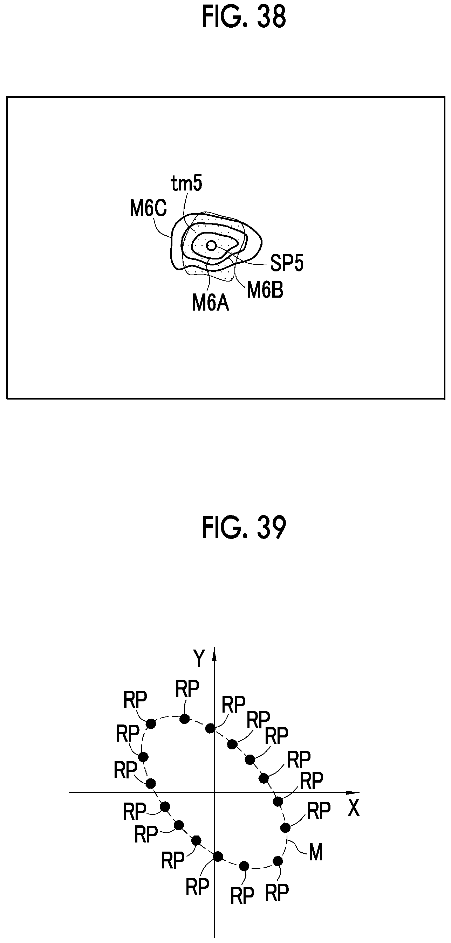

Further, as shown in Fig. 38, a plurality of distorted concentric circular virtual scales, which are distorted from the respective concentric circles, may be used as the first virtual scale other than the plurality of concentric circular virtual scales. In this case, a virtual scale M6A, a virtual scale M6B, and a virtual scale M6C having distorted concentric circular shapes are displayed in a captured image to be centered on a spot SP5 formed on a tumor tm5.

In a case where the endoscope 12 is connected to the endoscope connection portion, the table updating unit 64 creates a virtual scale image corresponding to the model and/or the serial number of the endoscope 12 with reference to the representative point data table 66 and updates the scale table 62.

Representative point data related to representative points of a virtual scale image obtained in calibration are stored in the representative point data table 66 in association with the irradiation position of the spot SP. The representative point data table 66 is created by a calibration method to be described later. As shown in Fig. 39, coordinate information (X-coordinates and Y-coordinates) of representative points RP that are some points extracted from an image M of a circular virtual scale, which is a virtual scale image, is included as the representative point data. The representative point data stored in the representative point data table 66 are data in a case where a positional relationship between the optical axis Lm of measurement light and the image pickup optical system 21 is a default positional relationship.

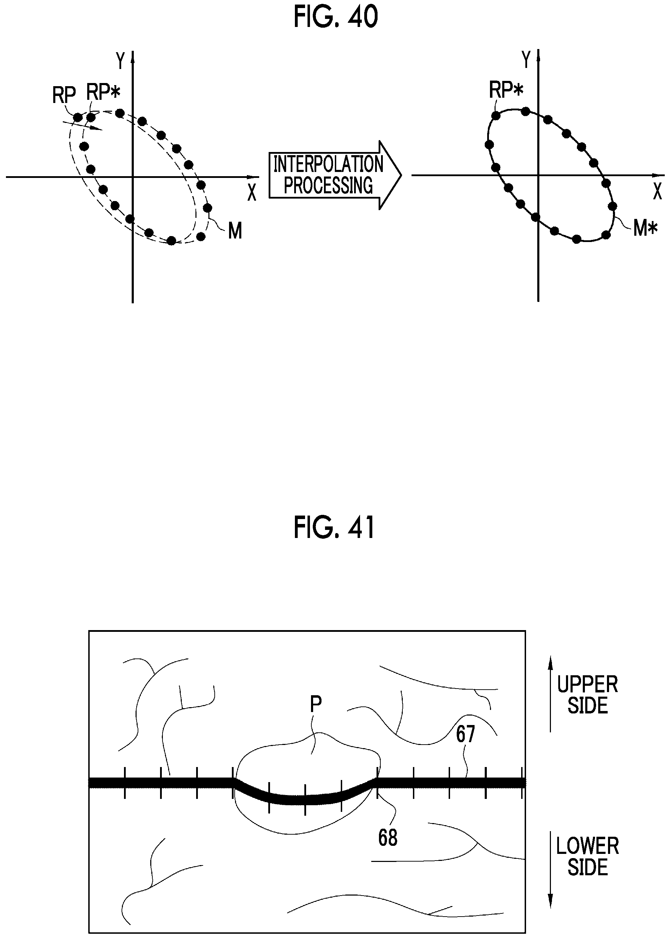

In a case where the endoscope 12 is connected to the endoscope connection portion, the table updating unit 64 acquires information about a positional relationship between the optical axis Lm of measurement light and the image pickup optical system 21 and updates the scale table 62 using the positional relationship and the representative point data table 66. Specifically, the table updating unit 64 calculates difference values of the coordinate information of the representative points RP from a difference between a positional relationship between the optical axis Lm of measurement light and the image pickup optical system 21 in the endoscope 12 connected to the endoscope connection portion and a default positional relationship. Then, the table updating unit 64 creates a virtual scale image M* on the basis of representative points RP* that are obtained in a case where the coordinates of default representative points RP are shifted by the calculated difference values as shown in Fig. 40. It is preferable that interpolation processing for connecting the representative points RP* is performed for the creation of the virtual scale image. The created virtual scale image subjected to the interpolation processing is associated with irradiation positions by the table updating unit 64. Accordingly, the update of the scale table 62 is completed. In Fig. 40, reference characters are given to only a part of the representative points RP and RP*.

Light, which forms a spot in a case where a subject is irradiated with the light, is used as the measurement light, but other light may be used. For example, planar measurement light, which is formed on the subject as an intersection line 67 as shown in Fig. 41 in a case where the subject is irradiated with the light, may be used. In this case, a second virtual scale that consists of the intersection line 67 and gradations 68 formed on the intersection line 67 and serving as an index of the size of the subject (for example, a polyp P) is generated as a virtual scale. In a case where planar measurement light is used, the irradiation position detector 61 detects the position of the intersection line 67 (the irradiation position of the measurement light). An observation distance is shorter as the intersection line 67 is positioned closer to the lower side, and an observation distance is longer as the intersection line 67 is positioned closer to the upper side. For this reason, an interval between the gradations 68 is larger as the intersection line 67 is positioned closer to the lower side, and an interval between the gradations 68 is smaller as the intersection line 67 is positioned closer to the upper side.

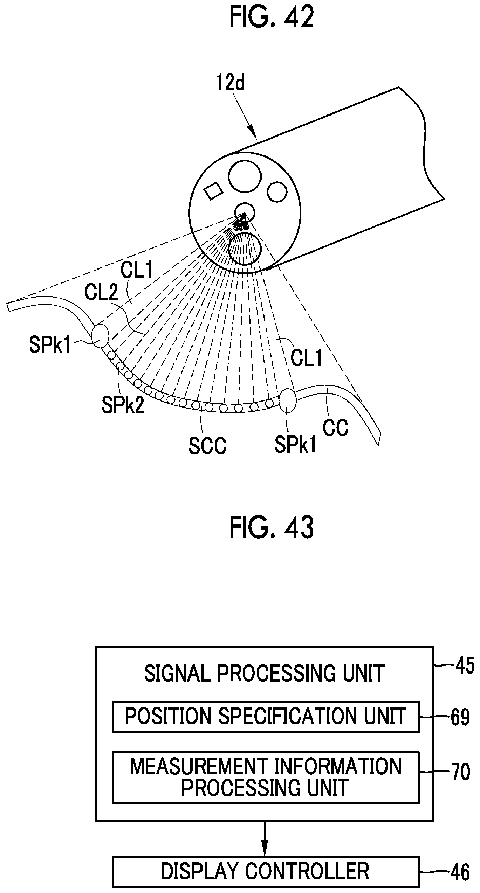

Further, the measurement light may be formed of planar light that includes at least two first feature lines CL as shown in Fig. 42. In a case where the subject is irradiated with the measurement light, an intersection curve CC is formed according to undulations on the subject and first spots SPk1 are formed on the intersection curve CC at positions corresponding to two first feature lines CL1, respectively. Further, the measurement light includes a plurality of second feature lines CL2 that are positioned between the two first feature lines CL1 and are different from the first feature lines CL1. In a case where the subject is irradiated with the measurement light including the first feature lines CL1 and the second feature lines CL2, second spots SPk2 are formed at positions corresponding to the plurality of second feature lines CL2, respectively. The second spot SPk2 is smaller than the first spot SPk1 and an interval between the second spots SPk2 is small. For this reason, a specific intersection curve SCC is formed on the intersection curve by the plurality of second spots SPk2. Measurement information is calculated on the basis of the position of the specific intersection curve SCC.

As shown in Fig. 43, the signal processing unit 45 of the augmented processor device 17 includes a position specification unit 69 and a measurement information processing unit 70 to recognize the positions of the first spots SPk1 or the second spots SPk2 and to calculate the measurement information. The position specification unit 69 specifies the positions of the first spots SPk1 or the second spots SPk2 from a captured image. As a method of specifying a position, for example, a captured image is binarized and the centroids of white portions (pixels at which the signal intensity is higher than a threshold value for binarization) of the binarized image are specified as the positions of the first spots SPk1 or the second spots SPk2.

The measurement information processing unit 70 calculates the measurement information from the positions of the first spots SPk1 or the second spots SPk2. The calculated measurement information is displayed in the captured image by the display controller 46. In a case where the measurement information is calculated on the basis of the positions of the two first spots SPk1, the measurement information can be accurately calculated even under a situation where the subject has a three-dimensional shape.

As shown in Fig. 44, the measurement information includes a first straight-line distance that indicates a straight-line distance between the two first and second spots SPk1 and SPk2. The measurement information processing unit 70 calculates the first straight-line distance by the following method. As shown in Fig. 45, the measurement information processing unit 70 obtains coordinates (xp1, yp1, zp1) that indicate the actual size of the first spot SPk1 on the basis of the position of the first spot SPk1. Coordinates corresponding to the actual size are obtained as xp1 and yp1 from the coordinates of the position of the first spot SPk1 in the captured image, respectively. A coordinate corresponding to the actual size is obtained as zp1 from the coordinates of the position of the first spot SPk1 and the coordinates of the position of a predetermined specific spot SPk. Likewise, the measurement information processing unit 70 obtains coordinates (xp2, yp2, zp2) that indicate the actual size of the second spot SPk2 on the basis of the position of the second spot SPk2. Further, coordinates corresponding to the actual size are obtained as xp2 and yp2 from the coordinates of the position of the second spot SPk2 in the captured image, respectively. A coordinate corresponding to the actual size is obtained as zp2 from the coordinates of the position of the second spot SPk2 and the coordinates of the position of the predetermined specific spot SPk. The first straight-line distance is calculated from the following equation).

$$\text{Equation) First straight-line distance} = ((xp2-xp1)^2+(yp2-yp1)^2+(zp2-zp1)^2)^{0.5}$$

The calculated first straight-line distance is displayed as measurement information 71 ("20 mm" in Fig. 44) in the captured image. The specific spot SPk may be displayed or may not be displayed on the augmented display 18.

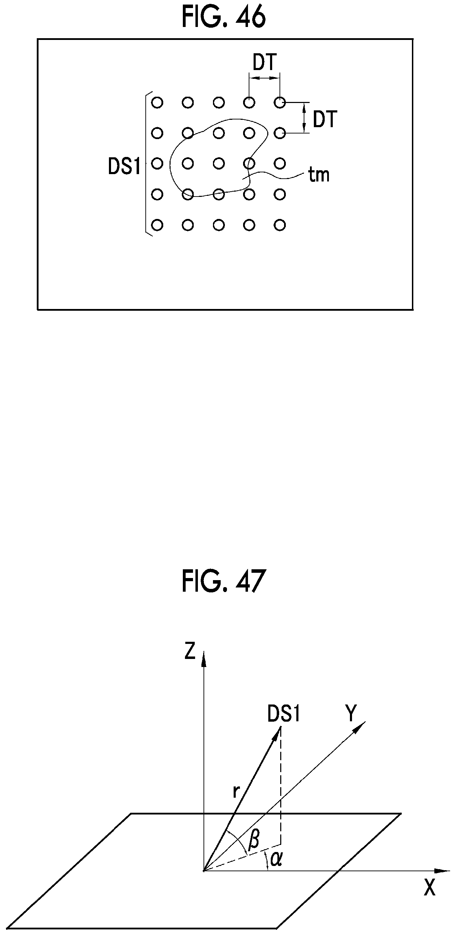

Further, a plurality of spotlights arranged in the form of a grid at predetermined intervals in a vertical direction and a horizontal direction may be used as the measurement light. In a case where an image of a tumor tm or the like present in a subject is picked up with spotlights arranged in the form of a grid, an image of diffraction spots DS1 is acquired as shown in Fig. 46. The signal processing unit 45 of the augmented processor device 17 measures an interval DT between the diffraction spots DS1. The intervals DT correspond to the number of pixels of an image pickup surface of the image pickup element 32. An interval between the plurality of diffraction spots DS1 at a specific portion (for example, an interval between the diffraction spots near the center of the image pickup surface) may be measured.

In a case where an interval between the diffraction spots DS1 is measured, a direction and a distance to a subject are calculated on the basis of a measurement result. A relationship between an interval between the diffraction spots DS1 (the number of pixels) and a distance to a subject is used in this processing. Specifically, a direction ($\alpha$, $\beta$) and a distance (r) to a diffraction spot DS1 as an object to be measured are calculated as shown in Fig. 47. After the direction and the distance to the subject are calculated, two-dimensional information or three-dimensional information of the subject is calculated on the basis of the calculated direction and the calculated distance. The shape, the size, the area, and the like of the subject in a two-dimensional space (an XY plane in Fig. 47) or the three-dimensional space (an XYZ space in Fig. 47) can be calculated as the two-dimensional information or the three-dimensional information of the subject. ($\alpha$, $\beta$, r) can be converted into (X, Y, Z) using the following equation A), the following equation B), and the following equation C), and the shape, the size, the area, and the like can be calculated from the (X, Y, Z) coordinates of each point of the subject.

$$\text{Equation A) } X = r \times \cos\alpha \times \cos\beta$$

$$\text{Equation B) } Y = r \times \cos\alpha \times \sin\beta$$

$$\text{Equation C) } Z = r \times \sin\alpha$$

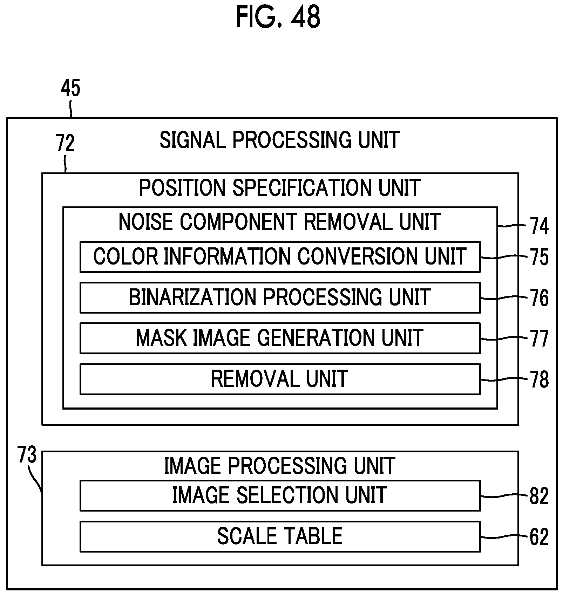

As shown in Fig. 48, the signal processing unit 45 of the augmented processor device 17 comprises a position specification unit 72 and an image processing unit 73 to recognize the position of a spot and to set a virtual scale. The position specification unit 72 specifies the position of the spot SP in the first captured image (an image based on the measurement light and the illumination light), and the image processing unit 73 processes the first captured image or the second captured image (an image based on the illumination light) to generate a length measurement image on the basis of the position of the spot SP.

The position specification unit 72 includes a noise component removal unit 74 that removes noise components hindering the specification of the position of the spot SP. In a case where a color (a color approximate to the color of the measurement light), which is different from the color of the measurement light forming the spot SP but is close to the color of the measurement light, is included in the first captured image, it may not possible to accurately specify the position of the spot SP. Accordingly, the noise component removal unit 74 removes components of the color approximate to the color of the measurement light from the first captured image as the noise components. The position specification unit 72 specifies the position of the spot SP on the basis of a noise-removed first captured image from which noise components have been removed.

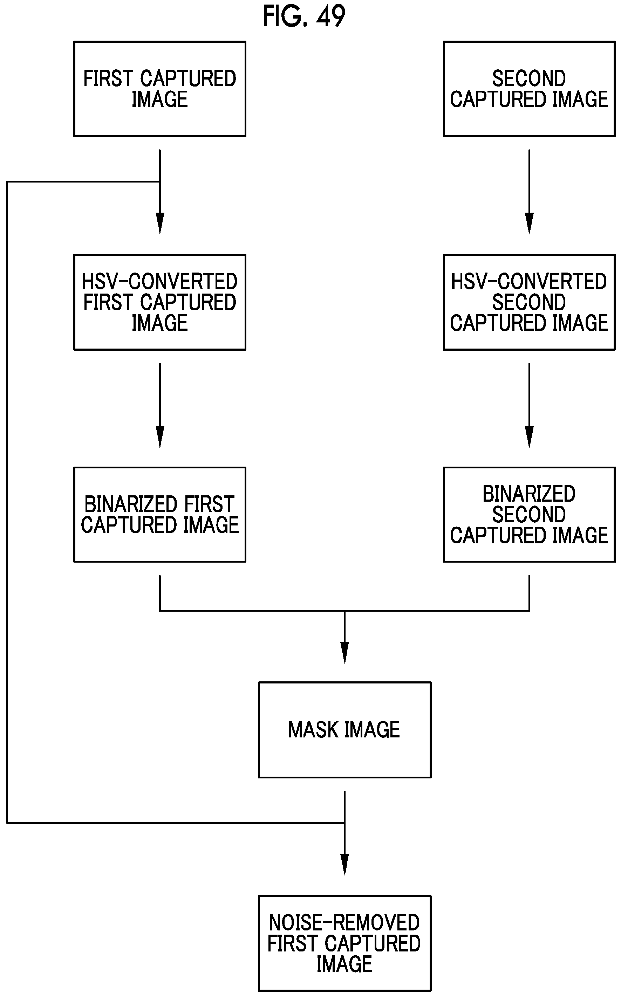

The noise component removal unit 74 comprises a color information conversion unit 75, a binarization processing unit 76, a mask image generation unit 77, and a removal unit 78. A flow of processing for obtaining the noise-removed first captured image will be described with reference to Fig. 49. The color information conversion unit 75 converts the first captured image, which is an RGB image, into a first color information image and converts the second captured image, which is an RGB image, into a second color information image. It is preferable that, for example, HSV (hue (H), saturation (S), and value (V)) are used as color information. In addition, color differences Cr and Cb may be used as color information.

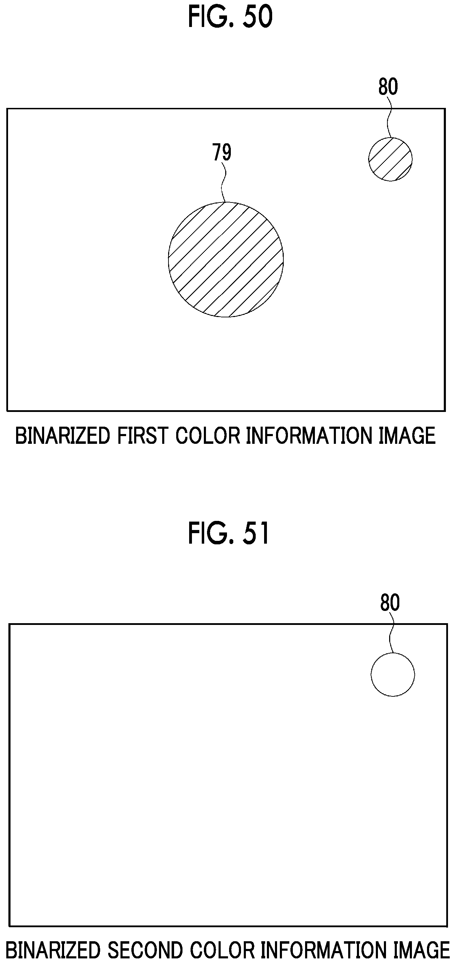

The binarization processing unit 76 binarizes the first color information image to generate a binarized first color information image, and binarizes the second color information image to generate a binarized second color information image. A threshold value for binarization including the color of the measurement light is used as a threshold value that is used for binarization. As shown in Figs. 50 and 51, color information 80 of noise components is included in the binarized first color information image in addition to the color information 79 of the measurement light.

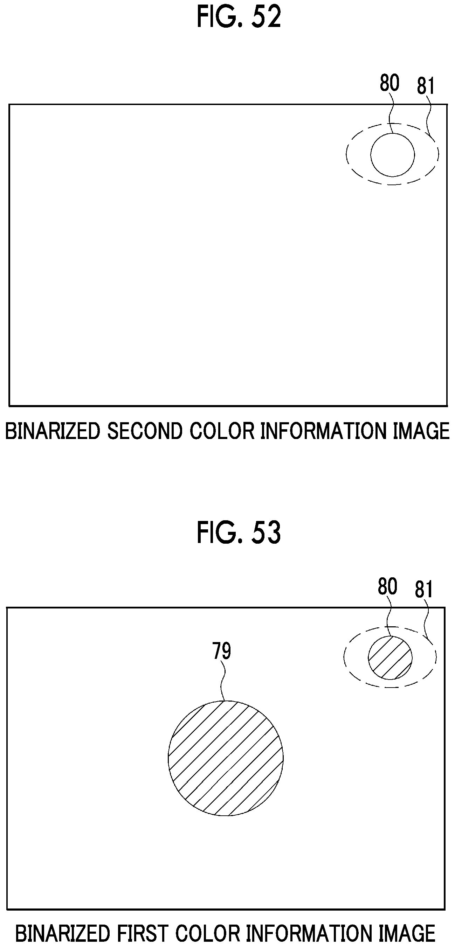

The mask image generation unit 77 removes color information of noise components from the first captured image and generates a mask image to be used to extract color information of the measurement light, on the basis of the binarized first color information image and the binarized second color information image. As shown in Fig. 52, the mask image generation unit 77 specifies a noise component region 81, which includes noise components, from noise components included in the binarized second captured image. It is preferable that the noise component region 81 is set to be larger than a region occupied by the color information 80 of noise components. The reason for this is that the size of a region of the color information 80 of noise components in a case where camera shake or the like occurs is larger than that in a case where camera shake or the like does not occur. Then, as shown in Fig. 53, the mask image generation unit 77 generates a mask image in which a region of the color information 79 of the measurement light of the binarized first color information image is set as an extraction region from which color information is to be extracted and the noise component region 81 thereof is set as a non-extraction region from which color information is not to be extracted. Figs. 50 to 53 are diagrams schematically illustrating the binarized first color information image, the binarized second color information image, the noise component region, and the mask image.

The removal unit 78 extracts color information from the first color information image using the mask image, so that a noise-removed first color information image from which color information of noise component has been removed and color information of the measurement light has been extracted is obtained. The noise-removed first color information image is changed to a noise-removed first captured image by being subjected to RGB conversion processing for returning color information to an RGB image. The position specification unit 72 specifies the position of the spot SP on the basis of the noise-removed first captured image. Since noise components are removed from a noise-removed second captured image, the position of the spot SP can be accurately specified.

The image processing unit 73 includes an image selection unit 82 and the scale table 62. The image selection unit 82 selects a processing target image, which is an image to be subjected to processing based on the position of the spot SP, from the first captured image or the second captured image. The image processing unit 73 performs the processing, which is based on the position of the spot SP, on the image selected as the processing target image. The image selection unit 82 selects the processing target image on the basis of a state related to the position of the spot SP. The image selection unit 82 may be adapted to select the processing target image according to a user's instruction. For example, the user interface 16 is used for a user's instruction.

Specifically, in a case where the spot SP is in a specific range for a specific period, it is considered that the subject or the distal end part 12d of the endoscope is less moved. Accordingly, the second captured image is selected as the processing target image. In a case where the subject or the distal end part 12d of the endoscope is less moved as described above, a virtual scale can be easily aligned with a lesion portion included in the subject even though there is no spot SP. Further, since color components of measurement light are not included in the second captured image, the color reproducibility of the subject is not impaired. On the other hand, in a case where the position of the spot SP is not in the specific range for a specific period, it is considered that the subject or the distal end part 12d of the endoscope is moved much. Accordingly, the first captured image is selected as the processing target image. In a case where the subject or the distal end part 12d of the endoscope is moved much as described above, a user operates the endoscope 12 such that the spot SP is positioned at a lesion portion. Therefore, a virtual scale is easily aligned with the lesion portion.

The image processing unit 73 generates a first virtual scale, which shows the actual size of the subject, as a virtual scale on the basis of the position of the spot SP in the first captured image. The image processing unit 73 calculates the size of the virtual scale from the position of the spot SP with reference to the scale table 62 in which a relationship between the position of the spot SP in the first captured image and the first virtual scale showing the actual size of the subject is stored. Then, the image processing unit 73 generates a first virtual scale corresponding to the size of the virtual scale.

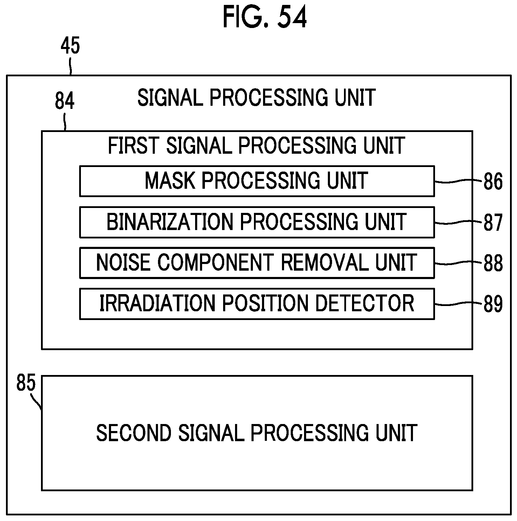

As shown in Fig. 54, the signal processing unit 45 of the augmented processor device 17 comprises a first signal processing unit 84 and a second signal processing unit 85 to recognize the position of the spot SP and to set a virtual scale. The first signal processing unit 84 detects the position of the spot SP in a captured image, and the second signal processing unit 85 sets a virtual scale according to the position of the spot SP.

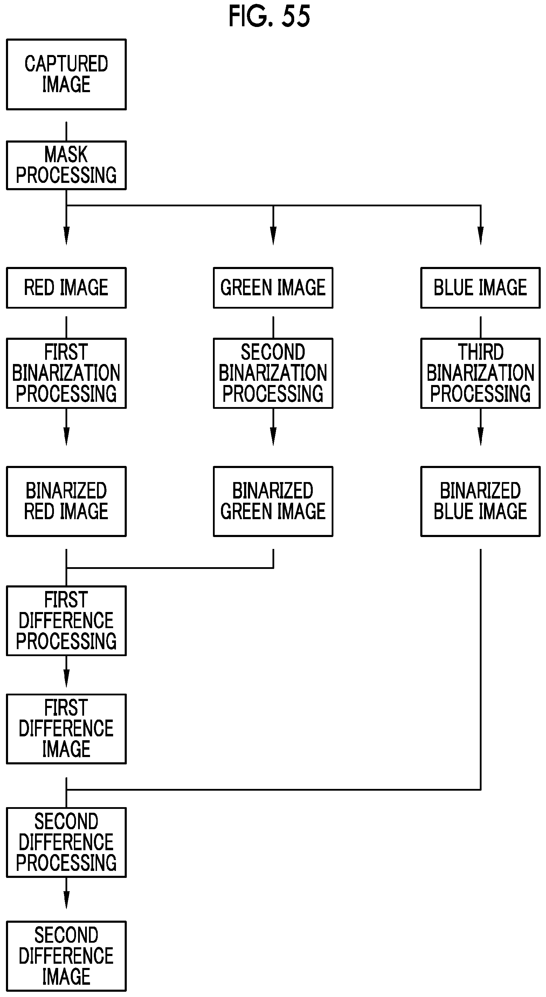

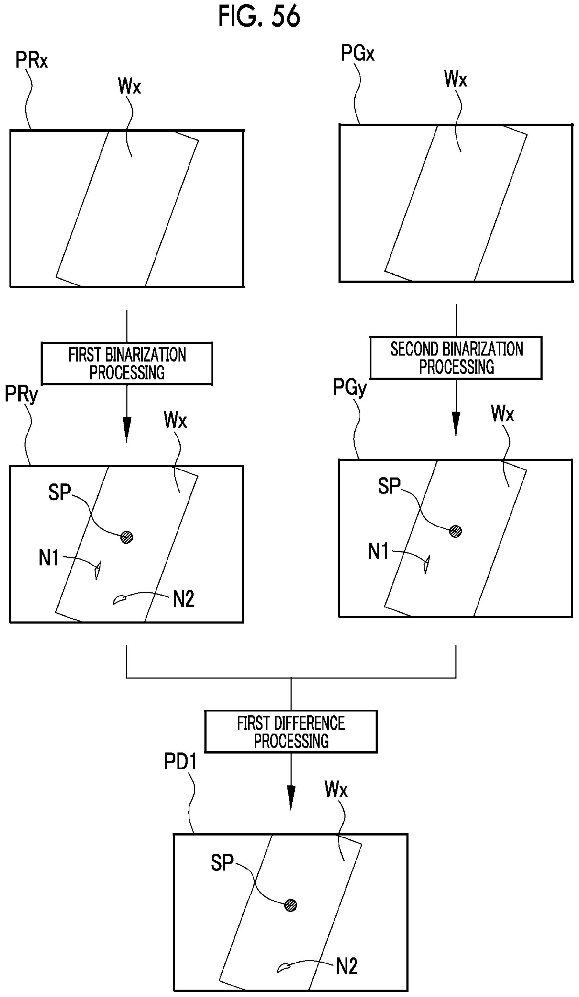

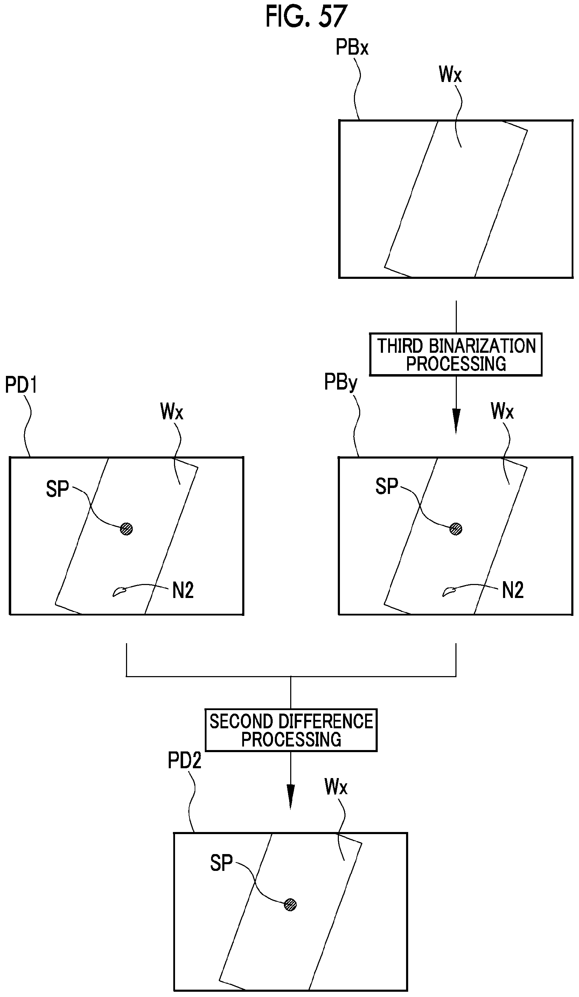

The first signal processing unit 84 comprises a mask processing unit 86, a binarization processing unit 87, a noise component removal unit 88, and an irradiation position detector 89. Processing for removing noise components in the first signal processing unit 84 will be described with reference to Figs. 55 to 57. The mask processing unit 86 performs mask processing of extracting a substantially parallelogram-shaped illumination position-movable range Wx, which represents the movable range of the illumination position of the measurement light on the subject, on a red image, a green image, and a blue image of the captured image. Accordingly, a red image PRx, a green image PGx, and a blue image PBx from which the illumination position-movable ranges Wx have been extracted and which have been subjected to the mask processing are obtained as shown in Figs. 56 and 57. Noise components are removed from pixels present in the illumination position-movable ranges, and the irradiation position of the spot SP is detected.

Next, the binarization processing unit 87 obtains a binarized red image PRy (binarized first spectral image) by performing first binarization processing on pixels present in the illumination position-movable range in the red image PRx subjected to the mask processing. In the first binarization processing, as a threshold value condition for the first binarization processing, pixels having a pixel value equal to or larger than "225" are defined as "1" and pixels having a pixel value less than "225" are defined as "0". The spot SP, which is a component of the measurement light, is detected by this first binarization processing. However, in the first binarization processing, a second noise component N2, which is halation (pixel saturation) caused by illumination light, is also detected in addition to a first noise component N1 that is a high-luminance component of a red component of the illumination light. These first and second noise components are factors that hinder the detection of the irradiation position of the spot SP. The threshold value condition refers to a condition that defines the range of the pixel value of a pixel defined as "0" by binarization and the range of the pixel value of a pixel defined as "1" by binarization in addition to a condition that is related to a threshold value indicating a boundary between the pixel value of a pixel defined as "0" by binarization and the pixel value of a pixel defined as "1" by binarization.

Then, in order to remove the first noise component, the noise component removal unit 88 performs first difference processing of the binarized red image PRy and a binarized green image PGy (binarized second spectral image) that is the green image PGx binarized by second binarization processing. The first noise component N1 has been removed in a first difference image PD1 that is obtained from the first difference processing. However, the second noise component N2 often remains in the first difference image PD1 without being removed. The pixel value of a pixel, which is defined as "0" or less by the first difference processing, is set to "0". In the second binarization processing, as a threshold value condition for the second binarization processing, pixels having a pixel value in the range of "30" to "220" are defined as "1" and pixels having a pixel value in other ranges, that is, a pixel value equal to or larger than "0" and less than "30" or exceeding "220" are defined as "0". The first noise component is removed by the first difference processing of the binarized red image and the binarized green image, but the first noise component may be removed by other first arithmetic processing.

Further, in order to remove the second noise component, as shown in Fig. 57, the noise component removal unit 88 performs second difference processing of the first difference image PD1 and a binarized blue image PBy that is the blue image PBx binarized by third binarization processing. The second noise component, which is difficult to be removed by the first difference processing, has been removed in a second difference image PD2 that is obtained from the second difference processing. The pixel value of a pixel, which is defined as "0" or less by the second difference processing as in the first difference processing, is set to "0". In the third binarization processing, as a threshold value condition for the third binarization processing, pixels having a pixel value equal to or larger than "160" are defined as "1" and pixels having a pixel value less than "160" are defined as "0". The second noise component is removed by the second difference processing of the first difference image and the binarized blue image, but the second noise component may be removed by other second arithmetic processing.

The irradiation position detector 89 detects the irradiation position of the spot SP from the first difference image or the second difference image. It is preferable that the coordinates of the position of the centroid of the spot SP are acquired in the irradiation position detector 89 as the irradiation position of the spot SP.

The second signal processing unit 85 sets a first virtual scale, which shows the actual size of the subject, as a virtual scale on the basis of the position of the spot SP. The second signal processing unit 85 calculates the size of the virtual scale from the position of the spot with reference to the scale table 62 in which a relationship between the position of the spot SP and the first virtual scale showing the actual size of the subject is stored. Then, the second signal processing unit 85 sets a first virtual scale corresponding to the size of the virtual scale.

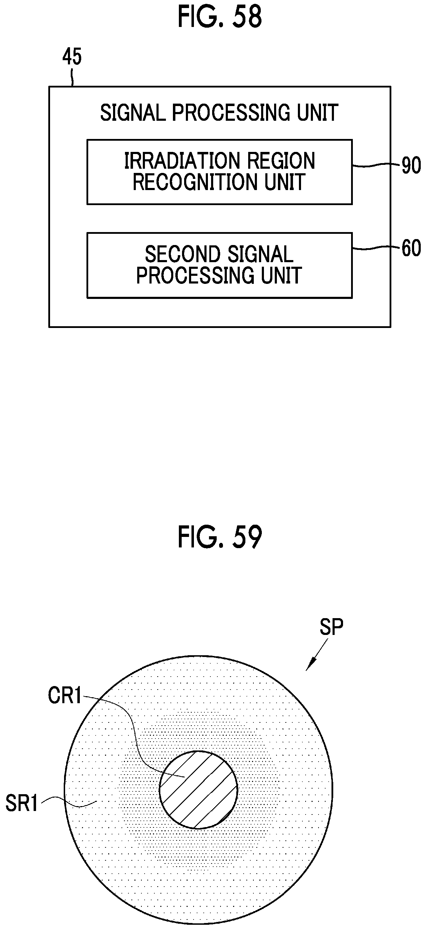

As shown in Fig. 58, the signal processing unit 45 of the augmented processor device 17 comprises an irradiation region recognition unit 90 and a second signal processing unit 60. The irradiation region recognition unit 90 recognizes a measurement light-irradiation region, which has a pattern having a specific shape, from the captured image. Specifically, as shown in Fig. 59, the pattern having a specific shape includes a white central region CR1 and a peripheral region SR1 that covers the periphery of the central region and has a feature quantity based on the measurement light. In a case where the measurement light-irradiation region is the above-mentioned spot SP, the pattern having a specific shape has a circular shape. In this case, the white central region CR1 has a circular shape, and the peripheral region SR1 has the shape of a ring.

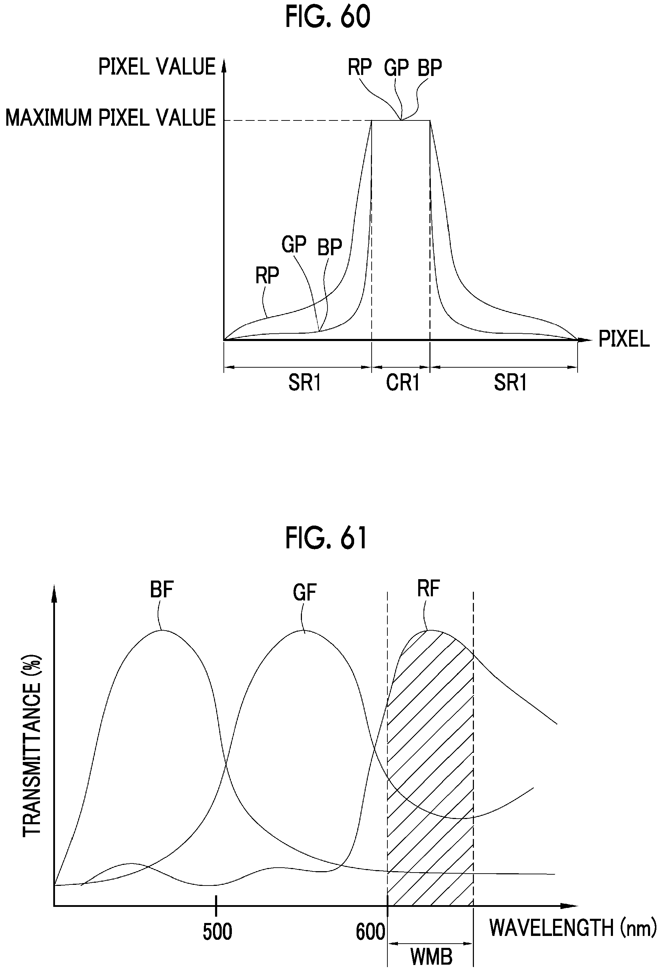

Fig. 60 shows the distribution of pixel values of the respective color images of the captured image that includes a red image RP, a green image GP, and a blue image BP as a plurality of color images. Since the pixel values of the red image RP, the green image GP, and the blue image BP in the central region CR1 reach the maximum pixel value (for example, 255), the central region CR1 is white. In a case where the measurement light is incident on the image pickup element 32 in this case, the measurement light is transmitted with the maximum transmittance of not only a red color filter RF of the image pickup element 32 but also a green color filter GF and a blue color filter BF in a wavelength range WMB of the measurement light as shown in Fig. 61. On the other hand, the pixel value of the red image RP is larger than the pixel value of the green image GP or the blue image BP in the peripheral region SR1. For this reason, the peripheral region SR1 has redness. The measurement light is emitted with a specific amount of light in the light source 23a, so that the pixel values of the red image RP, the green image GP, and the blue image BP in the central region CR1 are set to the maximum pixel value.

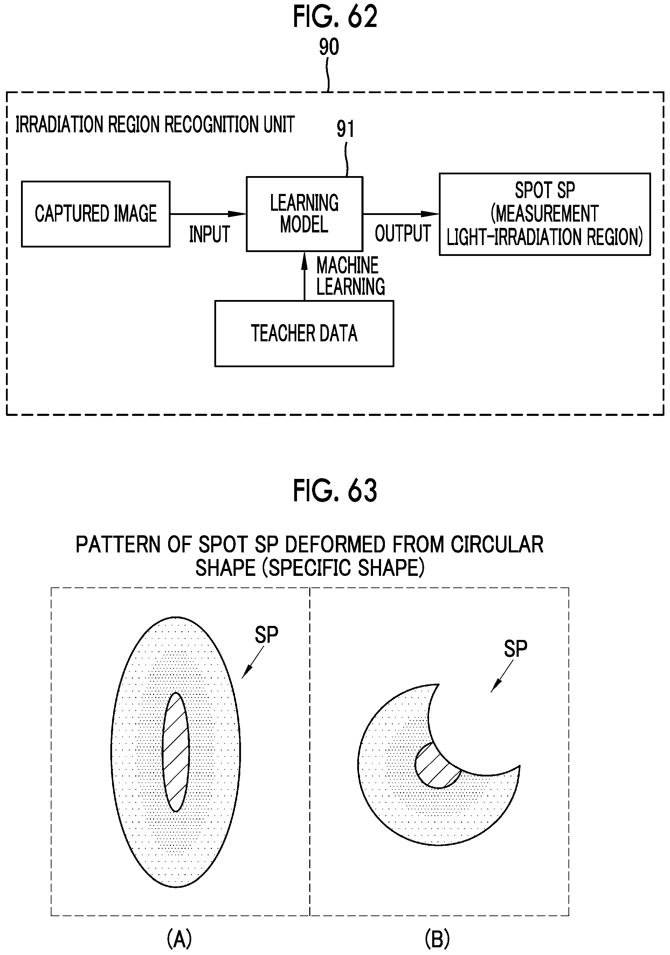

The irradiation region recognition unit 90 can recognize the spot SP that has the specific shape and the feature quantity described above. Specifically, it is preferable that the irradiation region recognition unit 90 includes a learning model 91 for recognizing the spot SP by outputting the spot SP, which is a measurement light-irradiation region, in response to the input of the captured image as shown in Fig. 62. The learning model 91 is subjected to machine learning using many teacher data in which captured images and already recognized measurement light-irradiation regions are associated with each other. It is preferable that a convolutional neural network (CNN) is used as the machine learning.

Since the spot SP is recognized using the learning model 91, not only the circular spot SP (see Fig. 59) that includes the circular central region CR1 and the ring-shaped peripheral region SR1 but also spots SP that have patterns deformed from a circular shape, which is a specific shape, can be recognized. For example, a spot SP, which is deformed in a vertical direction as shown in (A) of Fig. 63, can also be recognized. Further, a spot SP, which is deformed such that a part of a circular shape is cut out as shown in (B) of Fig. 63, can also be recognized. Furthermore, the feature quantity of the peripheral region SR1, which can be recognized by the learning model 91, includes a blue color or a green color in addition to a red color that is the color of the measurement light. Moreover, the feature quantity of the peripheral region SR1, which can be recognized by the learning model 91, includes the luminance, value, saturation, and hue of the measurement light.

It is preferable that luminance conversion processing or processing for converting value, saturation, or a hue is performed on the peripheral region of the spot SP included in the captured image to acquire the luminance, value, saturation, or hue of the measurement light.

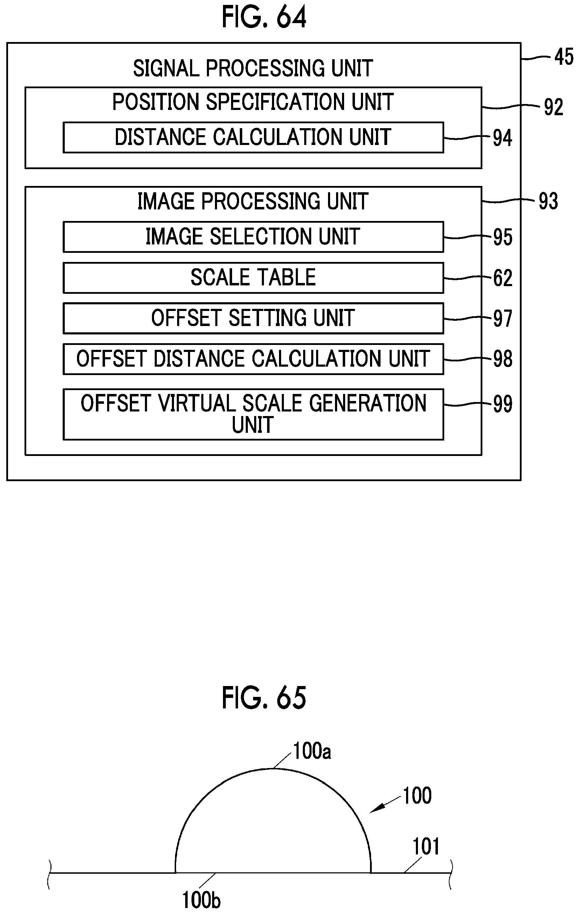

As shown in Fig. 64, the signal processing unit 45 of the augmented processor device 17 comprises a position specification unit 92 and an image processing unit 93 to recognize the position of the spot SP, to calculate an observation distance to the subject, and to set virtual scales. The position specification unit 92 specifies the position of the spot SP in the captured image, and calculates the observation distance. The image processing unit 93 sets various virtual scales on the basis of the observation distance, and generates a length measurement image that is obtained from the processing of the captured image using the various virtual scales.

The position specification unit 92 includes a distance calculation unit 94. The position specification unit 92 specifies the position of the spot SP, which is formed on the subject by the measurement light, on the basis of the captured image of the subject that is illuminated with the illumination light and the measurement light. The distance calculation unit 94 obtains an observation distance from the position of the spot SP.

The image processing unit 93 includes an image selection unit 95, a scale table 62, an offset setting unit 97, an offset distance calculation unit 98, and an offset virtual scale generation unit 99. The image selection unit 95 selects an image that is to be subjected to processing based on the position of the spot SP. The offset setting unit 97 sets an offset amount, which corresponds to the height of the spot SP of the convex polyp 100, for the observation distance. The offset distance calculation unit 98 adds the offset amount to the observation distance to calculate an offset distance. The offset virtual scale generation unit 99 generates an offset virtual scale on the basis of the offset distance.

An offset will be described below. First, the convex shape of the subject refers to a shape of the subject protruding from a peripheral portion. Accordingly, the convex shape has only to be a shape in which any portion protrudes from a peripheral portion, and other shapes, such as a size, the area of a shape, the height of a protruding portion and/or the number of protruding portions, and continuity of a height or the like, are not limited.

More specifically, for example, as shown in Fig. 65, the subject includes a convex polyp 100. The polyp 100 has a shape protruding from a subject around the polyp. The polyp 100 includes an apex portion 100a and a flat portion 100b. Fig. 65 shows a case where the polyp 100 is viewed in a horizontal direction in a case where the height direction of the polyp 100 is defined as a vertical direction, but the polyp 100 is also present on the front side and the back side of the plane of the paper in a direction perpendicular to the plane of the paper since the polyp 100 has a three-dimensional shape. A flat surface around the polyp 100 where a flat portion of the subject is formed and the polyp 100 is formed is defined as the flat portion 100b of the polyp 100. A flat surface around the polyp 100 where a flat portion is formed is an extension surface 101 of the flat portion 100b.

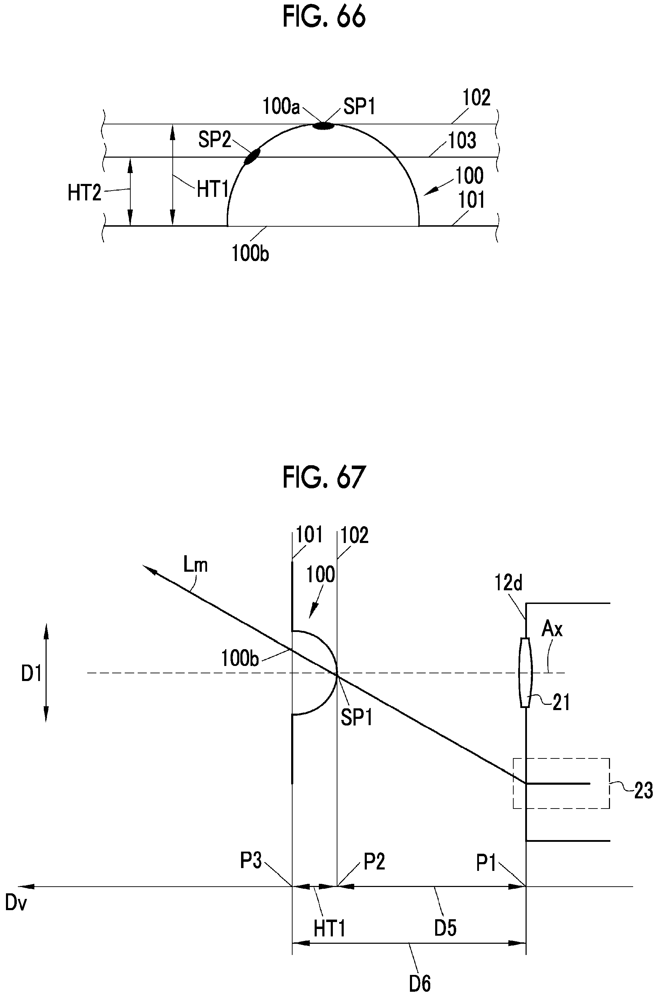

Next, the height of the spot SP of the convex polyp 100 will be described. In the present embodiment, the height of the spot SP of the polyp 100 is a distance between the spot SP of the polyp 100 and the flat portion 100b of the polyp 100 in the vertical direction. More specifically, a spot SP1 is formed at the apex portion 100a of the polyp 100 as shown in Fig. 66. Accordingly, in a case where a plane parallel to the extension surface 101 and passing through the apex portion 100a of the polyp 100 is defined as a parallel plane 102, a distance between the parallel plane 102 and the extension surface 101 is a height HT1 of the spot SP1 of the polyp 100 (a distance between the apex portion 100a of the polyp 100 and the flat portion 100b in the vertical direction). The polyp 100 and the height HT1 (the same applies to the following height HT2) are schematically shown, and the type, the shape, the size, or the like of the convex polyp are not limited as long as the polyp is a portion protruding from a peripheral portion.

Further, in Fig. 66, a spot SP2 is formed in a region of the polyp 100 that is not the apex portion 100a. That is, the spot SP2 is formed between the apex portion 100a of the polyp 100 and an end portion of the polyp 100. Accordingly, in a case where a plane parallel to the extension surface 101 and passing through the spot SP2 of the polyp 100 is defined as a parallel plane 103, a distance between the parallel plane 103 and the extension surface 101 is a height HT2 of the spot SP2 of the polyp 100. Therefore, the height HT2 of the spot SP2 of the polyp 100 is a distance between the spot SP2 of the polyp 100 and the flat portion 100b in the vertical direction.

The observation distance and the offset amount will be described below. As shown in Fig. 67, the spot SP1 is formed at the apex portion 100a of the polyp 100 by the measurement light. An observation distance obtained from the spot SP1 is a distance D5 between a position P1 of the distal end part 12d of the endoscope and a position P2 of the spot SP1. A virtual scale corresponding to the distance D5 is a virtual scale corresponding to the actual measurement on the parallel plane 102. Accordingly, in a case where the spot SP1 is formed at the apex portion 100a of the polyp 100 and a virtual scale corresponding to the distance D5 is to be generated and displayed, a virtual scale, which corresponds to the actual measurement of the subject positioned on the parallel plane 102, is displayed. Therefore, a virtual scale of which gradations or the like are shifted to a large side with respect to the actually measured value of the subject positioned on the extension surface 101 is displayed.

Accordingly, the offset setting unit 97 sets the height HT1 of the spot SP of the polyp 100 as an offset amount for the observation distance D5. Then, the offset distance calculation unit 98 adds the height HT1 of the spot SP1 of the polyp 100, which is an offset amount, to the observation distance D5 to calculate an offset distance D6. Accordingly, the offset distance calculation unit 98 calculates the offset distance D6 using the following equation OS). HT1 is a distance between the position P2 and a position P3.

$$\text{Equation OS) } D6=D5+HT1$$

After that, the offset virtual scale generation unit 99 generates a virtual scale, which is based on the observation distance D6, as an offset virtual scale. More specifically, the offset virtual scale generation unit 99 generates a virtual scale, which is obtained in a case where an observation distance is a distance D6, as an offset virtual scale with reference to the scale table 62. The offset virtual scale shows an actual distance to the subject positioned on the extension surface 101 or the size of the subject.

The image processing unit 93 performs processing for superimposing the generated offset virtual scale on the captured image to generate a length measurement image. It is preferable that the offset virtual scale is superimposed to be displayed at a position where the spot SP is formed for more accurate measurement. Accordingly, in a case where the offset virtual scale is to be displayed at a position away from the spot SP, the offset virtual scale is displayed close to the spot SP as much as possible. The length measurement image on which the offset virtual scale is superimposed is caused to be displayed on the augmented display 18 by the display controller 46.

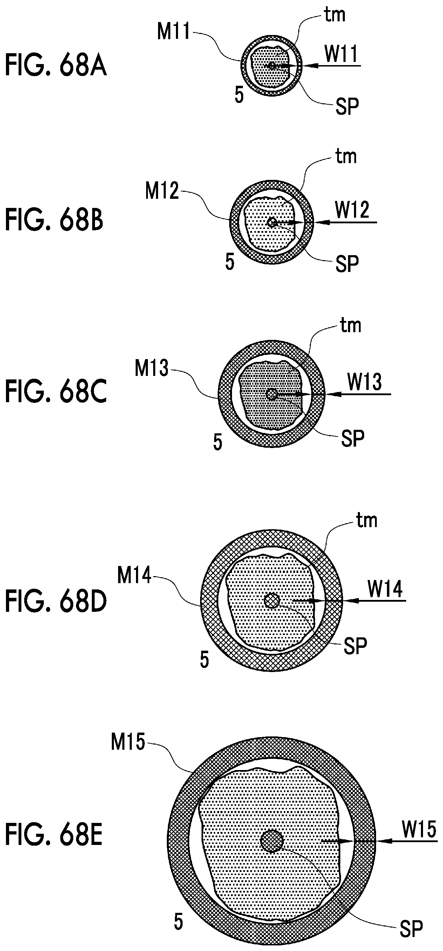

As shown in Figs. 68A to 68E, with regard to a virtual scale, the number of pixels corresponding to the width of a line of a virtual scale may be set to be larger as an observation distance is shorter, and the number of pixels corresponding to the width of a line of a virtual scale may be set to be smaller as an observation distance is longer.

In an example shown in Fig. 68A, in a case where an observation distance corresponds to the far end Pz of the range Rx and is 21 mm or more, a width W11 of a line of a virtual scale M11 is set to one pixel that is the smallest set value. In an example shown in Fig. 68B, in a case where an observation distance corresponds to a point between the far end Pz and the intermediate vicinity Py of the range Rx and is in a range of 13 mm to 20 mm, a width W12 of a line of a virtual scale M12 is set to two pixels. In an example shown in Fig. 68C, in a case where an observation distance corresponds to the intermediate vicinity Py of the range Rx and is in a range of 8 to 12 mm, a width W13 of a line of a virtual scale M13 is set to three pixels that is an intermediate value of the setting.

In an example shown in Fig. 68D, in a case where an observation distance corresponds to a point between the intermediate vicinity Py and the near end Px of the range Rx and is in a range of 4 to 7 mm, a width W14 of a line of a virtual scale M14 is set to four pixels. In an example shown in Fig. 68E, in a case where an observation distance corresponds to the near end Px of the range Rx and is 3 mm or less, a width W15 of a line of a virtual scale M15 is set to five pixels that are the largest set value.

Since the lines of the virtual scales M11 to M15 are changed according to an observation distance as described above, a medical doctor who is a user easily measures the accurate dimension of the subject. Further, since each of the widths W11 to W15 of the lines of the virtual scales M11 to M15 is set to a value inversely proportional to an observation distance, it is possible to recognize the magnitude of a dimensional error from the width of the line. For example, in consideration of the recognized error, it is understood that the size of a tumor tm is certainly smaller than the set actual sizes (5 mm or less in the examples shown in Figs. 68A to 68E) in a case where the tumor tm is positioned inside the lines of the virtual scales M11 to M15.

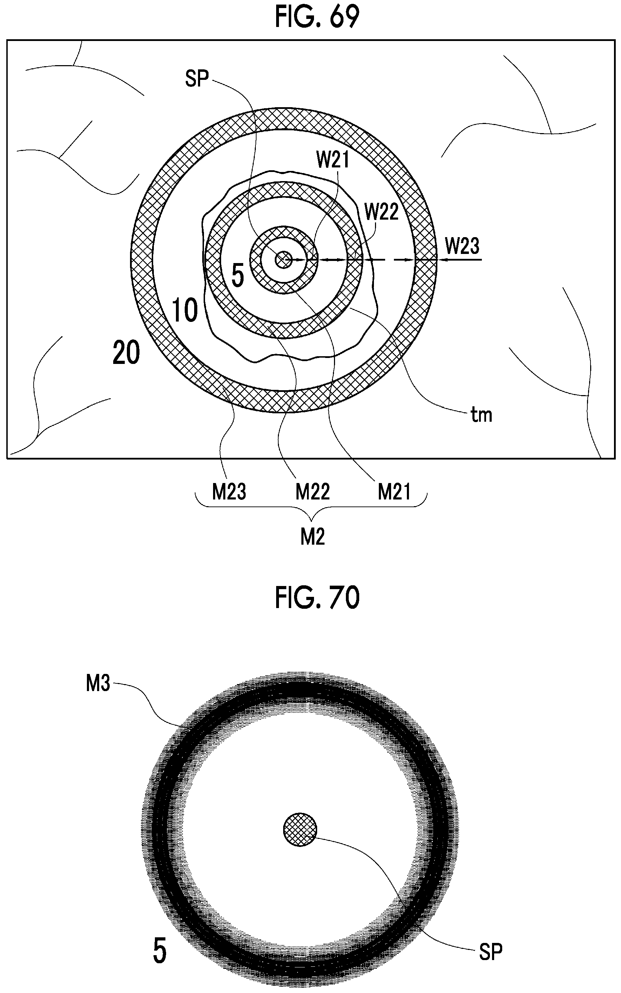

Furthermore, a concentric circular virtual scale M2 including three concentric circles having different sizes may be set on the basis of the position of one spot SP as shown in Fig. 69. The three concentric circles M21, M22, and M23 of the virtual scale M2 show actual sizes of, for example, "5 mm", "10 mm", and "20 mm". Further, each of the concentric circles M21, M22, and M23 of the virtual scale M2 is shown as a circular line with cross-hatching for the convenience of showing the virtual scale, but is actually one line filled with one color.

A width W22 of the concentric circle M22, which is positioned immediately outside the concentric circle M21 positioned on the innermost side, is set to be larger than a width W21 of the concentric circle M21, and a width W23 of the concentric circle M23 positioned on the outermost side is set to be larger than the width W22 of the concentric circle M22. In an example shown in Fig. 69, the width W23 is set to a value that is $\sqrt{2}$ times the width W22 and two times the width W21.

Each of the widths W21 to W23 is set to a value inversely proportional to an observation distance while a ratio of the widths W21 to W23 (a ratio of $W21:W22:W23=1:\sqrt{2}:2$ in the example shown in Fig. 69) is maintained. Accordingly, it is possible to recognize the magnitude of a dimensional error from the width of the line. For example, in consideration of the recognized error, it is certainly understood that the size of the tumor tm is in the range of the set actual size (10 mm or more and 20 mm or less in the example shown in Fig. 69) in a case where the tumor tm is positioned outside the line of the concentric circle M22 and inside the line of the concentric circle M23.

As shown in Fig. 70, gradation in which density is gradually lowered to the outside from the middle of a line of the virtual scale M3 in a width direction may be applied to the line of the virtual scale M3. Further, the width of the line to which gradation is applied is changed according to an observation distance as in this case.

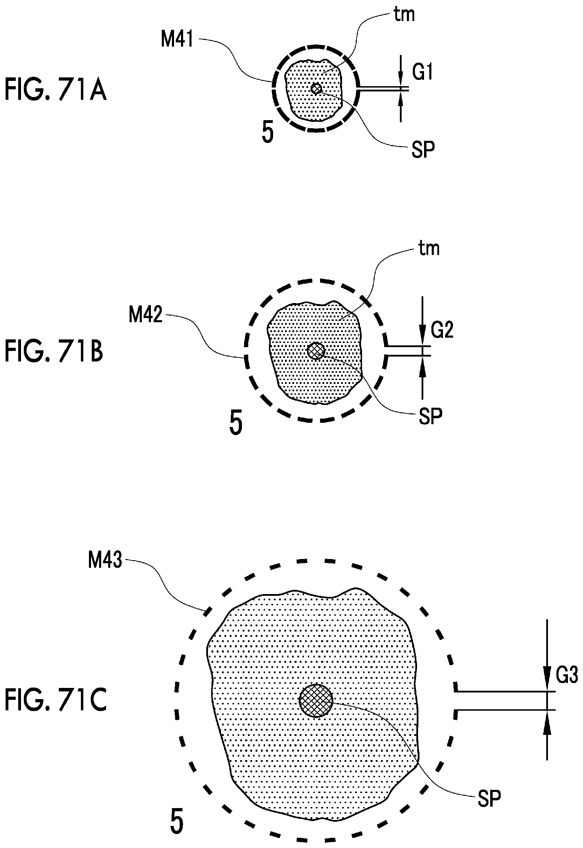

As shown in Figs. 71A to 71C, a broken line is used as each of lines of virtual scales M41 to M43 and a gap of each broken line is set to a value inversely proportional to an observation distance. Figs. 71A to 71C show the circular virtual scales M41, M42, and M43 in a case where images are picked up at the respective points of the far end Pz, the intermediate vicinity Py, and the near end Px of the range Rx of an observation distance, respectively.

In an example shown in Fig. 71A, a gap G1 of the broken line of the virtual scale M41 is set to the smallest set value in the case of the far end Pz of the range Rx. In the example shown in Fig. 71A, the virtual scale M41 is formed of a broken line. However, only in the case of the far end Pz, the gap G1 may be set to 0, that is, the virtual scale M41 may be formed of a solid line. In an example shown in Fig. 71B, a gap G2 of the broken line of the virtual scale M42 is set to an intermediate value of the setting in the case of the intermediate vicinity Py of the range Rx. In an example shown in Fig. 71C, a gap G3 of the broken line of the virtual scale M43 is set to the largest set value in the case of the near end Px of the range Rx.

Since each of the gaps G1 to G3 of the broken lines of the virtual scales M41 to M43 is set to a value inversely proportional to an observation distance as described above, it is possible to recognize the magnitude of a dimensional error from the gap of the broken line.

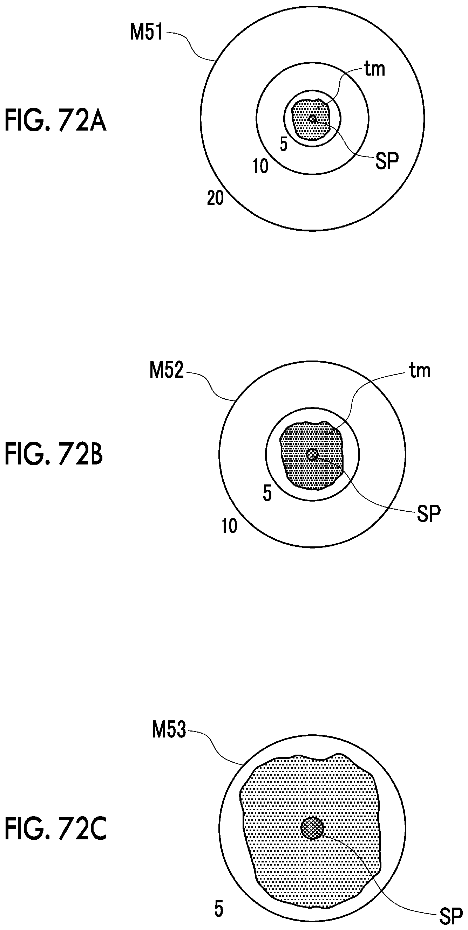

A virtual scale is formed of the same number of lines regardless of an observation distance. As shown in Figs. 72A to 72C, the numbers of lines of virtual scales M51 to M53 are changed according to an observation distance. Figs. 72A to 72C show the virtual scales M51, M52, and M53 in a case where images are picked up at the respective points of the far end Pz, the intermediate vicinity Py, and the near end Px of the range Rx of an observation distance, respectively.

In an example shown in Fig. 72A, the virtual scale M51 is formed of three lines, that is, three concentric circles having different sizes in the case of the far end Pz of the range Rx. The three concentric circles show actual sizes of, for example, "5 mm", "10 mm", and "20 mm". In an example shown in Fig. 72B, the virtual scale M52 is formed of two lines, that is, two concentric circles having different sizes in the case of the intermediate vicinity Py of the range Rx. The two concentric circles show actual sizes of, for example, "5 mm" and "10 mm". In an example shown in Fig. 72C, the virtual scale M53 is formed of one line, that is, one circular shape in the case of the near end Px of the range Rx. The one circular shape shows an actual size of, for example, "5 mm". Since the number of lines of each of the virtual scales M51 to M53 is set to a value proportional to an observation distance as described above, it is possible to recognize the magnitude of a dimensional error from the number of lines.

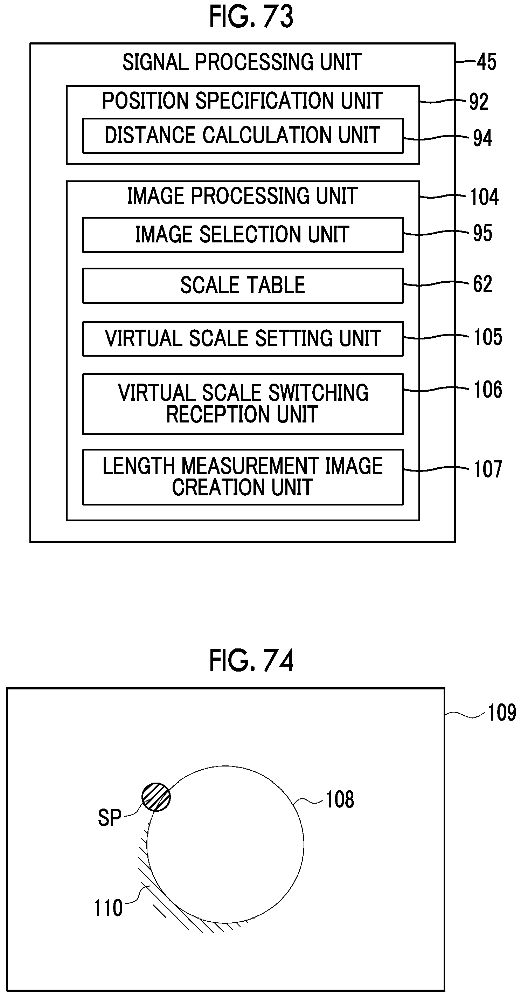

As shown in Fig. 73, the signal processing unit 45 of the augmented processor device 17 comprises a position specification unit 92 including a distance calculation unit 94 and an image processing unit 104. The image processing unit 104 includes an image selection unit 95, a scale table 62, a virtual scale setting unit 105, a virtual scale switching reception unit 106, and a length measurement image creation unit 107. The virtual scale setting unit 105 sets a virtual scale that shows the actual size of an object to be observed on the subject according to the position of the spot SP and includes gradations of which an end portion serves as a base point. The virtual scale switching reception unit 106 receives an instruction to switch and set a plurality of virtual scales. The length measurement image creation unit 107 creates a length measurement image in which the virtual scale set by the virtual scale setting unit 105 is superimposed on a captured image such that the position of the spot SP and the base point of the gradations of the virtual scale overlap with each other.

The functions of the virtual scale setting unit 105 and the length measurement image creation unit 107 will be described below. As shown in Fig. 74, a captured image 109 in which a subject including a polyp 108 is illuminated is input to the signal processing unit 45. For example, since the polyp 108 has a three-dimensional spherical shape, the captured image 109 includes polyp 108 and the spot SP and includes a shadow 110 in some cases.

The position specification unit 92 specifies the position of the spot SP on the basis of the captured image 109 input to the signal processing unit 45. The virtual scale setting unit 105 sets a virtual scale, which shows the actual size of the object to be observed corresponding to the position of the spot SP and includes gradations of which an end portion serves as a base point, with reference to the scale table 62. The end portion is a portion closer to an outer portion than a middle portion, or a starting point, an end point, or the like in the shape of the virtual scale.

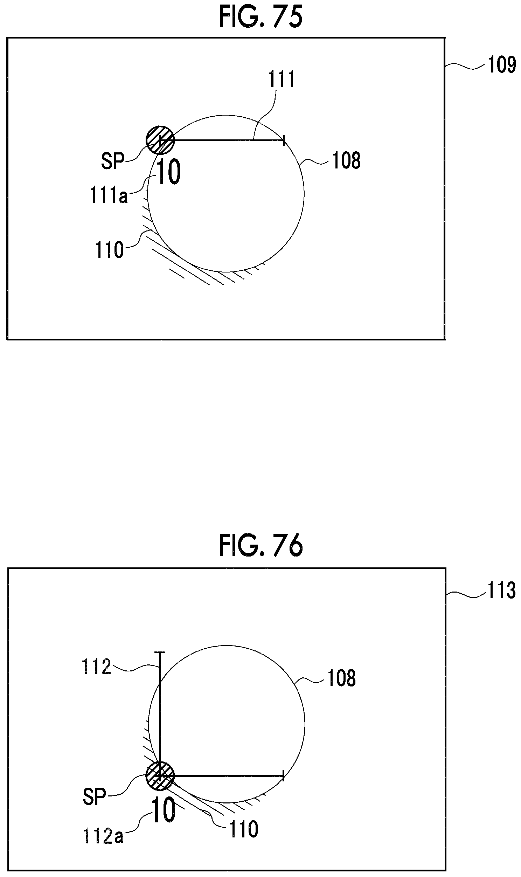

As shown in Fig. 75, the length measurement image creation unit 107 creates the length measurement image in which a virtual scale 111 set by the virtual scale setting unit 105 is superimposed on the captured image 109 such that the position of the spot SP and the base point of the gradations of the virtual scale 111 overlap with each other. It is preferable that the virtual scale 111 is superimposed to be displayed at the position of the spot SP for more accurate measurement. Accordingly, it is preferable that the virtual scale 111 is displayed close to the spot SP as much as possible even in a case where the virtual scale 111 is to be displayed at a position away from the spot SP. The virtual scale 111 is a straight line segment, and includes gradations, which are line segments perpendicular to the straight line segment, at the starting point and the end point of the line segment. In a case where the virtual scale 111 is a line segment or the like and has a starting point and an end point, the starting point and/or the end point themselves may be used as gradations. In this case, for example, gradations having the shape of a line segment perpendicular to the straight line segment may not be provided. Further, the virtual scale 111 may include a numeral "10" near the base point of the gradations. This numeral "10" is a gradation label 111a of the virtual scale 111, and is given to allow the actual size of the line segment of the virtual scale 111 to be easily recognized as 10 mm. Hereinafter, numerals included in virtual scales have the same meaning. The numerical value of the gradation label 111a can be changed by setting, and a virtual scale 111 of which a gradation label 111a itself is not displayed may be provided.

Various types of virtual scales are used depending on settings. For example, a virtual scale having the shape of a straight line segment or a shape in which straight line segments are combined with each other, a virtual scale having a circular shape or a shape in which circles are combined with each other, a virtual scale having a shape in which a circle and a straight line segment are combined with each other, and the like are used.

As shown in Fig. 76, for example, a captured image 113 includes a virtual scale 112 having a shape in which straight line segments are combined with each other. The virtual scale 112 has a shape in which straight line segments are combined in an L shape, the line segments extend upward along the plane of paper and in a direction along the plane of paper from the corner of the L shape as a base point, and the virtual scale 112 includes a gradation at each of end points of the line segments with the base point as a starting point. Further, as with the virtual scale 111, the virtual scale 112 includes a numerical value "10", which is a gradation label 112a, near the base point of the gradations.

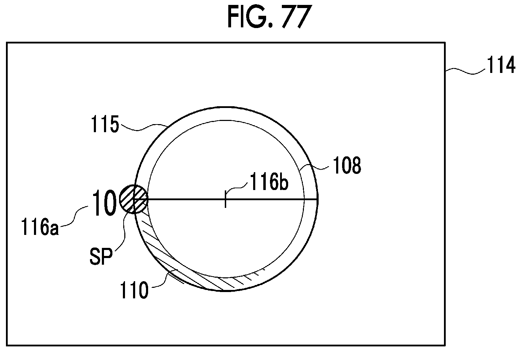

As shown in Fig. 77, for example, a captured image 114 includes a virtual scale 115 having a shape in which a straight line segment and a circle are combined with each other. The virtual scale 115 has a shape in which a circle and a line segment corresponding to the diameter of this circle are combined with each other, and intersections between the line segment and the circle serve as gradations. The virtual scale 115 may include a gradation 116 at a point where the line segment is divided in half or the center of the circle. Further, as with the virtual scale 111 or the virtual scale 112, the virtual scale 115 includes a numeral "10", which is a gradation label 116a, near the base point of the gradations. A gradation label 116b indicates a half of the gradation label 116a.

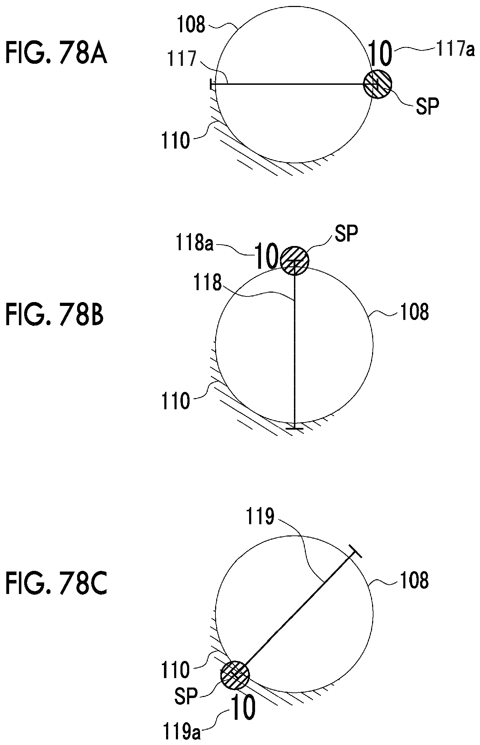

As shown in Figs. 78A to 78C, in addition to these, the virtual scale may have various shapes as with, for example, a virtual scale 117 (Fig. 78A) of which a line segment extends from a base point to the left along the plane of paper and which includes a gradation label 117a, a virtual scale 118 (Fig. 78B) of which a line segment extends downward along the plane of paper from a base point and which includes a gradation label 118a, a virtual scale 119 (Fig. 78C) of which a line segment extends from a base point in a direction oblique to the upper right side along the plane of paper and which includes a gradation label 119a, or the like.

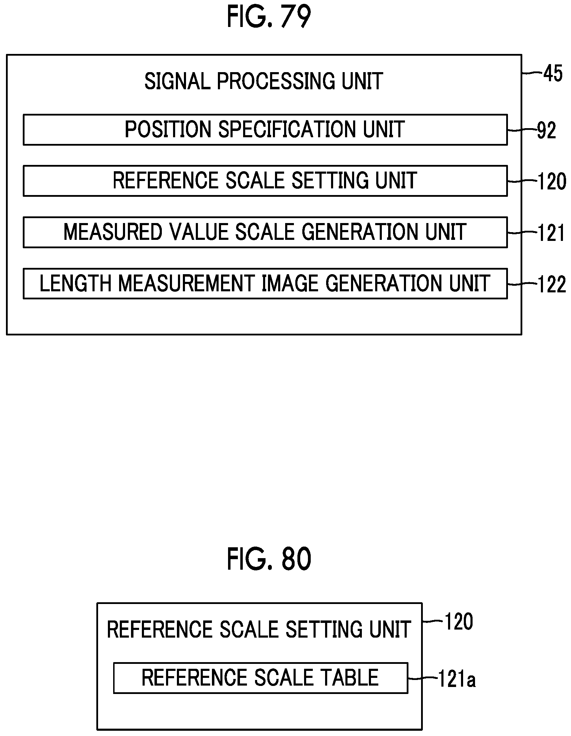

As shown in Fig. 79, the signal processing unit 45 of the augmented processor device 17 comprises a position specification unit 92, a reference scale setting unit 120, a measured value scale generation unit 121, and a length measurement image generation unit 122. The reference scale setting unit 120 sets a reference scale, which shows the actual size of the subject, on the basis of the position of the spot SP. The measured value scale generation unit 121 generates a measured value scale, which indicates a measured value obtained from the measurement of a measurement portion of a region of interest, on the basis of the set reference scale. Since the reference scale and the measured value scale are virtual elements to be displayed in a captured image, the reference scale and the measured value scale correspond to a virtual scale.

The region of interest is a region which is included in the subject and to which a user is to pay attention. The region of interest is, for example, a polyp or the like, and is a region that is likely to need to be measured. Further, a measurement portion is a portion, of which the length or the like is to be measured, of the region of interest. For example, in a case where the region of interest is a reddened portion, a measurement portion is the longest portion or the like of the reddened portion. Alternatively, in a case where the region of interest has a circular shape, a measurement portion is a diameter portion or the like of the region of interest.

The length measurement image generation unit 122 creates a length measurement image in which the measured value scale is superimposed on the captured image. The measured value scale is superimposed on the captured image to be aligned with the measurement portion of the region of interest. The length measurement image is displayed on the augmented display 18.

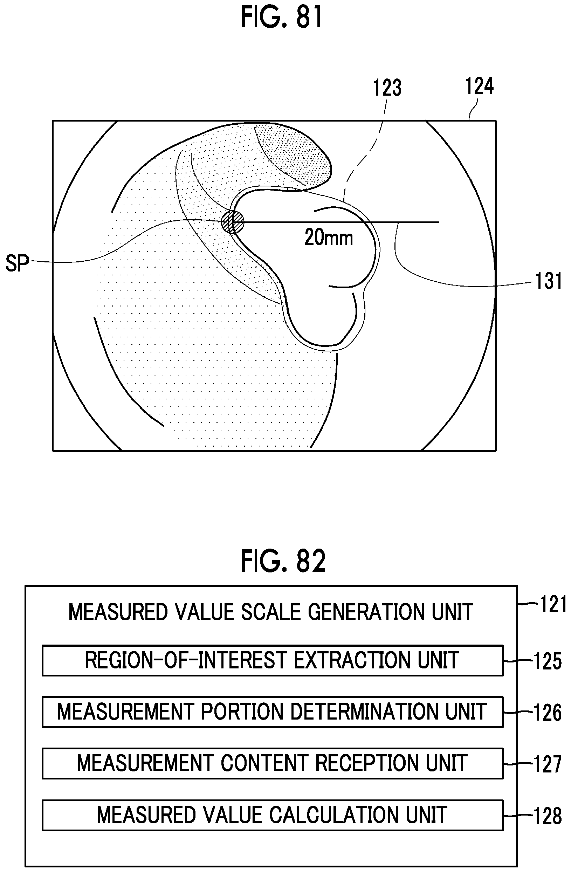

As shown in Fig. 80, the reference scale setting unit 120 comprises a reference scale table 121a. The reference scale table 121a is correspondence information in which the position of the spot SP and measurement information corresponding to the actual size of the subject are associated with each other. In the length measurement mode, the captured image 114 in which an image of the subject including a polyp 123 as an object to be observed is picked up is input to the signal processing unit 45. As shown in Fig. 81, in a captured image 124, a polyp 123 has, for example, a three-dimensional shape in which spheres overlap with each other. For example, a spot SP is formed at the end portion of the polyp 123. The position specification unit 92 specifies the position of the spot SP on the basis of the captured image 124. The reference scale setting unit 120 sets a reference scale 131, which corresponds to the specified position of the spot SP and shows the actual size of the subject, with reference to the reference scale table 121a.

The reference scale 131 includes, for example, a line segment that has the number of pixels corresponding to an actual size of 20 mm and a numerical value and a unit that represent the actual size. The reference scale 131 is not usually displayed on the augmented display 18 but is displayed like the captured image 124 in a case where the reference scale 131 is displayed on the augmented display 18.

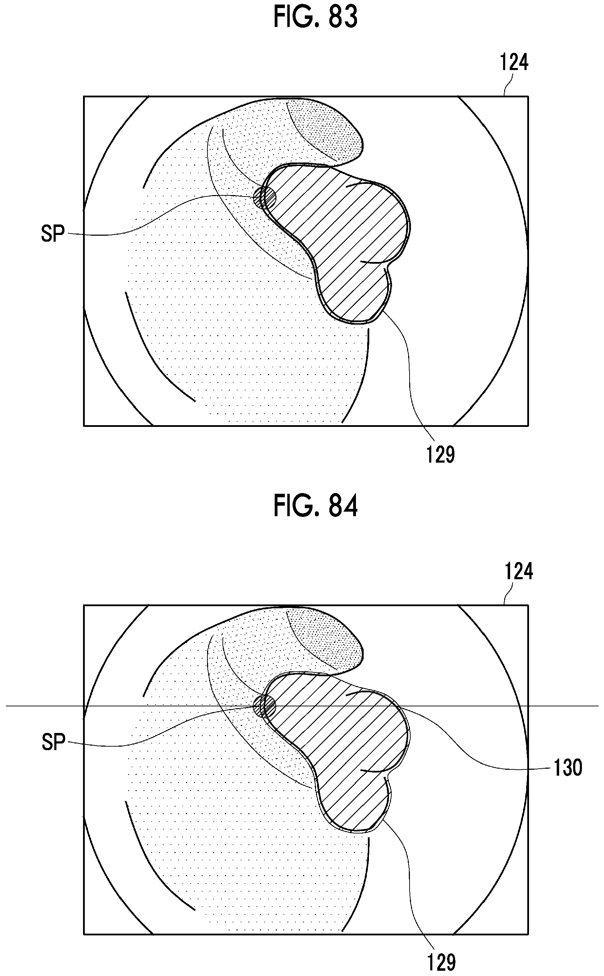

As shown in Fig. 82, the measured value scale generation unit 121 comprises a region-of-interest extraction unit 125, a measurement portion determination unit 126, a measurement content reception unit 127, and a measured value calculation unit 128. As shown in Fig. 83, the region-of-interest extraction unit 125 extracts a hatched region as a region 129 of interest as in the captured image 124. Then, as shown in Fig. 84, for example, in a case where a preset reference is a reference that is used to measure a portion of the region of interest in the horizontal direction with the spot SP as a base point, the measurement portion determination unit 126 extracts a horizontal edge position 130 with the spot SP as a base point as in the captured image 124. A portion between the spot SP and the horizontal edge position 130 is a measurement portion.

For example, in a case where the actual size of the reference scale is denoted by L0, the number of pixels of the reference scale 131 in the captured image 124 is denoted by Aa, the number of pixels of the measurement portion in a case where the reference scale 131 is superimposed on the region 129 of interest in the captured image 124 is denoted by Ba, and the actual size of a measured value scale 132 is denoted by L1, the measured value calculation unit 128 generates the measured value scale 132 such that the following equation (K1) is satisfied.

$$\text{Equation (K1)} \quad L1 = L0 \times Ba/Aa$$

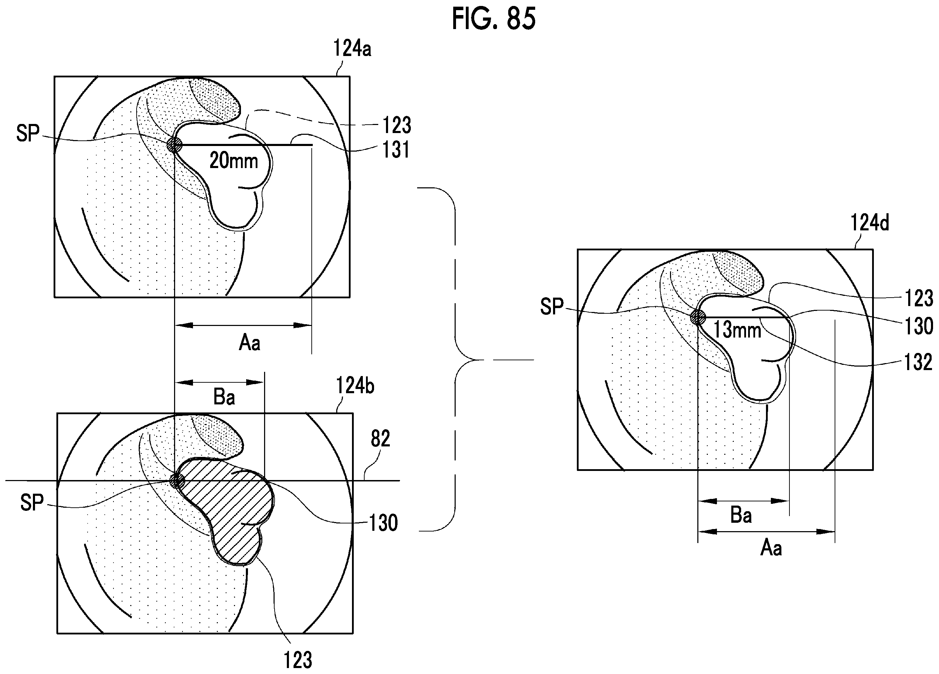

As shown in Fig. 85, for example, in a case where Ba/Aa is 0.7 and the actual size of the reference scale 131 is 20 mm, the measured value calculation unit 128 calculates the actual size of the measured value scale 132 as 13 mm as in a captured image 124d by using the number Aa of pixels corresponding to the reference scale 131 shown in a captured image 124a and the number Bb of pixels corresponding to the measurement portion between the spot SP and the horizontal edge position 130 shown in a captured image 124b.

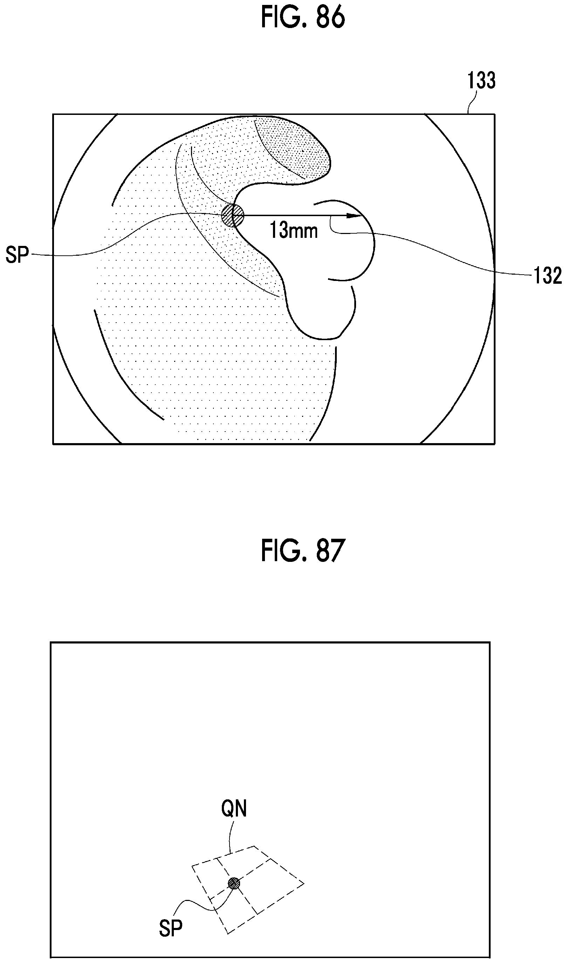

The length measurement image generation unit 122 generates a length measurement image 133 in which the measured value scale 132 is superimposed on the captured image 124. For example, as shown in Fig. 86, the measured value scale 132 is superimposed on the captured image 124 as a figure, such as an arrow, having the shape of a straight line segment. The length measurement image 133 may include a numerical value of the actual size of the measured value scale 132. The numerical value of the actual size of the measured value scale 132 may be superimposed on the captured image 124 in a state where the numerical value is separated from the figure, such as an arrow.

The type of the measured value scale 132 can be selected from a plurality of types. The measurement content reception unit 127 receives the setting of the contents of the measured value scale and sends the contents of the measured value scale to the measured value scale generation unit 121, and the length measurement image generation unit 122 generates the length measurement image 133 using the measured value scale 132 that is generated by the measured value scale generation unit 121 on the basis of the contents of the measured value scale.

It is preferable that the region-of-interest extraction unit 125 extracts a region of interest using a trained model trained using captured images acquired in the past. Various models suitable for image recognition using machine learning can be used as a model used as the trained model. A model using a neural network can be preferably used for the purpose of recognizing a region of interest in an image. In a case where these models are to be trained, these models are trained using captured images, which include information about the region of interest, as teacher data. Examples of the information about the region of interest include the presence or absence of the region of interest, the position or range of the region of interest, and the like. Some models may be trained using captured images not including the information about the region of interest.

Further, it is preferable that the measurement portion determination unit 126 also determines a measurement portion using a trained model trained using captured images acquired in the past. Models and the like used as the trained model are the same as those of the region-of-interest extraction unit. However, in a case where these models are to be trained, these models are trained using captured images that include information about the measurement portion. The information about the measurement portion includes a measured value and the measurement portion. Some models may be trained using captured images not including the information about the measurement portion. The trained model used by the region-of-interest extraction unit 125 and the trained model used by the measurement portion determination unit 126 may be common. In a case where a purpose is to extract the measurement portion, one trained model may be adapted to extract the measurement portion without extracting the region of interest from the captured image 124.

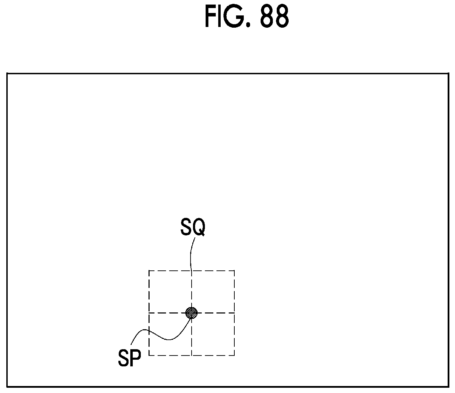

In the second signal processing unit 60, the scale table 62, which is used to display a virtual scale deformed according to the position of the spot SP, is updated from the representative point data table 66 in which the irradiation position of measurement light and the representative points of a virtual scale are stored (see Figs. 39 and 40). However, the scale table 62 may be created with other methods. For example, as shown in Fig. 87, a distorted grid region QN in which a circular virtual scale centered on the spot SP is inscribed is acquired from an image that is obtained in a case where an image of a square grid-like chart is picked up. A grid of the distorted grid region QN is further distorted due to the distortion of the image pickup optical system 21 as a distance between the grid and the center of a screen is increased. The distorted grid region QN is converted into a square grid region SQ shown in Fig. 88 by an affine transformation matrix. The coordinates of points indicating a circular virtual scale are calculated in the square grid region SQ. Then, the coordinates of the points of the virtual scale in the square grid region SQ are converted into a distorted circular virtual scale, which is distorted by the image pickup optical system 21, by an inverse matrix of the affine transformation matrix. The coordinates of this distorted circular virtual scale and the position of the spot SP are stored in the scale table 62 in association with each other.

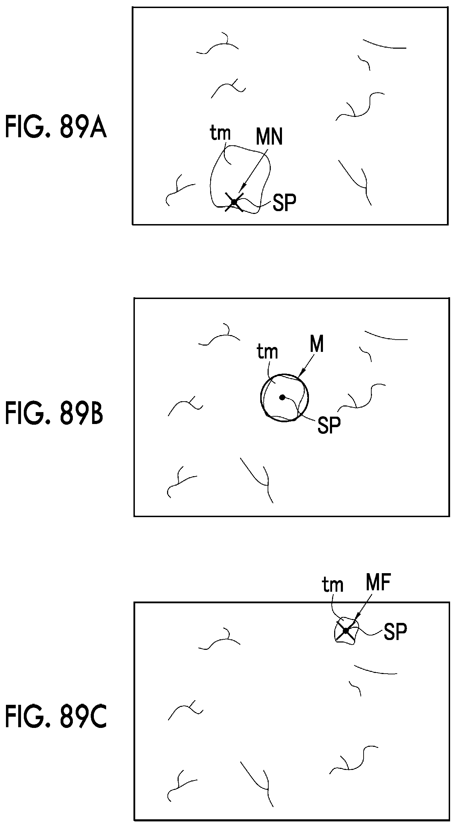

In consideration of the distortion of the image pickup optical system 21, the display aspect of a virtual scale may be changed between a region in which measurement using the virtual scale is effective and other regions. Specifically, in a case where a spot SP is present outside a range of an effective measurement region (near end Px side) as shown in Fig. 89A and a case where a spot SP is present outside a range of an effective measurement region (far end Pz side) as shown in Fig. 89C, the measurement of a tumor tm using the virtual scale is not effective. Accordingly, cruciform virtual scales MN and MF are displayed, respectively. On the other hand, in a case where a spot SP is present in a region in which measurement using a circular virtual scale M is effective, the circular virtual scale M is displayed as shown in Fig. 89B.

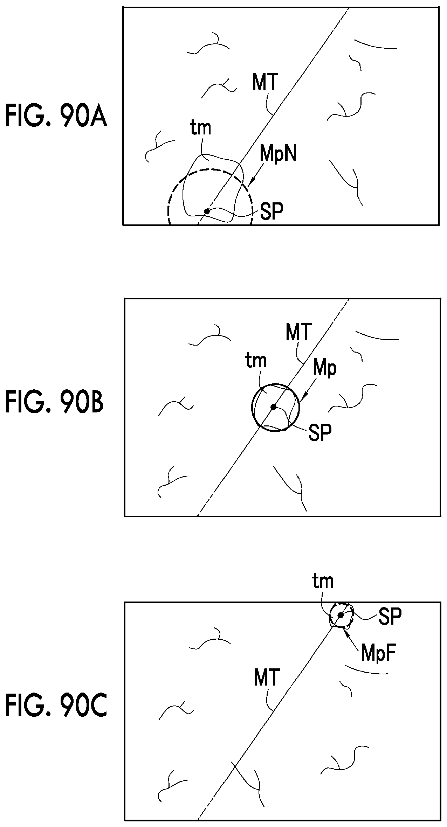

Furthermore, the type of a line of the virtual scale may be changed depending on whether the spot SP is present inside or outside the range of the effective measurement region. In this case, it is preferable that a movement locus MT of the spot SP is displayed as shown in Figs. 90A to 90C so that a change in the type of the line of the virtual scale can be known. In a case where a spot SP is present outside a range of an effective measurement region (near end Px side) as shown in Fig. 90A and a case where a spot SP is present outside a range of an effective measurement region (far end Pz side) as shown in Fig. 90C, the measurement of a tumor tm using the virtual scale is not effective. Accordingly, circular virtual scales MpN and MpF are displayed with dotted lines, respectively. On the other hand, in a case where a spot SP is present in a region in which measurement using a circular virtual scale Mp is effective, the circular virtual scale Mp is displayed with a solid line as shown in Fig. 90B. The type of the line of the virtual scale is changed between a dotted line and a solid line depending on whether the spot SP is present inside or outside the range of the effective measurement region, but virtual scales may be displayed with different colors. For example, the type of the line of the virtual scale may be set to blue in a case where the spot SP is present outside the range of the effective measurement region, and the type of the line of the virtual scale may be set to white in a case where the spot SP is present inside the range of the effective measurement region.

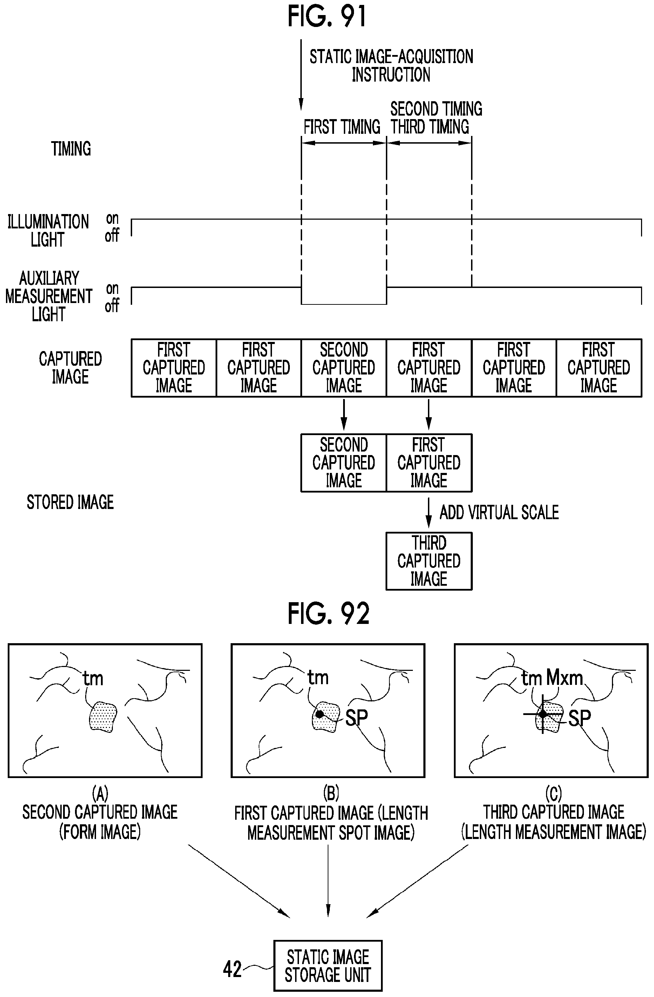

The details of the acquisition of a static image in the length measurement mode will be described. In a case where a static image-acquisition instruction is not given, the system controller 41 controls the light source device 13 to emit illumination light and measurement light. As shown in Fig. 91, in a case where the static image-acquisition instruction switch 12g is operated to give a static image-acquisition instruction, illumination light is turned on (on) but measurement light is turned off (off) in a first timing including the static image-acquisition instruction. In a second timing and a third timing after the first timing passes, measurement light is turned on again while the turning on of illumination light is maintained. The second timing and the third timing are the same timing, but may be different timings.

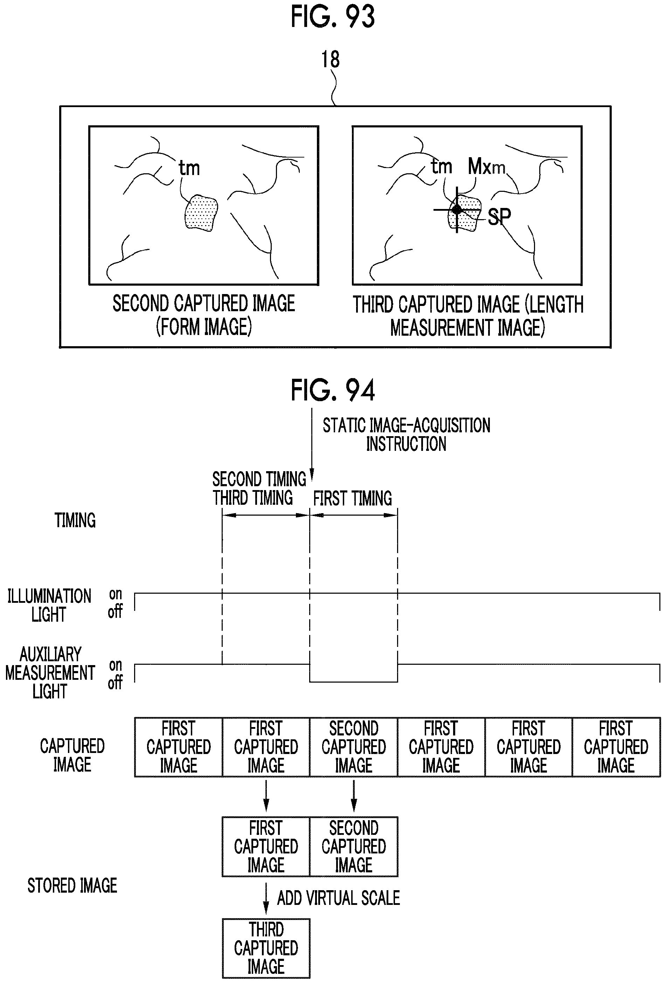

A second captured image obtained from the image pickup of a subject illuminated with measurement light is obtained in the first timing. A first captured image obtained from the image pickup of the subject illuminated with illumination light and measurement light is obtained in the second timing and the third timing. Then, as shown in Figs. 91 and 92, the system controller 41 stores a static image of the first captured image and a static image of the second captured image as stored images that are to be stored in the static image storage unit 42. Further, the signal processing unit 45 of the augmented processor device 17 acquires a static image of a third captured image in which a virtual scale Mxm set according to the position of a spot SP is displayed in the first captured image. The static image of the third captured image is sent to the processor device 14 and is stored in the static image storage unit 42. Furthermore, in order to notify a user that the static images are recorded, as shown in Fig. 93, the display controller 46 causes the augmented display 18 to display the second captured image and the third captured image for a certain period of time after the static images are stored. It is preferable that at least two of the first captured image, the second captured image, and the third captured image are stored in the static image storage unit 42 in response to one static image-acquisition instruction. For example, it is preferable that two captured images, that is, the second captured image and the third captured image are stored. Moreover, the third captured image corresponds to a stored image, which is to be stored in the static image storage unit 42, of the length measurement image on which the virtual scale is superimposed and displayed as described above.

As shown in Fig. 94, the second timing or the third timing may be earlier than the first timing. In this case, the first captured image of several frames corresponding to the second timing or the third timing needs to be stored in a temporary storage unit (not shown) of the processor device 14 in the length measurement mode. In a case where a static image-acquisition instruction is given, the first captured image stored in the temporary storage unit is stored in the static image storage unit 42 as the first captured image of the second timing and the third captured image in which a virtual scale is added to the first captured image stored in the temporary storage unit is stored in the static image storage unit 42.

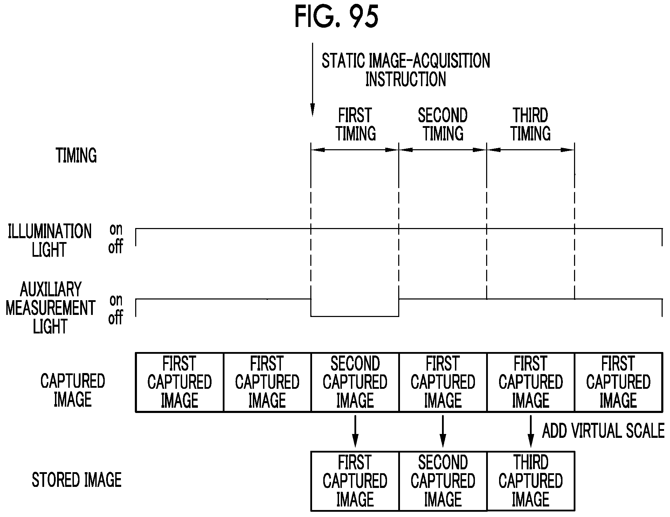

Further, a second timing and a third timing may be timings different from each other as shown in Fig. 95. In this case, a first captured image obtained in the second timing is stored in the static image storage unit 42 in the same manner as described above. Further, before a first captured image obtained in the third timing is stored in the static image storage unit 42, a virtual scale is added to the first captured image so that the first captured image is converted into a third captured image. Then, the third captured image is stored in the static image storage unit 42.

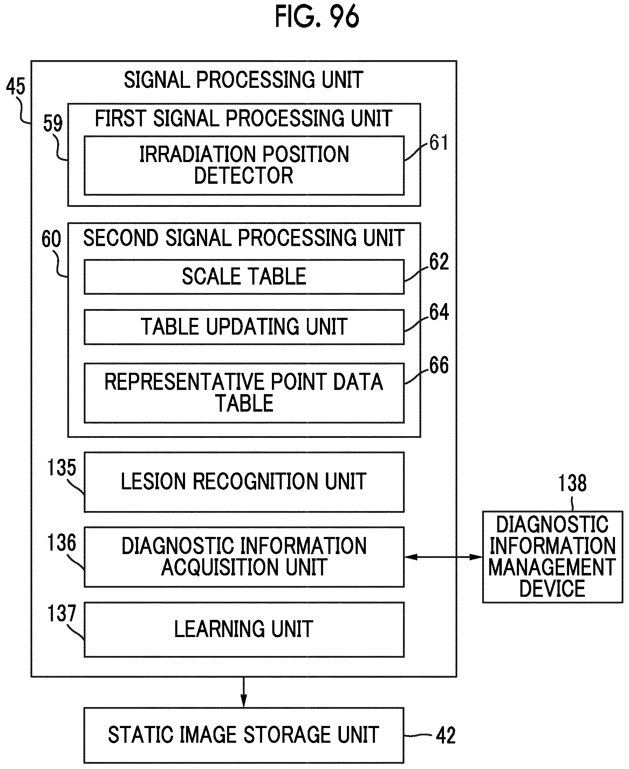

As shown in Fig. 96, the signal processing unit 45 of the augmented processor device 17 may be provided with a lesion recognition unit 135, a diagnostic information acquisition unit 136, and a learning unit 137 in addition to a first signal processing unit 59 and a second signal processing unit 60. The lesion recognition unit 135 performs image processing on the first captured image (an image based on illumination light and measurement light) to perform recognition processing. Detection processing of detecting a region of interest, such as a lesion portion, from the first captured image is performed as the recognition processing that is performed by the lesion recognition unit 135. It is preferable that a learning model subjected to machine learning is used for the recognition processing. That is, the detection result of the region of interest is output from the learning model in response to the input of the first captured image to the learning model. It is preferable that the learning model is a learning model subjected to machine learning, such as convolutional neural network (CNN). The recognition processing performed by the lesion recognition unit 135 may be discrimination processing for discriminating the degree of progression of a lesion of the lesion portion recognized from the first captured image. Further, the lesion recognition unit 135 may perform image processing on the second captured image (an image based on only illumination light) to perform recognition processing.

The diagnostic information acquisition unit 136 acquires diagnostic information about the first captured image or the second captured image from a diagnostic information management device 138. The diagnostic information acquisition unit 136 acquires a medical chart of a patient who is an object to be examined as the diagnostic information. The medical chart is information in which the progress and the like of medical care or examination for a patient are recorded, and includes, for example, a record, such as the name, the gender and age, the name of a disease, major symptoms, the contents of prescription or treatment, or the medical history of a patient. Information about the lesion portion that is subjected to recognition processing by the lesion recognition unit 135, and diagnostic information about the first captured image or the second captured image, which is acquired by the diagnostic information acquisition unit 136, are stored in the static image storage unit 42 as attached data of a data set DS in association with the first captured image or the second captured image.

The learning unit 137 performs machine learning using the first captured image or the second captured image that is stored in the static image storage unit 42 and the attached data (data set) that are associated with these first and second captured images. Specifically, the learning unit 137 performs machine learning on the learning model of the lesion recognition unit 135. It is preferable that the second captured image is used as a teacher data candidate for machine learning. Since the second captured image is an image that is obtained in response to a static image-acquisition instruction during the measurement of a tumor tm or the like, the second captured image is an image in which a region of interest as an object to be observed is highly likely to be included. Further, since the second captured image is a normal endoscopic image that is obtained in a case where measurement light is not emitted, the second captured image is highly useful as teacher data for machine learning. Furthermore, since information about a lesion portion, diagnostic information, and the like are also attached as the attached data, a user does not need to input the information about a lesion portion, the diagnostic information, and the like in a case where machine learning is performed. Since the second captured image as a teacher data candidate is accumulated, the accuracy of recognition processing performed by the lesion recognition unit 135 is improved as machine learning is performed. In a case where the first captured image is to be used as a teacher data candidate for machine learning, the first captured image may be used as it is but it is more preferable that a portion other than an irradiation region of measurement light is used as a teacher data candidate.

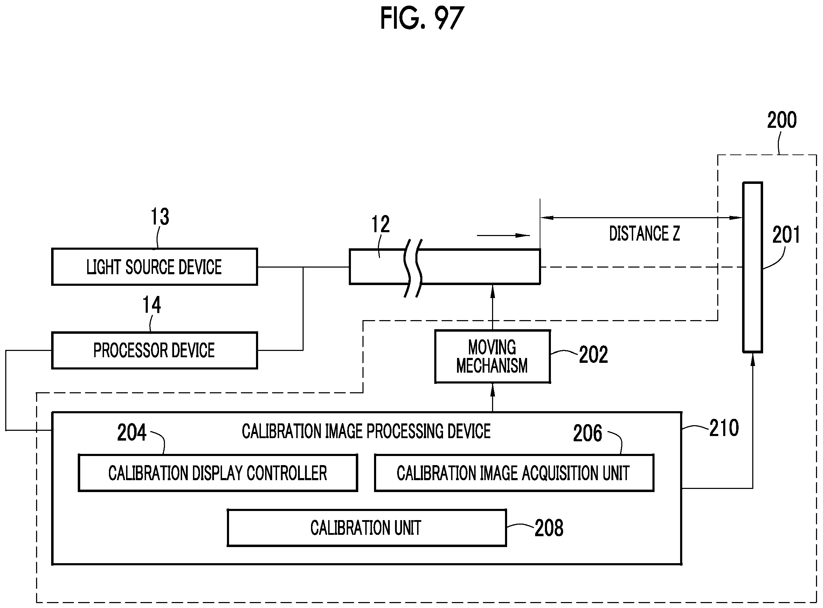

A calibration method of creating the representative point data table 66 using a calibration apparatus 200 shown in Fig. 97 will be described below. The calibration apparatus 200 comprises a calibration display 201, a moving mechanism 202, a calibration display controller 204, a calibration image acquisition unit 206, and a calibration unit 208. The calibration display controller 204, the calibration image acquisition unit 206, and the calibration unit 208 are provided in a calibration image processing device 210. The calibration image processing device 210 is electrically connected to the processor device 14, the calibration display 201, and the moving mechanism 202.

The moving mechanism 202 includes a holding unit (not shown) that holds the distal end part 12d of the endoscope 12 toward the calibration display 201, and moves the holding unit at specific intervals to change a distance Z between the distal end part 12d of the endoscope 12 and the calibration display 201. Whenever the distance Z is changed by the moving mechanism 202, the calibration display controller 204 displays an image of a virtual scale of a first display aspect, which is not affected by the image pickup optical system 21, at the irradiation position of measurement light on the calibration display 201. Since an influence of distortion or the like caused by the image pickup optical system 21 is not considered for the image of the virtual scale of the first display aspect, the image of the virtual scale of the first display aspect is not displayed with a size, a shape, or the like corresponding to a scale display position in a case where the image of the virtual scale of the first display aspect is displayed on the augmented display 18.

The calibration image acquisition unit 206 acquires a calibration image, which is obtained from the image pickup of the virtual scale of the first display aspect displayed on the calibration display 201, by the endoscope 12. In a case where the endoscope 12 picks up an image whenever the distance Z is changed, that is, whenever the virtual scale of the first display aspect is displayed, the calibration image is acquired. For example, in a case where the virtual scale of the first display aspect is displayed n times, n calibration images are obtained.

An image of a virtual scale of a second display aspect, which is affected by the image pickup optical system 21, is included at the irradiation position of measurement light in the calibration image. Since an influence of distortion or the like caused by the image pickup optical system 21 is considered for the image of the virtual scale of the second display aspect, the image of the virtual scale of the second display aspect is displayed with a size, a shape, or the like corresponding to a scale display position.

The calibration unit 208 calibrates the display of the virtual scale on the augmented display 18 on the basis of the calibration image acquired by the calibration image acquisition unit 206. Specifically, in the calibration unit 208, a representative point data table, which is created by representative point extraction processing and table creation processing, is sent to the augmented processor device 17 and is stored in the representative point data table 66. The representative point extraction processing is to extract representative points from the image of the virtual scale of the second display aspect included in the calibration image, and the table creation processing is to create a representative point data table by associating representative point data related to the representative points with an irradiation position in a timing when the calibration image is acquired.

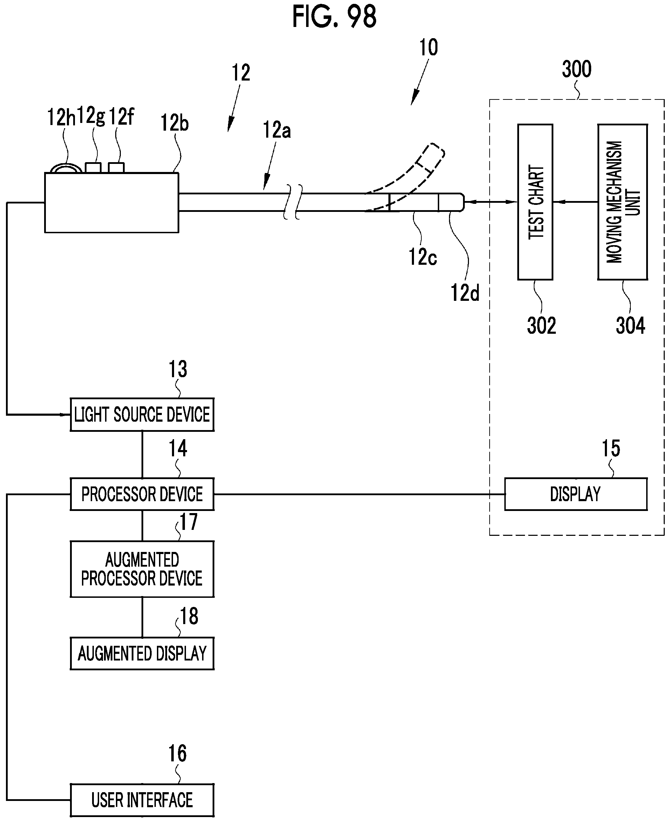

As shown in Fig. 98, an inspection system 300 is used for the inspection of the accuracy of a scale, such as whether or not a virtual scale has a predetermined shape. The inspection system 300 comprises a test chart 302, the display 15, and a moving mechanism unit 304. The display 15 is shared with the endoscope system 10, but a display for accuracy inspection may be separately provided.

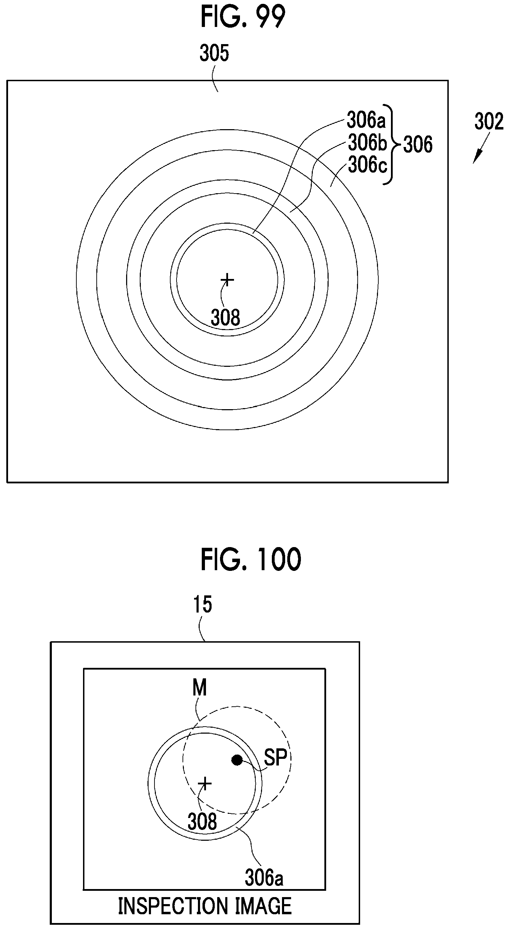

As shown in Fig. 99, the test chart 302 includes a chart body 305, and the chart body 305 is provided with an inspection region portion 306 that includes inspection regions having a specific shape and an inspection reference position 308 that is used as a reference to be aligned with the irradiation position of measurement light during accuracy inspection. The inspection region portion 306 comprises three circular inspection regions 306a, 306b, and 306c as the inspection regions having a specific shape. These three inspection regions 306a, 306b, and 306c are concentrically provided to be centered on the inspection reference position 308. The chart body 205 is illuminated with measurement light (for example, a spot SP) emitted from the endoscope 12 used for a test for confirming a virtual scale of 5 mm (indicating that a diameter is "5 mm"), a virtual scale of 10 mm (indicating that a diameter is "10 mm"), and a virtual scale of 20 mm (indicating that a diameter is "20 mm") and an image of the chart body 205 is picked up, so that inspection images of the inspection regions 306a, 306b, and 306c are acquired.

As shown in Fig. 100, the inspection image is displayed on the display 15. A virtual scale M corresponding to the irradiation position of measurement light (the position of the spot SP) is displayed in the inspection image in addition to the inspection region portion 306 and the inspection reference position 308. During accuracy inspection, the test chart 302 is moved by the moving mechanism unit 304 such that the irradiation position of measurement light (the position of the spot SP) is aligned with the inspection reference position. In a case where the irradiation position of measurement light coincides with the inspection reference position, a user determines whether or not the virtual scale M is properly displayed.

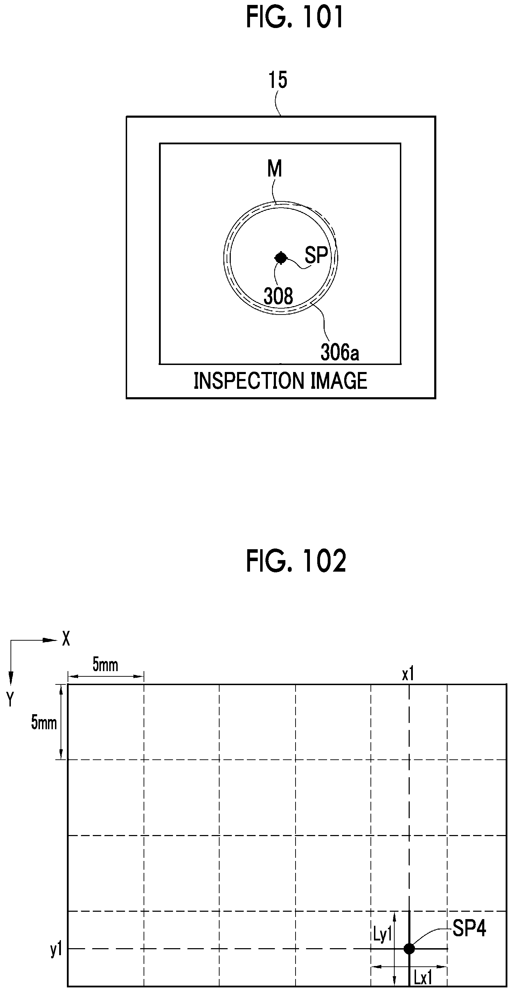

For example, in a case where the irradiation position of measurement light is present at the inspection reference position 308 in the inspection image and the virtual scale M enters the inspection region 306a, a user determines that the virtual scale M is properly displayed. On the other hand, in a case where even a part of the virtual scale M does not enter the inspection region 306a, such as a case where even a part of the virtual scale M protrudes from the inspection region 306a, as shown in Fig. 101, a user determines that the virtual scale M of 5 mm is not properly displayed.

The scale table 62 may be created as follows. A relationship between the position of a spot and the size of a virtual scale can be obtained from the image pickup of a chart in which a pattern having an actual size is regularly formed. For example, spot-like measurement light is emitted to the chart; the image of a graph paper-shaped chart including ruled lines (5 mm) having the same size as the actual size or ruled lines (for example, 1 mm) having a size smaller than the actual size is picked up while an observation distance is changed to change the position of a spot; and a relationship between the position of the spot (the pixel coordinates of the spot on the image pickup surface of the image pickup element 32) and the number of pixels corresponding to the actual size (how many pixels are used to represent an actual size of 5 mm?) is acquired.

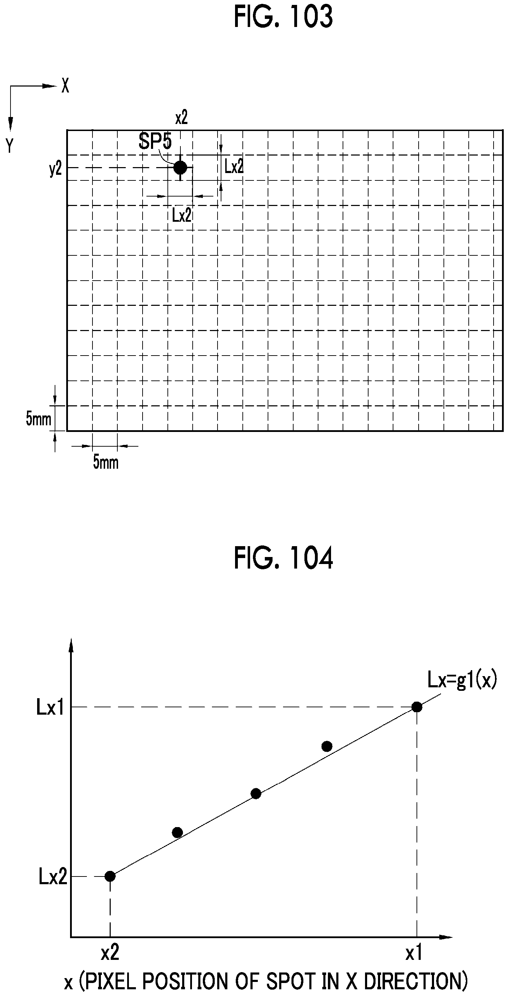

As shown in Fig. 102, $(x1, y1)$ is the pixel position of a spot SP4 in X and Y directions on the image pickup surface of the image pickup element 32 (an upper left point is the origin of a coordinate system). The number of pixels in the X direction corresponding to an actual size of 5 mm at the position $(x1, y1)$ of the spot SP4 is denoted by Lx1, and the number of the pixels in the Y direction corresponding to the actual size at the position is denoted by Ly1. Such a measurement is repeated while an observation distance is changed. Fig. 103 shows a state where the image of a chart including ruled lines having the same interval, which is 5 mm, as that in Fig. 102 is picked up, but is a state where an image pickup distance is closer to the far end than in the state shown in Fig. 102 and an interval between the ruled lines is displayed so as to be narrower. In the state shown in Fig. 103, the number of pixels in the X direction corresponding to an actual size of 5 mm at the position $(x2, y2)$ of a spot SP5 on the image pickup surface of the image pickup element 32 is denoted by Lx2, and the number of the pixels in the Y direction corresponding to the actual size at the position is denoted by Ly2. Further, measurement shown in Figs. 102 and 103 is repeated while an observation distance is changed; and results thereof are plotted. In Figs. 102 and 103, the images of the chart are displayed without the consideration of the distortion of the image pickup optical system 21.

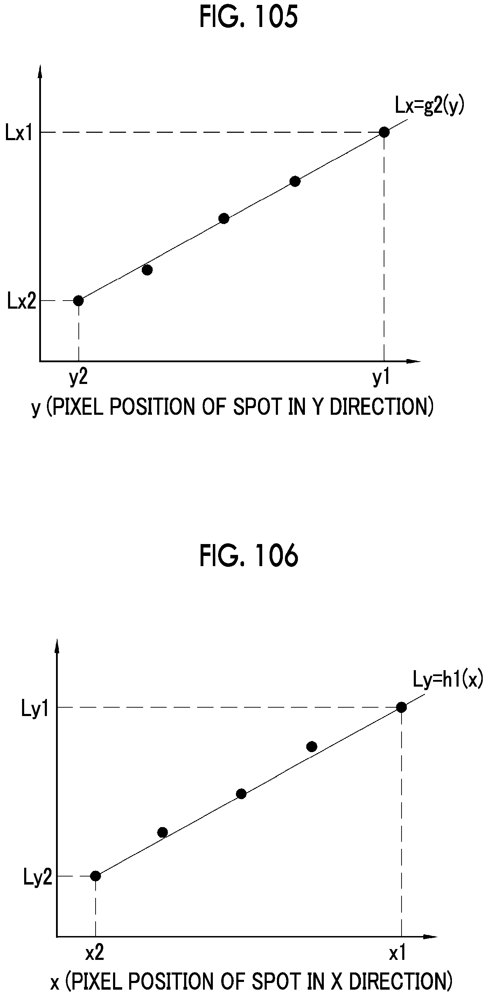

Fig. 104 shows a relationship between the X-coordinate of the position of a spot and Lx (the number of pixels of the first virtual scale in the X direction), and Fig. 105 shows a relationship between the Y-coordinate of the position of a spot and Lx. Lx is expressed as Lx=g1(x) from the relationship shown in Fig. 104 as a function of a position in the X direction, and Ly is expressed as Ly=g2(y) from the relationship shown in Fig. 105 as a function of a position in the Y direction. g1 and g2 can be obtained from the above-mentioned plotted results by, for example, a least-squares method.

Since the X-coordinate and the Y-coordinate of a spot have a one-to-one correspondence, basically the same results (the same number of pixels for the same position of a spot) are obtained even though any one of the function g1 or g2 is used. Accordingly, in a case where the size of the first virtual scale is to be calculated, either function may be used and a function having higher sensitivity of a change in the number of pixels to a change in a position may be selected between g1 and g2. Further, in a case where the values of g1 and g2 are significantly different from each other, it may be determined that "the position of a spot could not be recognized".

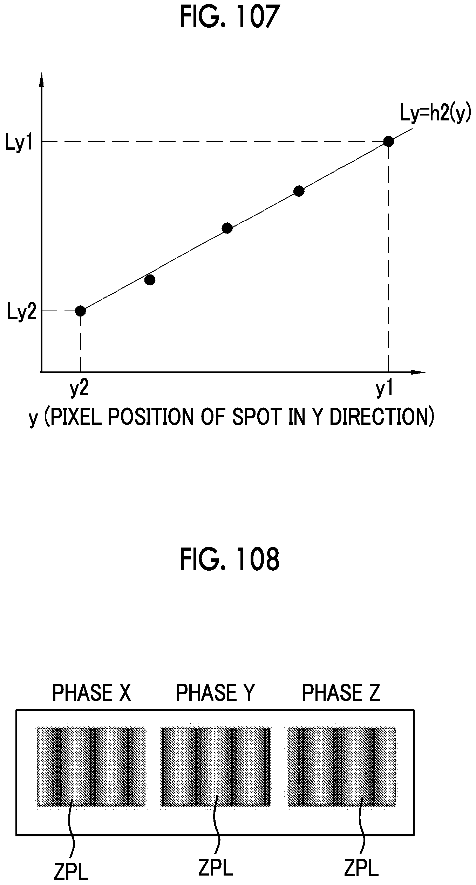

Fig. 106 shows a relationship between the X-coordinate of the position of a spot and Ly (the number of pixels in the Y direction), and Fig. 107 shows a relationship between the Y-coordinate of the position of a spot and Ly. Ly is expressed as Ly=h1(x) from the relationship shown in Fig. 106 as the coordinate of a position in the X direction, and Ly is expressed as Ly=h2(y) from the relationship shown in Fig. 108 as the coordinate of a position in the Y direction. Any one of the function h1 or h2 may be used as Ly as with Lx.

The functions g1, g2, h1, and h2 obtained as described above are stored in the scale table 62 in a look-up table format. The functions g1 and g2 may be stored in the scale table 62 in a function format.

Stripe-pattern light ZPL, which is formed as light having a stripe pattern on a subject as shown in Fig. 108 in a case where the subject is irradiated with the light, may be used as measurement light (for example, see JP2016-198304A). The stripe-pattern light ZPL is obtained in a case where a variable-transmittance liquid crystal shutter (not shown) is irradiated with specific laser light, and is formed of two different vertical stripe patterns in which a region (transmissive region) through which specific laser light is transmitted by the liquid crystal shutter and a region (non-transmissive region) through which the specific laser light is not transmitted are periodically repeated in the horizontal direction. In a case where the stripe-pattern light is used as the measurement light, the period of the stripe-pattern light changes depending on a distance from the subject. Accordingly, the subject is irradiated with the stripe-pattern light plural times while the period or phase of the stripe-pattern light is shifted by the liquid crystal shutter, and the three-dimensional shape of the subject is measured on the basis of a plurality of images obtained using the shift of the period or phase.

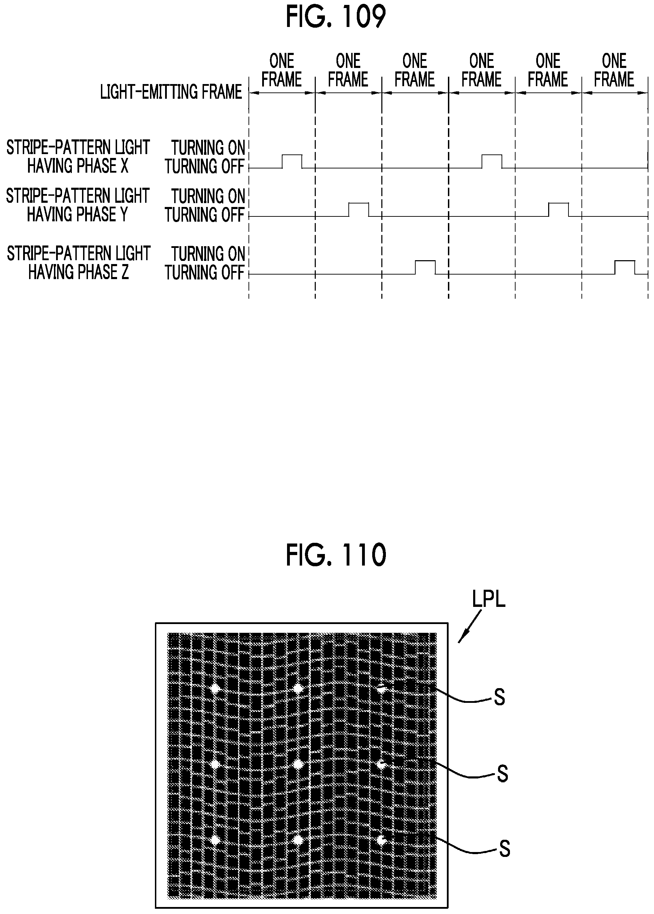

For example, a subject is alternately irradiated with stripe-pattern light having a phase X, stripe-pattern light having a phase Y, and stripe-pattern light having a phase Z. The phases of the vertical stripe patterns of the stripe-pattern light having the phase X, the stripe-pattern light having the phase Y, and stripe-pattern light having the phase Z are shifted from each other by 120° (2π/3). In this case, the three-dimensional shape of the subject is measured using three types of images obtained on the basis of the respective types of stripe-pattern light. For example, it is preferable that the subject is irradiated with the stripe-pattern light having the phase X, the stripe-pattern light having the phase Y, and the stripe-pattern light having the phase Z while the stripe-pattern light having the phase X, the stripe-pattern light having the phase Y, and the stripe-pattern light having the phase Z are switched every frame (or every few frames) as shown in Fig. 109. It is preferable that the subject is constantly irradiated with the illumination light.

Grid-pattern measurement light LPL, which is formed as a grid pattern as shown in Fig. 110 in a case where a subject is irradiated with the light, may be used as the measurement light (for example, see JP2017-217215A). In this case, since the three-dimensional shape of the subject is measured according to the state of deformation of the grid pattern in a case where the subject is irradiated with the grid-pattern measurement light LPL, it is required to accurately detect the grid pattern. For this reason, the shape of the grid-pattern measurement light LPL is not a perfect grid shape and is set to a wave shape or the like, that is, is slightly deformed from a grid shape to improve the detection accuracy of the grid pattern. Further, the grid pattern is provided with S codes indicating that the end points of the left and right horizontal line segments are continuous. In a case where the grid pattern is detected, not only the pattern but also the S codes are detected to improve the detection accuracy of the pattern. The grid pattern may be a pattern in which a plurality of spots are arranged in a grid shape in the vertical and horizontal directions in addition to a pattern in which vertical lines and horizontal lines are regularly arranged.

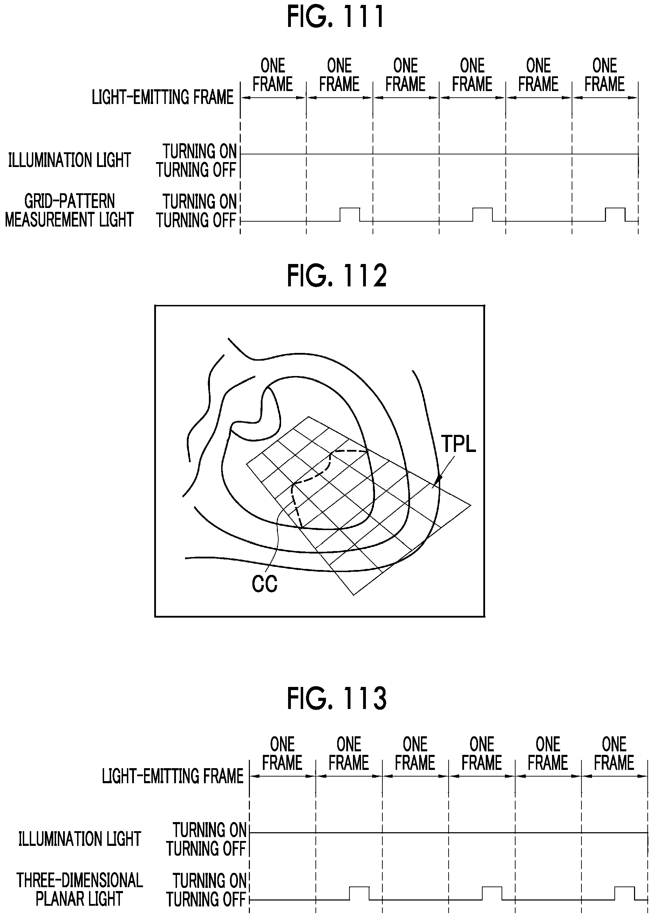

In a case where the grid-pattern measurement light LPL is used as the measurement light, in the length measurement mode, the subject may be constantly irradiated with the illumination light and the grid-pattern measurement light LPL or the subject may be constantly irradiated with the illumination light and the grid-pattern measurement light LPL may be repeatedly turned on and turned off (dimmed) every frame (or every few frames) as shown in Fig. 111 so that the subject is intermittently irradiated with the grid-pattern measurement light LPL. In this case, the three-dimensional shape is measured on the basis of the grid-pattern measurement light LPL in a frame where the grid-pattern measurement light LPL is turned on. Further, it is preferable that the measurement result of the three-dimensional shape is superimposed and displayed on an image obtained in a frame where the subject is irradiated with only the illumination light.

Three-dimensional planar light TPL, which is represented in a subject image by mesh lines as shown in Fig. 112, may be used as the measurement light (for example, see JP2017-508529A). In this case, a user moves the distal end part 12d so that the three-dimensional planar light TPL is aimed at an object to be measured. Then, in a case where the three-dimensional planar light TPL intersects with the object to be measured, a distance from an intersection curve CC between the three-dimensional planar light TPL and the subject is calculated by processing based on a manual operation of the user interface or the like, or automatic processing.

In a case where the three-dimensional planar light TPL is used as the measurement light, in the length measurement mode, the subject may be constantly irradiated with the illumination light and the three-dimensional planar light TPL or the subject may be constantly irradiated with the illumination light and the three-dimensional planar light TPL may be repeatedly turned on and turned off (or dimmed) every frame (or every few frames) as shown in Fig. 113 so that the subject is intermittently irradiated with the three-dimensional planar light TPL.

In the embodiment, the hardware structures of processing units, which perform various types of processing, such as the reception unit 38, the signal processing unit 39, the display controller 40, the system controller 41, the static image storage unit 42, the data transmission/reception unit 43, the data transmission/reception unit 44, the signal processing unit 45, and the display controller 46 (including various controllers or processing units provided in these controllers and the like (for example, the length measurement mode controller 50, the first signal processing unit 59, and the like)), are various processors to be described below. Various processors include: a central processing unit (CPU) that is a general-purpose processor functioning as various processing units by executing software (program); a programmable logic device (PLD) that is a processor of which the circuit configuration can be changed after manufacture, such as a field programmable gate array (FPGA); a dedicated electrical circuit that is a processor having circuit configuration designed exclusively to perform various types of processing; and the like.

One processing unit may be formed of one of these various processors, or may be formed of a combination of two or more same kind or different kinds of processors (for example, a plurality of FPGAs, or a combination of a CPU and an FPGA). Further, a plurality of processing units may be formed of one processor. As an example where a plurality of processing units are formed of one processor, first, there is an aspect where one processor is formed of a combination of one or more CPUs and software as typified by a computer, such as a client or a server, and functions as a plurality of processing units. Second, there is an aspect where a processor fulfilling the functions of the entire system, which includes a plurality of processing units, by one integrated circuit (IC) chip as typified by System On Chip (SoC) or the like is used. In this way, various processing units are formed using one or more of the above-mentioned various processors as hardware structures.

In addition, the hardware structures of these various processors are more specifically electrical circuitry where circuit elements, such as semiconductor elements, are combined. Further, the hardware structure of the storage unit is a storage device, such as a hard disc drive (HDD) or a solid state drive (SSD).

Explanation of References

10: endoscope system
12: endoscope
12a: insertion part
12b: operation part
12c: bendable part
12d: distal end part
12f: observation mode selector switch
12g: static image-acquisition instruction switch
12h: zoom operation part
13: light source device
14: processor device
15: display
16: user interface
17: augmented processor device
18: augmented display
18a: accessory information display region
18b: observation image display region
19: balloon
19a: distal end portion
19b: proximal end portion
19c: intermediate bulging portion
20a, 20b: ring
21: image pickup optical system
21a: objective lens

21b: zoom lens
21c: distal end surface
22: illumination optical system
22a: illumination lens
22b: distal end surfaces
23: measurement light-emitting unit
23a: light source
23b: DOE
23c: prism
24: opening
25: air/water supply nozzle
25a: jetting tube portion
25b: jetting port
26: intestinal canal
27: distal end cap
27a, 27b, 27c, 27d: through-hole
28: distal end surface
28a, 28b: flat surface
30: light source unit
31: light source processor
32: image pickup element
33: image pickup controller
34: CDS/AGC circuit
35: A/D converter
36: communication interface (I/F)
37: communication interface (I/F)
38: reception unit
39: signal processing unit
40: display controller
41: system controller
42: static image storage unit
43: data transmission/reception unit
44: data transmission/reception unit
45: signal processing unit
46: display controller
47: housing portion
48: transparent lid
49: prism
50: length measurement mode controller
53: brightness information calculation unit
54: illumination light-amount-level setting unit
55: first light emission control table
56: second light emission control table
57: rising line
58: diagonal line
59: first signal processing unit
60: second signal processing unit
61: irradiation position detector
62: scale table
64: table updating unit
66: representative point data table
67: intersection line
68: gradation
69: position specification unit
70: measurement information processing unit
71: measurement information
72: position specification unit
73: image processing unit

74: noise component removal unit

75: color information conversion unit

76: binarization processing unit

77: mask image generation unit

78: removal unit

79: color information of measurement light

80: color information of noise components

81: the noise component region

82: image selection unit

84: first signal processing unit

85: second signal processing unit

86: mask processing unit

87: binarization processing unit

88: noise component removal unit

89: irradiation position detector

90: irradiation region recognition unit

91: learning model

92: position specification unit

93: image processing unit

94: distance calculation unit

95: image selection unit

97: offset setting unit

98: offset distance calculation unit

99: offset virtual scale generation unit

100: polyp

100a: apex portion

100b: flat portion

101: extension surface

101X: solid line

102, 103: parallel plane

102X: dotted line

104: image processing unit

105: virtual scale setting unit

106: virtual scale switching reception unit

107: length measurement image creation unit

108: polyp

109: captured image

110: shadow

111, 112: virtual scale

111a: gradation label

113, 114: captured image

115: virtual scale

116: gradation

116a: gradation label

116b: gradation label

118: virtual scale

118a: gradation label

119: virtual scale

119a: gradation label

120: reference scale setting unit

121: measured value scale generation unit

121a: reference scale table

122: length measurement image generation unit

123: polyp

124: captured image

125: region-of-interest extraction unit

126: measurement portion determination unit

127: measurement content reception unit

128: measured value calculation unit

129: region of interest

130: horizontal edge position

131: reference scale

132: measured value scale

133: length measurement image

135: lesion recognition unit

136: diagnostic information acquisition unit

137: learning unit

138: diagnostic information management device

140: length measurement-compatible endoscope-availability determination unit

141: measurement light-ON/OFF switching unit

142: length measurement image-display setting-ON/OFF switching unit

143: length measurement function-operation state display-ON/OFF switching unit

144: virtual scale-display switching controller

146: scale display icon

147: virtual scale

147a, 147b, 147c: virtual scale

148: scale non-display icon

149: unswitched image display setting-storage unit

200: calibration apparatus

201: calibration display

202: moving mechanism

204: calibration display controller

206: calibration image acquisition unit

208: calibration unit

210: calibration image processing device

300: inspection system

302: test chart

304: moving mechanism unit

305: chart body

306: inspection region portion

306a, 306b, 306c: inspection region

308: inspection reference position

Aa, Bb: the number of pixels

Ax: optical axis

BLC: balloon control device

BF: blue color filter

CL1: first feature lines

CL2: second feature lines

CC: intersection curve

CR1: white central region

D1: first direction

D2: second direction

D3: third direction

D5: distance

D6: offset distance

DS1: diffraction spots

DT: interval

EP: measurement points

G1, G2, G3: gap

GF: green color filter

HT1, HT2: height

LG: light guide

Ls, Lt: line

Lm: measurement light

Lx1, Lx2: the number of pixels in the X direction

Ly1, Lx2: the number of the pixels in the Y direction

LPL: grid-pattern measurement light

M: circular virtual scale

M1, M2, M3: virtual scale

M11, M12, M13, M14, M15: virtual scale

M21, M22, M23: concentric circle

M41, M42, M43: virtual scale

M4A, M4B, M4C, M5A, M5B, M5C: virtual scale

M51, M52, M53: virtual scale

M6A, M6B, M6C: distorted concentric circular virtual scales

MN, MF: cruciform virtual scale

MpN, Mp, MpF: circular virtual scale

MT: movement locus

Mx: gradations

Mxm: virtual scale

N1: first noise component

N2: second noise component

P: polyp

P1, P2, P3: position

Px: far end

Py: intermediate vicinity

Pz: near end

RP, PRx: red image

PRy: binarized red image

GP, PGx: green image

PGy: binarized green image

BP, PBx: blue image

PBy: binarized blue image

PD1: first difference image

Qx, Qy, Qz: arrow

QN: distorted grid region

RP, RP*: representative points

RF: red color filter

SCC: specific intersection curve

SP: spot

SP1, SP2, SP3, SP4, SP5: spot

SPk1: first spot

SPk2: second spot

SQ: square grid region

SR1: peripheral region

tm, tm1, tm2, tm3, tm4, tm5: tumor

TPL: three-dimensional planar light

W11, W12, W13, W14, W15: width

W21, W22, W23: width

Wx: illumination position-movable range

WMB: wavelength range of measurement light

ZPL: stripe-pattern light

**Claims**

1. An endoscope system (10) comprising:

   an endoscope (12);
   a processor device (14) that includes an image control processor,
   wherein the image control processor is configured to:

   in a case where the endoscope (12) is connected to the processor device (14), read out a scope ID from the
   endoscope (12), and based on the scope ID, determine whether or not the endoscope (12) is a length measurement-compatible endoscope (12);

enable switching of a mode to a length measurement mode in a case where the endoscope (12) is the length measurement-compatible endoscope (12), in a state where the switching of a mode to the length measurement mode is enabled, perform switching of ON or OFF of a measurement light (Lm);

with reference to a representative point data table (66), create a virtual scale image corresponding to the model and/or the serial number of the endoscope (12), and

superimpose the virtual scale image on an image captured by the endoscope (12) and display the superimposed image on a display (18).

2. The endoscope system (10) according to claim 1,

wherein in a case where the endoscope (12) is the length measurement-compatible endoscope (12), the endoscope (12) is capable of emitting measurement light (Lm) and causing the display (18) to display a length measurement image displaying a virtual scale (147) based on the measurement light (Lm), and

the image control processor is configured to, in a state where the switching of a mode to the length measurement mode is enabled, perform switching of ON or OFF of length measurement image-display settings (142) related to the length measurement image, switching of ON or OFF of length measurement function-operation state display (143) showing that the virtual scale (147) is being displayed on the display (18), or switching of ON or OFF of display of the virtual scale (147) or display aspect change of the virtual scale (147) by an operation for switching a mode to the length measurement mode.

3. The endoscope system (10) according to claim 2,
wherein the image control processor is configured to switch the measurement light (Lm) to ON, switch the length measurement image-display settings to ON, switch the length measurement function-operation state display to ON, and switch the display of the virtual scale (147) to ON by the operation for switching a mode to the length measurement mode.

4. The endoscope system (10) according to claim 3,
wherein the image control processor is configured to, in a case where conditions in switching a mode are not satisfied in the operation for switching a mode to the length measurement mode, prohibit the switching of the measurement light (Lm) to ON, prohibits the switching of the length measurement image-display settings to ON, prohibit the switching of the length measurement function-operation state display to ON, and prohibit the switching of the display of the virtual scale to ON.

5. The endoscope system (10) according to claim 4,
wherein length measurement function-operation state-unavailability display showing that the virtual scale (147) is not being displayed is switched to ON instead of prohibiting the switching of the length measurement function-operation state display to ON.

6. The endoscope system (10) according to claim 3,
wherein the image control processor is configured to, in a case where the length measurement image-display settings are switched to ON, store image display settings before a mode is switched to the length measurement mode.

7. The endoscope system (10) according to claim 3,
wherein display aspect change of the virtual scale (147) is performed according to a selection from a plurality of scale patterns.

8. The endoscope system according to claim 2,
wherein the image control processor is configured to switch the measurement light (Lm) to OFF, switch the length measurement image-display settings to OFF, switch the length measurement function-operation state display to OFF, and switch the display of the virtual scale to OFF by an operation for switching the length measurement mode to another mode.

9. The endoscope system (10) according to claim 2 or 8,
wherein the image control processor is configured to, in a case where the length measurement image-display settings are switched to OFF, switch image display settings to image display settings stored before a mode is switched to the length measurement mode.

**Patentansprüche**

1. Endoskopsystem (10), umfassend:

ein Endoskop (12);
eine Prozessorvorrichtung (14), die einen Bildsteuerprozessor enthält,
wobei der Bildsteuerprozessor so konfiguriert ist, dass er:

in einem Fall, in dem das Endoskop (12) mit der Prozessorvorrichtung (14) verbunden ist, eine Endoskop-ID aus dem Endoskop (12) ausliest und auf der Grundlage der Endoskop-ID bestimmt, ob das Endoskop (12) ein längenmesskompatibles Endoskop (12) ist oder nicht;
Umschalten eines Modus auf einen Längenmessmodus in einem Fall, in dem das Endoskop (12) das längenmesskompatible Endoskop (12) ist, ermöglicht, und in einem Zustand, in dem das Umschalten eines Modus auf den Längenmessmodus aktiviert ist, Umschalten von EIN oder AUS eines Messlichts (Lm) durchführt;
unter Bezugnahme auf eine Datentabelle (66) repräsentativer Punkte ein virtuelles Maßstabsbild, das dem Modell und/oder der Seriennummer des Endoskops (12) entspricht, erstellt, und
das virtuelle Maßstabbild auf ein Bild, das durch das Endoskop (12) aufgenommen wurde, überlagert und das überlagerte Bild auf einer Anzeige (18) anzeigt.

2. Endoskopsystem (10) nach Anspruch 1,

wobei in einem Fall, in dem das Endoskop (12) das längenmesskompatible Endoskop (12) ist, das Endoskop (12) in der Lage ist, Messlicht (Lm) zu emittieren und die Anzeige (18) zu veranlassen, ein Längenmessbild, das einen virtuellen Maßstab (147) auf der Grundlage des Messlichts (Lm) anzeigt, anzuzeigen, und
der Bildsteuerungsprozessor so konfiguriert ist, dass er in einem Zustand, in dem das Umschalten eines Modus auf den Längenmessmodus aktiviert ist, Umschalten von EIN oder AUS von Längenmessbild-Anzeigeeinstellungen (142) in Bezug auf das Längenmessbild, Umschalten von EIN oder AUS von Längenmessfunktions-Betriebszustandsanzeige (143), die zeigt, dass der virtuelle Maßstab (147) auf der Anzeige (18) angezeigt wird, oder Umschalten von EIN oder AUS von Anzeige des virtuellen Maßstabs (147) oder Anzeigeaspektänderung des virtuellen Maßstabs (147) durch eine Bedienung zum Umschalten eines Modus auf den Längenmessmodus durchführt.

3. Endoskopsystem (10) nach Anspruch 2,
wobei der Bildsteuerungsprozessor so konfiguriert ist, dass er das Messlicht (Lm) auf EIN schaltet, die Längenmessbild-Anzeigeeinstellungen auf EIN schaltet, die Längenmessfunktions-Betriebszustandsanzeige auf EIN schaltet, und die Anzeige des virtuellen Maßstabs (147) auf EIN durch die Bedienung zum Umschalten eines Modus auf den Längenmessungsmodus schaltet.

4. Endoskopsystem (10) nach Anspruch 3,
wobei der Bildsteuerungsprozessor so konfiguriert ist, dass er in einem Fall, in dem Bedingungen bei Umschalten eines Modus bei der Bedienung zum Umschalten eines Modus auf den Längenmessmodus nicht erfüllt sind, das Umschalten des Messlichts (Lm) auf EIN verbietet, das Umschalten der Längenmessbild-Anzeigeeinstellungen auf EIN verbietet, das Umschalten der Längenmessfunktions-Betriebszustandsanzeige auf EIN verbietet und das Umschalten der Anzeige des virtuellen Maßstabs auf EIN verbietet.

5. Endoskopsystem (10) nach Anspruch 4,
wobei anstelle des Verbietens des Umschaltens der Längenmessfunktions-Betriebszustandsanzeige auf EIN die Längenmessfunktions-Betriebsunfähigkeitsanzeige, die anzeigt, dass der virtuelle Maßstab (147) nicht angezeigt wird, auf EIN umgeschaltet wird.

6. Endoskopsystem (10) nach Anspruch 3,
wobei der Bildsteuerungsprozessor so konfiguriert ist, dass er in einem Fall, in dem die Längenmessbild-Anzeigeneinstellungen auf EIN umgeschaltet werden, Bildanzeigeeinstellungen speichert, bevor ein Modus auf den Längenmessmodus umgeschaltet wird.

7. Endoskopsystem (10) nach Anspruch 3,
wobei Anzeigeaspektänderung des virtuellen Maßstabs (147) gemäß einer Auswahl aus mehreren Maßstabsmustern durchgeführt wird.

**8.** Endoskopsystem nach Anspruch 2,
wobei der Bildsteuerungsprozessor so konfiguriert ist, dass er das Messlicht (Lm) auf AUS schaltet, die Längenmessbild-Anzeigeeinstellungen auf AUS schaltet, die Längenmessfunktions-Betriebszustandsanzeige auf AUS schaltet und die Anzeige des virtuellen Maßstabs auf AUS durch eine Bedienung zum Umschalten des Längenmessmodus auf einen anderen Modus schaltet.

**9.** Endoskopsystem (10) nach Anspruch 2 oder 8,
wobei der Bildsteuerungsprozessor so konfiguriert ist, dass er in einem Fall, in dem die Längenmessbild-Anzeigeneinstellungen auf AUS umgeschaltet werden, Bildanzeigeneinstellungen auf Bildanzeigeneinstellungen, die gespeichert sind, bevor ein Modus auf den Längenmessmodus umgeschaltet wird, umschaltet.

**Revendications**

**1.** Système d'endoscope (10) comprenant :

un endoscope (12) ;
un dispositif processeur (14) qui inclut un processeur de contrôle d'images,
dans lequel le processeur de contrôle d'images est configuré pour :

dans un cas où l'endoscope (12) est connecté au dispositif processeur (14), lire un identifiant de scope de l'endoscope (12), et sur la base de l'identifiant de scope, déterminer si l'endoscope (12) est ou non un endoscope (12) compatible avec la mesure de longueur ;
permettre la commutation d'un mode vers un mode de mesure de longueur dans un cas où l'endoscope (12) est l'endoscope (12) compatible avec la mesure de longueur, dans un état où la commutation d'un mode vers le mode de mesure de longueur est activée, effectuer la commutation en ON ou OFF d'une lumière de mesure (Lm) ;
en référence à une table de données de points représentatifs (66), créer une image d'échelle virtuelle correspondant au modèle et/ou au numéro de série de l'endoscope (12), et
superposer l'image d'échelle virtuelle sur une image capturée par l'endoscope (12) et afficher l'image superposée sur un affichage (18).

**2.** Système d'endoscope (10) selon la revendication 1,

dans lequel dans un cas où l'endoscope (12) est l'endoscope (12) compatible avec la mesure de longueur, l'endoscope (12) est capable d'émettre une lumière de mesure (Lm) et d'amener l'affichage (18) à afficher une image de mesure de longueur affichant une échelle virtuelle (147) sur la base de la lumière de mesure (Lm), et le processeur de contrôle d'images est configuré pour, dans un état où la commutation d'un mode vers le mode de mesure de longueur est activée, effectuer la commutation en ON ou OFF des paramètres d'affichage d'image de mesure de longueur (142) liés à l'image de mesure de longueur, la commutation en ON ou OFF de l'affichage d'état d'opération de fonction de mesure de longueur (143) montrant que l'échelle virtuelle (147) est affichée sur l'affichage (18), ou la commutation en ON ou OFF de l'affichage de l'échelle virtuelle (147) ou le changement d'aspect d'affichage de l'échelle virtuelle (147) par une opération pour commuter un mode vers le mode de mesure de longueur.

**3.** Système d'endoscope (10) selon la revendication 2,
dans lequel le processeur de contrôle d'images est configuré pour commuter la lumière de mesure (Lm) en ON, commuter les paramètres d'affichage d'image de mesure de longueur en ON, commuter l'affichage d'état d'opération de fonction de mesure de longueur en ON, et commuter l'affichage de l'échelle virtuelle (147) en ON par l'opération pour commuter un mode vers le mode de mesure de longueur.

**4.** Système d'endoscope (10) selon la revendication 3,
dans lequel le processeur de contrôle d'images est configuré pour, dans un cas où des conditions dans la commutation d'un mode ne sont pas satisfaites dans l'opération pour commuter un mode vers le mode de mesure de longueur, interdire la commutation de la lumière de mesure (Lm) en ON, interdire la commutation des paramètres d'affichage d'image de mesure de longueur en ON, interdire la commutation de l'affichage d'état d'opération de fonction de mesure de longueur en ON, et interdire la commutation de l'affichage de l'échelle virtuelle en ON.

5. Système d'endoscope (10) selon la revendication 4,
dans lequel l'affichage d'indisponibilité d'état d'opération de fonction de mesure de longueur montrant que l'échelle virtuelle (147) n'est pas affichée est commuté en ON au lieu d'interdire la commutation de l'affichage d'état d'opération de fonction de mesure de longueur en ON.

6. Système d'endoscope (10) selon la revendication 3,
dans lequel le processeur de contrôle d'images est configuré pour, dans un cas où les paramètres d'affichage d'image de mesure de longueur sont commutés en ON, stocker les paramètres d'affichage d'image avant qu'un mode ne soit commuté vers le mode de mesure de longueur.

7. Système d'endoscope (10) selon la revendication 3,
dans lequel le changement d'aspect d'affichage de l'échelle virtuelle (147) est effectué en fonction d'une sélection parmi une pluralité de motifs d'échelle.

8. Système d'endoscope selon la revendication 2,
dans lequel le processeur de contrôle d'images est configuré pour commuter la lumière de mesure (Lm) en OFF, commuter les paramètres d'affichage d'image de mesure de longueur en OFF, commuter l'affichage d'état d'opération de fonction de mesure de longueur en OFF, et commuter l'affichage de l'échelle virtuelle en OFF par une opération pour commuter le mode de mesure de longueur vers un autre mode.

9. Système d'endoscope (10) selon la revendication 2 ou la revendication 8,
dans lequel le processeur de contrôle d'images est configuré pour, dans un cas où les paramètres d'affichage d'image de mesure de longueur sont commutés en OFF, commuter les paramètres d'affichage d'image vers des paramètres d'affichage d'image stockés avant qu'un mode ne soit commuté vers le mode de mesure de longueur.

# FIG. 1

| LIGHT SOURCE DEVICE |
|---|

| PROCESSOR DEVICE |
|---|

| AUGMENTED PROCESSOR DEVICE |
|---|

| DISPLAY |
|---|

| AUGMENTED DISPLAY |
|---|

| USER INTERFACE |
|---|

FIG. 2

# FIG. 3

BALLOON CONTROL
DEVICE

EP 4 209 821 B1

# FIG. 4

EP 4 209 821 B1

# FIG. 5

# FIG. 6

# FIG. 7

**FIG. 8**

# FIG. 9

(A)

(B)

# FIG. 10

# FIG. 11

# FIG. 12

# FIG. 13

# FIG. 14

SYSTEM CONTROLLER

41 ―

| LENGTH MEASUREMENT-COMPATIBLE ENDOSCOPE-AVAILABILITY DETERMINATION UNIT | ―140 |

| MEASUREMENT LIGHT-ON/OFF SWITCHING UNIT | ―141 |

| LENGTH MEASUREMENT IMAGE-DISPLAY SETTING-ON/OFF SWITCHING UNIT | ―142 |

| LENGTH MEASUREMENT FUNCTION-OPERATION STATE DISPLAY-ON/OFF SWITCHING UNIT | ―143 |

| VIRTUAL SCALE-DISPLAY SWITCHING CONTROLLER | ―144 |

| UNSWITCHED IMAGE DISPLAY SETTING-STORAGE UNIT | ―149 |

# FIG. 15

# FIG. 16

41 ─┐

SYSTEM CONTROLLER

LENGTH MEASUREMENT MODE
CONTROLLER ─50

# FIG. 17

SET MODE

SPECIAL OBSERVATION MODE

OPERATION FOR
SWITCHING MODE
TO LENGTH
MEASUREMENT
MODE

SPECIAL OBSERVATION MODE

## FIG. 18

18

SWITCHING OF MODE TO LENGTH
MEASUREMENT MODE IS PROHIBITED

## FIG. 19

SET MODE

SPECIAL OBSERVATION MODE

OPERATION FOR
SWITCHING MODE
TO LENGTH
MEASUREMENT
MODE

LENGTH MEASUREMENT MODE

## FIG. 20

SET MODE

LENGTH MEASUREMENT MODE

SETTING CHANGE
OPERATION FOR
TURNING ON
ZOOM FUNCTION

LENGTH MEASUREMENT MODE

DISABLEMENT

## FIG. 21

18

SETTING CHANGE OPERATION FOR
TURNING ON ZOOM FUNCTION IS DISABLED

## FIG. 22

```
┌ ─ ─ ─ ─ ─ ─ ─ ─ ─ ─ ─ ─ ─ ─ ─ ─ ─ ─ ┐
             SET MODE
  ┌─────────────────────────────────┐
│ │    LENGTH MEASUREMENT MODE      │ │
  └─────────────────────────────────┘
│                 │                    │
                  │
│                 │                    │
                  │         ┌──────────────────┐
│                 │         │ SETTING CHANGE   │
                  │◄────────│ OPERATION FOR    │
│                 │         │ TURNING ON       │
                  │         │ ZOOM FUNCTION    │
│                 │         └──────────────────┘
                  ▼
│ ┌─────────────────────────────────┐ │
  │    NORMAL OBSERVATION MODE       │
│ └─────────────────────────────────┘ │
└ ─ ─ ─ ─ ─ ─ ─ ─ ─ ─ ─ ─ ─ ─ ─ ─ ─ ─ ┘
```

## FIG. 23

18

```
┌──────────────────────────────────────────────────────┐
│                                                        │
│                                                        │
│  ┌──────────────────────────────────────────────────┐ │
│  │  LENGTH MEASUREMENT MODE IS CANCELED AND          │ │
│  │  MODE IS SWITCHED TO NORMAL OBSERVATION MODE      │ │
│  └──────────────────────────────────────────────────┘ │
│                                                        │
│                                                        │
└──────────────────────────────────────────────────────┘
```

# FIG. 24

41

| SYSTEM CONTROLLER |
|---|
| 53 BRIGHTNESS INFORMATION CALCULATION UNIT |
| 54 ILLUMINATION LIGHT-AMOUNT-LEVEL SETTING UNIT |
| 55 FIRST LIGHT EMISSION CONTROL TABLE |
| 56 SECOND LIGHT EMISSION CONTROL TABLE |

# FIG. 25

| COORDINATE INFORMATION OF SPOT SP | LIGHT AMOUNT LEVEL OF MEASUREMENT LIGHT |
|---|---|
| COORDINATE AREA 1 | Level1 |
| COORDINATE AREA 2 | Level2 |
| COORDINATE AREA 3 | Level3 |
| COORDINATE AREA 4 | Level4 |
| COORDINATE AREA 5 | Level5 |

55

# FIG. 26

| |
|---|
| COORDINATE AREA 5 |
| COORDINATE AREA 4 |
| COORDINATE AREA 3 |
| COORDINATE AREA 2 |
| COORDINATE AREA 1 |

## FIG. 27

| COORDINATE INFORMATION OF SPOT SP | LIGHT AMOUNT LEVEL OF ILLUMINATION LIGHT | LIGHT AMOUNT LEVEL OF MEASUREMENT LIGHT |
|---|---|---|
| COORDINATE AREA 1 | Level1 | Level1 |
| | Level2 | Level2 |
| | Level3 | Level3 |
| | Level4 | Level3 |
| | Level5 | Level3 |
| COORDINATE AREA 5 | Level1 | Level3 |
| | Level2 | Level3 |
| | Level3 | Level3 |
| | Level4 | Level4 |
| | Level5 | Level5 |

56

## FIG. 28

| LIGHT-EMITTING FRAME | FLx | FLy | FLx | FLy | FLx | FLy |
|---|---|---|---|---|---|---|

ILLUMINATION LIGHT  on / off

MEASUREMENT LIGHT  on / off

# FIG. 29

| | NORMAL OBSERVATION MODE | LENGTH MEASUREMENT MODE | | | | | | |
|---|---|---|---|---|---|---|---|---|

CCD (FRAME PERIOD) GLOBAL SHUTTER: 57, 57, 57, 57, 57, 57, 57

ONE FRAME

ILLUMINATION LIGHT: on / off

MEASUREMENT LIGHT: on / off

| OUTPUT OF IMAGE PICKUP ELEMENT | N | N+Lm | N | N+Lm | N | N+Lm | N |
|---|---|---|---|---|---|---|---|
| OUTPUT OF DISPLAY IMAGE | N | S | S | S | S | S | S |

| TIMING | T1 | T2 | T3 | T4 | T5 | T6 | T7 | T8 |
|---|---|---|---|---|---|---|---|---|

# FIG. 30

| | NORMAL OBSERVATION MODE | LENGTH MEASUREMENT MODE | | | | | | |
|---|---|---|---|---|---|---|---|---|

Ls  ONE FRAME

CMOS (FRAME PERIOD) ROLLING SHUTTER

58 Lt, 58, 58, 58, 58, 58, 58, 58

ILLUMINATION LIGHT: on / off

MEASUREMENT LIGHT: on / off

| OUTPUT OF IMAGE PICKUP ELEMENT | N | N+Lm | N+Lm | N | N+Lm | N+Lm | N |
|---|---|---|---|---|---|---|---|
| OUTPUT OF DISPLAY IMAGE | N | S | S | S | S | S | S |

| TIMING | T1 | T2 | T3 | T4 | T5 | T6 | T7 | T8 |
|---|---|---|---|---|---|---|---|---|

# FIG. 31

45

**SIGNAL PROCESSING UNIT**

59

**FIRST SIGNAL PROCESSING UNIT**

| IRRADIATION POSITION DETECTOR | — 61 |

60

**SECOND SIGNAL PROCESSING UNIT**

| SCALE TABLE | — 62 |
| TABLE UPDATING UNIT | — 64 |
| REPRESENTATIVE POINT DATA TABLE | — 66 |

| DISPLAY CONTROLLER | — 46 |

# FIG. 32

M1
tm1
SP1

## FIG. 33

## FIG. 34

## FIG. 35

| CRUCIFORM SHAPE | CRUCIFORM SHAPE WITH GRADATIONS | DISTORTED CRUCIFORM SHAPE | CIRCULAR-AND-CRUCIFORM SHAPE | SHAPE OF MEASUREMENT POINT GROUP |
|---|---|---|---|---|
|  | Mx ... Mx |  |  | EP ... EP |

FIG. 36

FIG. 37

## FIG. 38

## FIG. 39

## FIG. 40

INTERPOLATION PROCESSING

## FIG. 41

UPPER SIDE

LOWER SIDE

# FIG. 42

# FIG. 43

# FIG. 44

# FIG. 45

## FIG. 46

## FIG. 47

# FIG. 48

45

## SIGNAL PROCESSING UNIT

72

### POSITION SPECIFICATION UNIT

NOISE COMPONENT REMOVAL UNIT — 74

COLOR INFORMATION CONVERSION UNIT — 75

BINARIZATION PROCESSING UNIT — 76

MASK IMAGE GENERATION UNIT — 77

REMOVAL UNIT — 78

73

### IMAGE PROCESSING UNIT

IMAGE SELECTION UNIT — 82

SCALE TABLE — 62

# FIG. 49

```
┌─────────────────┐              ┌─────────────────┐
│  FIRST CAPTURED │              │     SECOND      │
│      IMAGE      │              │ CAPTURED IMAGE  │
└─────────────────┘              └─────────────────┘
         │                                │
         ▼                                ▼
┌─────────────────┐              ┌─────────────────┐
│  HSV-CONVERTED  │              │  HSV-CONVERTED  │
│  FIRST CAPTURED │              │     SECOND      │
│      IMAGE      │              │ CAPTURED IMAGE  │
└─────────────────┘              └─────────────────┘
         │                                │
         ▼                                ▼
┌─────────────────┐              ┌─────────────────┐
│ BINARIZED FIRST │              │    BINARIZED    │
│ CAPTURED IMAGE  │              │     SECOND      │
│                 │              │ CAPTURED IMAGE  │
└─────────────────┘              └─────────────────┘
         │                                │
         └────────────┬───────────────────┘
                      ▼
            ┌─────────────────┐
            │   MASK IMAGE    │
            └─────────────────┘
                      │
                      ▼
            ┌─────────────────┐
            │  NOISE-REMOVED  │
            │ FIRST CAPTURED  │
            │      IMAGE      │
            └─────────────────┘
```

# FIG. 50

BINARIZED FIRST COLOR INFORMATION IMAGE

# FIG. 51

BINARIZED SECOND COLOR INFORMATION IMAGE

# FIG. 52

80 81

BINARIZED SECOND COLOR INFORMATION IMAGE

# FIG. 53

80 81

79

BINARIZED FIRST COLOR INFORMATION IMAGE

# FIG. 54

45

## SIGNAL PROCESSING UNIT

84

### FIRST SIGNAL PROCESSING UNIT

| | |
|---|---|
| MASK PROCESSING UNIT | 86 |
| BINARIZATION PROCESSING UNIT | 87 |
| NOISE COMPONENT REMOVAL UNIT | 88 |
| IRRADIATION POSITION DETECTOR | 89 |

85

### SECOND SIGNAL PROCESSING UNIT

# FIG. 55

```
┌──────────────┐
│   CAPTURED   │
│    IMAGE     │
└──────┬───────┘
       │
       ▼
┌──────────────┐
│     MASK     │
│  PROCESSING  │
└──────┬───────┘
       │
   ┌───┼──────────────────────┬─────────────────────────┐
   │                          │                         │
   ▼                          ▼                         ▼
┌──────────┐          ┌──────────────┐          ┌──────────────┐
│RED IMAGE │          │ GREEN IMAGE  │          │  BLUE IMAGE  │
└────┬─────┘          └──────┬───────┘          └──────┬───────┘
     │                       │                         │
     ▼                       ▼                         ▼
┌──────────┐          ┌──────────────┐          ┌──────────────┐
│  FIRST   │          │   SECOND     │          │    THIRD     │
│BINARIZA- │          │ BINARIZATION │          │ BINARIZATION │
│  TION    │          │ PROCESSING   │          │ PROCESSING   │
│PROCESSING│          └──────┬───────┘          └──────┬───────┘
└────┬─────┘                 │                         │
     ▼                       ▼                         ▼
┌──────────┐          ┌──────────────┐          ┌──────────────┐
│ BINARIZED│          │  BINARIZED   │          │  BINARIZED   │
│RED IMAGE │          │ GREEN IMAGE  │          │  BLUE IMAGE  │
└────┬─────┘          └──────┬───────┘          └──────┬───────┘
     │                       │                         │
     │◄──────────────────────┘                         │
     ▼                                                 │
┌──────────┐                                           │
│  FIRST   │                                           │
│DIFFERENCE│                                           │
│PROCESSING│                                           │
└────┬─────┘                                           │
     ▼                                                 │
┌──────────┐                                           │
│  FIRST   │                                           │
│DIFFERENCE│                                           │
│  IMAGE   │                                           │
└────┬─────┘                                           │
     │◄────────────────────────────────────────────────┘
     ▼
┌──────────┐
│  SECOND  │
│DIFFERENCE│
│PROCESSING│
└────┬─────┘
     ▼
┌──────────┐
│  SECOND  │
│DIFFERENCE│
│  IMAGE   │
└──────────┘
```

# FIG. 56

# FIG. 57

PBx

Wx

THIRD BINARIZATION
PROCESSING

PD1

Wx

SP

N2

PBy

Wx

SP

N2

SECOND DIFFERENCE
PROCESSING

PD2

Wx

SP

# FIG. 58

45

SIGNAL PROCESSING UNIT

IRRADIATION REGION
RECOGNITION UNIT — 90

SECOND SIGNAL
PROCESSING UNIT — 60

# FIG. 59

SP

CR1

SR1

## FIG. 60

## FIG. 61

## FIG. 62

90

**IRRADIATION REGION RECOGNITION UNIT**

91

CAPTURED IMAGE →INPUT→ LEARNING MODEL →OUTPUT→ SPOT SP (MEASUREMENT LIGHT-IRRADIATION REGION)

↑ MACHINE LEARNING

TEACHER DATA

## FIG. 63

### PATTERN OF SPOT SP DEFORMED FROM CIRCULAR SHAPE (SPECIFIC SHAPE)

SP

SP

(A)                    (B)

# FIG. 64

┌─────────────────────────────────────────────┐ ─45
│ SIGNAL PROCESSING UNIT                        │
│ ┌───────────────────────────────────────┐ ─92│
│ │ POSITION SPECIFICATION UNIT           │    │
│ │ ┌─────────────────────────────────┐ ─94│  │
│ │ │ DISTANCE CALCULATION UNIT       │    │  │
│ │ └─────────────────────────────────┘    │  │
│ └───────────────────────────────────────┘    │
│ ┌───────────────────────────────────────┐ ─93│
│ │ IMAGE PROCESSING UNIT                 │    │
│ │ ┌─────────────────────────────────┐ ─95│  │
│ │ │ IMAGE SELECTION UNIT            │    │  │
│ │ └─────────────────────────────────┘    │  │
│ │ ┌─────────────────────────────────┐ ─62│  │
│ │ │ SCALE TABLE                     │    │  │
│ │ └─────────────────────────────────┘    │  │
│ │ ┌─────────────────────────────────┐ ─97│  │
│ │ │ OFFSET SETTING UNIT             │    │  │
│ │ └─────────────────────────────────┘    │  │
│ │ ┌─────────────────────────────────┐ ─98│  │
│ │ │ OFFSET DISTANCE CALCULATION UNIT│    │  │
│ │ └─────────────────────────────────┘    │  │
│ │ ┌─────────────────────────────────┐ ─99│  │
│ │ │ OFFSET VIRTUAL SCALE GENERATION │    │  │
│ │ │ UNIT                            │    │  │
│ │ └─────────────────────────────────┘    │  │
│ └───────────────────────────────────────┘    │
└─────────────────────────────────────────────┘

# FIG. 65

100a

100

101

100b

# FIG. 66

# FIG. 67

FIG. 68A

FIG. 68B

FIG. 68C

FIG. 68D

FIG. 68E

## FIG. 69

## FIG. 70

FIG. 71A

FIG. 71B

FIG. 71C

FIG. 72A

M51
tm
5
10
20
SP

FIG. 72B

M52
tm
5
10
SP

FIG. 72C

M53
5
SP

# FIG. 73

| SIGNAL PROCESSING UNIT | —45 |
|---|---|

POSITION SPECIFICATION UNIT —92

DISTANCE CALCULATION UNIT —94

IMAGE PROCESSING UNIT —104

IMAGE SELECTION UNIT —95

SCALE TABLE —62

VIRTUAL SCALE SETTING UNIT —105

VIRTUAL SCALE SWITCHING RECEPTION UNIT —106

LENGTH MEASUREMENT IMAGE CREATION UNIT —107

# FIG. 74

—109

108

SP

110

## FIG. 75

## FIG. 76

FIG. 77

FIG. 78A

108
117
110
10
117a
SP

FIG. 78B

118a
118
110
10
SP
108

FIG. 78C

119
108
110
SP
10
119a

## FIG. 79

| SIGNAL PROCESSING UNIT | 45 |
| POSITION SPECIFICATION UNIT | 92 |
| REFERENCE SCALE SETTING UNIT | 120 |
| MEASURED VALUE SCALE GENERATION UNIT | 121 |
| LENGTH MEASUREMENT IMAGE GENERATION UNIT | 122 |

## FIG. 80

| REFERENCE SCALE SETTING UNIT | 120 |
| REFERENCE SCALE TABLE | 121a |

## FIG. 81

## FIG. 82

| MEASURED VALUE SCALE GENERATION UNIT | 121 |
|---|---|
| REGION-OF-INTEREST EXTRACTION UNIT | 125 |
| MEASUREMENT PORTION DETERMINATION UNIT | 126 |
| MEASUREMENT CONTENT RECEPTION UNIT | 127 |
| MEASURED VALUE CALCULATION UNIT | 128 |

# FIG. 83

# FIG. 84

FIG. 85

# FIG. 86

# FIG. 87

# FIG. 88

FIG. 89A

FIG. 89B

FIG. 89C

FIG. 90A

FIG. 90B

FIG. 90C

# FIG. 91

STATIC IMAGE-ACQUISITION
INSTRUCTION

TIMING

ILLUMINATION
LIGHT

AUXILIARY
MEASUREMENT
LIGHT

CAPTURED
IMAGE

| FIRST CAPTURED IMAGE | FIRST CAPTURED IMAGE | SECOND CAPTURED IMAGE | FIRST CAPTURED IMAGE | FIRST CAPTURED IMAGE | FIRST CAPTURED IMAGE |
|---|---|---|---|---|---|

| SECOND CAPTURED IMAGE | FIRST CAPTURED IMAGE |
|---|---|

STORED IMAGE

ADD VIRTUAL SCALE

THIRD
CAPTURED
IMAGE

# FIG. 92

(A)
SECOND CAPTURED IMAGE
(FORM IMAGE)

(B)
FIRST CAPTURED IMAGE (LENGTH
MEASUREMENT SPOT IMAGE)

(C)
THIRD CAPTURED IMAGE
(LENGTH MEASUREMENT IMAGE)

42 — STATIC IMAGE
STORAGE UNIT

# FIG. 93

18

SECOND CAPTURED IMAGE
(FORM IMAGE)

THIRD CAPTURED IMAGE (LENGTH
MEASUREMENT IMAGE)

# FIG. 94

STATIC IMAGE-ACQUISITION
INSTRUCTION

SECOND TIMING
THIRD TIMING    FIRST TIMING

TIMING

ILLUMINATION    on
LIGHT    off

AUXILIARY
MEASUREMENT    on
LIGHT    off

CAPTURED
IMAGE

| FIRST CAPTURED IMAGE | FIRST CAPTURED IMAGE | SECOND CAPTURED IMAGE | FIRST CAPTURED IMAGE | FIRST CAPTURED IMAGE | FIRST CAPTURED IMAGE |
|---|---|---|---|---|---|

| FIRST CAPTURED IMAGE | SECOND CAPTURED IMAGE |
|---|---|

STORED IMAGE

ADD VIRTUAL SCALE

| THIRD CAPTURED IMAGE |
|---|

# FIG. 95

## FIG. 96

**45**

SIGNAL PROCESSING UNIT

**59** — FIRST SIGNAL PROCESSING UNIT

IRRADIATION POSITION DETECTOR — **61**

**60** — SECOND SIGNAL PROCESSING UNIT

SCALE TABLE — **62**

TABLE UPDATING UNIT — **64**

REPRESENTATIVE POINT DATA TABLE — **66**

**135** — LESION RECOGNITION UNIT

**136** — DIAGNOSTIC INFORMATION ACQUISITION UNIT

**137** — LEARNING UNIT

**138**

DIAGNOSTIC INFORMATION MANAGEMENT DEVICE

STATIC IMAGE STORAGE UNIT — **42**

## FIG. 97

LIGHT SOURCE DEVICE — 13

PROCESSOR DEVICE — 14

12

DISTANCE Z

200

201

MOVING MECHANISM — 202

CALIBRATION IMAGE PROCESSING DEVICE — 210

CALIBRATION DISPLAY CONTROLLER — 204

CALIBRATION IMAGE ACQUISITION UNIT — 206

CALIBRATION UNIT — 208

EP 4 209 821 B1

# FIG. 98

EP 4 209 821 B1

## FIG. 99

## FIG. 100

INSPECTION IMAGE

101

# FIG. 101

INSPECTION IMAGE

# FIG. 102

# FIG. 103

# FIG. 104

x (PIXEL POSITION OF SPOT IN X DIRECTION)

# FIG. 105

Lx=g2(y)

Lx1

Lx2

y2          y1

y (PIXEL POSITION OF SPOT IN Y DIRECTION)

# FIG. 106

Ly=h1(x)

Ly1

Ly2

x2          x1

x (PIXEL POSITION OF SPOT IN X DIRECTION)

# FIG. 107

y (PIXEL POSITION OF SPOT IN Y DIRECTION)

# FIG. 108

# FIG. 109

| LIGHT-EMITTING FRAME | | ONE FRAME | ONE FRAME | ONE FRAME | ONE FRAME | ONE FRAME | ONE FRAME |
|---|---|---|---|---|---|---|---|

STRIPE-PATTERN LIGHT HAVING PHASE X — TURNING ON / TURNING OFF

STRIPE-PATTERN LIGHT HAVING PHASE Y — TURNING ON / TURNING OFF

STRIPE-PATTERN LIGHT HAVING PHASE Z — TURNING ON / TURNING OFF

# FIG. 110

LPL

S

S

S

# FIG. 111

# FIG. 112

# FIG. 113

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018051680 A
- US 2019204068 A1
- US 2019320886 A1
- US 2018008124 A1
- JP 2016198304 A
- JP 2017217215 A
- JP 2017508529 A